# EUROPEAN PATENT SPECIFICATION

(11) **EP 2 089 430 B1**
(45) Date of publication and mention of the grant of the patent: **02.04.2014**
(21) Application number: 07821930.0
(22) Date of filing: 26.10.2007
(51) Int. Cl.: C07K 16/00, C12N 15/10, C12N 15/86, C07K 16/08, C07K 16/10

(54) **SELECTION OF HUMAN MONOCLONAL ANTIBODIES BY MAMMALIAN CELL DISPLAY**
SELEKTION HUMANER MONOKLONALER ANTIKÖRPER DURCH SÄUGETIERZELLENDISPLAY
SELECTION D'ANTICORPS MONOCLONAUX HUMAINS PAR AFFICHAGE DE CELLULES DE MAMMIFERES

(30) Priority: 07.11.2006 EP 06123620
(43) Date of publication of application: 19.08.2009
(73) Proprietor: Elatos GmbH, 1030 Wien (AT)
(72) Inventor: BACHMANN, Martin, 8472 Seuzach (CH); BAUER, Monika, 8048 Zürich (CH); BEERLI, Roger, 8106 Adlikon b. Regensdorf (CH)
(74) Representative: Bösl, Raphael Konrad
(86) International application number: PCT/EP2007/061570
(87) International publication number: WO 2008/055795

(56) References cited:
- WO-A-92/01047
- US-B1- 6 468 738
- HOOGENBOOM H R: "Selecting and screening recombinant antibody libraries" NATURE BIOTECHNOLOGY, NATURE PUBLISHING GROUP, NEW YORK, NY, US, vol. 23, no. 9, September 2005 (2005-09), pages 1105-1116, XP002348401 ISSN: 1087-0156 cited in the application
- MARCO BOORSMA ET AL: "New applications of alphavirus-based expression vectors" CYTOTECHNOLOGY, KLUWER ACADEMIC PUBLISHERS, DO, vol. 35, no. 3, 1 March 2001 (2001-03-01), pages 203-212, XP019236681 ISSN: 1573-0778
- KOLLER DANIEL ET AL: "A high-throughput alphavirus-based expression cloning system for mammalian cells" NATURE BIOTECHNOLOGY, NATURE PUBLISHING GROUP, NEW YORK, NY, US, vol. 19, no. 9, September 2001 (2001-09), pages 851-855, XP002424050 ISSN: 1087-0156
- DE LARA CAPURRO M ET AL: "Virus-expressed, recombinant single-chain antibody blocks sporozoite infection of salivary glands in Plasmodium gallinaceum-infected Aedes aegypti." THE AMERICAN JOURNAL OF TROPICAL MEDICINE AND HYGIENE APR 2000, vol. 62, no. 4, April 2000 (2000-04), pages 427-433, XP002430953 ISSN: 0002-9637
- HO MITCHELL ET AL: "Isolation of anti-CD22 Fv with high affinity by Fv display on human cells." PROCEEDINGS OF THE NATIONAL ACADEMY OF SCIENCES OF THE UNITED STATES OF AMERICA 20 JUN 2006, vol. 103, no. 25, 20 June 2006 (2006-06-20), pages 9637-9642, XP002430952 ISSN: 0027-8424 cited in the application
- HAWKINS R E ET AL: "CELL SELECTION STRATEGIES FOR MAKING ANTIBODIES FROM VARIABLE GENE LIBRARIES: TRAPPING THE MEMORY POOL" EUROPEAN JOURNAL OF IMMUNOLOGY, WEINHEIM, DE, vol. 22, no. 3, March 1992 (1992-03), pages 867-870, XP008056245 ISSN: 0014-2980

## Description

### FIELD OF THE INVENTION

The present invention is related to the fields of vaccinology, monoclonal antibodies and medicine. The invention provides methods for generating and selecting a eukaryotic cell expressing and displaying on its surface an antibody, preferably a single chain monoclonal antibody (e.g. scFv) which is capable of specifically binding an antigen of interest. Said cell is selected from a populations of eukaryotic, preferably mammalian, cells expressing a library of the variable regions of immunoglobulins derived from B cells which were pre-selected for their specificity towards said antigen of interest. The variable regions of the antibody with the desired specificity can be (i) cloned from the selected eukaryotic cell, (ii) reassembled to a species specific, preferably to a fully human, recombinant monoclonal antibody (mAb), and (iii) produced in large scale by expression in vitro. Recombinant antibodies comprising said variable regions can be expressed in various forms, including scFv fusions, Fab fragments, and whole antibodies such as IgG, IgE, IgD, IgA and IgM. Monoclonal antibodies produced by the method of the invention may be used for research purposes, diagnostic purposes or the treatment of diseases.

### RELATED ART

Monoclonal antibodies (mAbs) have proven their usefulness as tools for a wide spectrum of research and diagnostic applications as well as in therapeutic applications. Monoclonal antibodies generated by the conventional hybridoma technology comprise mouse sequences, giving rise to an undesired immune response against the foreign sequence when administered to humans. Such an anti-immunoglobuline responses can interfere with therapy (Miller et al. 1983, Blood 62:988-995) or cause allergic or immune complex hypersensitivity (Ratner B., Allergy, Anaphylaxis and Immunotherapy, Basic Principles and Practice, Williams & Wilkins Company, Baltimore, 1943).

Humanized antibodies (GB 2188638 B, 1987; Riechmann et al. 1988 Nature 332:323-327; Foote and Winter 1992 Mol. Biol. 224:487-499) or fully human antibodies (Mendez 1997, Nat Genet. 15:146-156) are therefore becoming increasingly important for the treatment of a growing number of diseases, including cancer, heart disease, infection and immune disorders.

Given the usefulness of mAbs in general, and the enormous therapeutic and commercial potential of human mAbs in particular, a lot of effort has been put into the development of screening platforms allowing for the isolation of mAbs with predetermined selectivity.

The numerous strategies available for production of recombinant antibodies have been reviewed recently (Hauwkins, 1992, Eur. J. Immunol. 22: 867-870;Hoogenboom 2005, Nature BiotechnoL 23:1105-1116). In each case, a number of consecutive steps are involved: (1) cloning of the immunological diversity contained in the antibodies' variable regions by recombinant DNA technology; (2) expression of such antibody libraries using a suitable expression system, thereby coupling phenotype (i. e. the expressed antibody) with genotype (i. e. the nucleic acid encoding it); (3) application of an appropriate selective pressure, typically selection for binding to antigen; and (4) amplification of the selected antibody-encoding clones, leading to an enrichment of specific binders. Typically, antibody libraries are enriched by several such rounds of selection before individual clones are analyzed.

The most frequently used screening methods for the isolation of recombinant antibodies are phage display (Hogenboom 2002, Methods Mol. Biol. 178:1-37), ribosome/mRNA display (Lipovsek and Plückthun 2004, J. Immunol. Method 290:51-67) and microbial cell display (Boder and Wittrup 1997, Nat. Biotechnol. 15:553-557). While each of these screening platforms has its specific advantages, they share the same drawback: they are all based on expression of antibodies in an unnatural environment, namely in bacteria (phage display), in vitro in a test tube (ribosome/mRNA display), or in yeast (microbial cell display). It is important to remember that the chemical and physical properties of antibodies are very variable due to the sequence variability inherent to this class of proteins. Therefore, every screening method involving the expression of antibodies under such unnatural conditions is likely to lead to a strongly biased set of antibodies, by selecting not only for the desired binding properties, but also for chemical and physical properties advantageous under the respective screening conditions. In contrast, a selection platform based on the expression of antibodies in their natural environment, i. e. the secretory pathway of mammalian cells, ensures that all the cellular components normally involved in antibody synthesis and processing (folding, disulfide bond formation, glycosylation etc.) are available in a physiological form and concentration. Therefore, screening for antibodies in a mammalian expression/selection system is likely to yield a set of antibodies much less biased by properties other than binding to the desired antigen.

Currently, there are two reports of screening systems based on cell surface expression of antibodies in mammalian cells. One screening system is based on Vaccinia virus-mediated expression of whole antibodies in mammalian cells (US2002/0123057A1). With this method, antibody heavy and light chain libraries are expressed from separate vectors, by consecutive infection and transfection: the heavy chains are expressed in target cells using a high-titer vaccinia virus heavy chain library, such that each cell produces in average one heavy chain; the light chains are shortly after expressed in these infected cells by transfection of a light chain plasmid library. This leads to libraries of cells, each expressing one heavy chain paired with an undefined number of different light chains, which can be screened for binding to antigen. However, there are significant drawbacks to this method: Two separate libraries need to be constructed and transferred to target cells for expression and screening. In addition, the method initially selects only for a specific heavy chain, and the matching light chain has to be isolated in a second screen. Finally, similar to phage and ribosome/mRNA display, multiple selection rounds have to be carried out, both for the initial isolation of the heavy chain, as well as for the identification of the matching light chain.

A second screening system based on cell surface expression of antibodies in mammalian cells has been described recently (Ho et al. 2006, Proc. Natl. Acad. Sci. USA 103:9637-9642). In this method, a scFv library is expressed in HEK-293T cells via transfection of plasmid DNA. This leads to pools of transfected cells expressing pools of scFv antibodies on their surface (i.e. more than one antibody per cell is displayed), which can be screened for binding to antigen. The scFv display method described by Ho et al. suffers from two main disadvantages. First, transfection is not the optimal method to introduce an antibody expression library into cells, since all transfection methods lead to the delivery of an undefined number of plasmid molecules to each cell. Thus, each transfected cell expresses an undefined number of different antibodies, further increasing the selective disadvantage of poorly expressed or otherwise problematic antibodies. Second, since the enrichment was reported to be only about 240-fold, also this method requires multiple rounds of selection to be carried out in order to isolate an antibody of interest from a complex library. Non-cytopathic and inducible expression vectors based on Sindbis and Semliki Forest viral vectors which can be applied to bioprocess development strategies were described. Furthermore, Sindbis-based expression cloning systems were developed that allow for the rapid identification of genes encoding proteins with a selected functional activity (Boorsma et al., 2001, Cytotechnology 35: 203-212).

One major drawback of performing antibody screens in mammalian cells is the limited number of antibodies that can be screened. This is in part due to the relatively small numbers of cells that can be handled at a time.

Thus, whereas phage display routinely allows for the screening of 10¹² to even 10¹³ clones in a single panning round (Barbas III et al. (eds.), Phage Display - A Laboratory manual, Cold Spring Habour Press, 2001), the throughput of a mammalian screening procedure in a one antibody per cell format is limited to the concomitant analysis of about 10⁶ to 10⁷ clones.

### SUMMARY OF THE INVENTION

We herein describe for the first time a screening platform for the isolation of species specific, preferably human, antibodies specifically binding an antigen of interest, that profits from the advantages of a mammalian cell-based expression system, while circumventing the disadvantages specific to the methods described above. A particular advantage of the screening platform described herein is the fact that it can be performed in a "one antibody per cell" format, which is preferred because it allows the screen to be completed in one single round of selection.

The invention provides a method of generating, selecting and isolating a cell expressing an antibody of desired specificity, preferably a monoclonal single chain antibody, most preferably a scFv. The invention also provides methods which allow to clone the variable regions of said antibody from that isolated cell and to recombinantly produce antibodies comprising said variable regions as fusion protein with a purification tag, eg. as Fc-fusion, as Fab fragment. The invention further provides methods which allow to clone the variable regions of said antibody from that isolated cell and to recombinantly produce whole antibodies comprising said variable regions , preferably as IgG1, IgG2 or IgG4. Said methods also allows to recombinantly produce antibodies with desired specificity in a fully species specific form, preferably as fully human antibodies.

It has surprisingly been found that the combination of pre-selection of antigen specific B cells with eukaryotic, preferably mammalian cell display of antibodies in a one antibody per cell format allows to set up an antibody screen which is complete after only one single round of screening.

Thus, the present invention relates to a method of isolating a cell expressing an antibody specifically binding an antigen of interest, said method comprising the steps of:
(a) selecting from a population of isolated B cells a sub-population of B cells by selecting B cells for their capability of specifically binding said antigen of interest;
(b) generating an alphaviral expression library, wherein each member of said alphaviral expression library encodes an antibody comprising at least one variable region (VR), preferably a heavy chain variable region (HCVR) and a light chain variable region (LCVR), by
   (i) generating a multitude of DNA molecules, wherein said generating comprises the step of amplifying a pool of DNA molecules from said sub-population of B cells, wherein each of said DNA molecules of said pool of DNA molecules encodes one of said at least one variable region (VR); and
   (ii) cloning a specimen of said multitude of DNA molecules into an alphaviral expression vector;
(c) introducing said alphaviral expression library into a first population of mammalian cells;
(d) displaying antibodies of said alphaviral expression library on the surface of said mammalian cells; and
(e) isolating from said first population of mammalian cells a cell, wherein said cell is selected for the capability of the antibody displayed on its surface of specifically binding said antigen of interest or antigenic determinant thereof.

Also described is a method of isolating a cell expressing an antibody specifically binding an antigen of interest, said method comprising the steps of: (a) providing a population of B cells; (b) selecting from said population of B cells a sub-population of B cells by selecting B cells for their capability of specifically binding said antigen of interest; (c) generating an expression library, wherein each member of said expression library encodes an antibody comprising at least one variable region (VR), by (i) generating a multitude of DNA molecules, wherein said generating comprises the step of amplifying a pool of DNA molecules from said sub-population of B cells, wherein each of

said DNA molecules of said pool of DNA molecules encodes one of said at least one variable region (VR); and (ii) cloning said multitude of DNA molecules into an expression vector; (d) introducing said expression library into a first population of eukaryotic, preferably mammalian cells; (e) displaying antibodies of said expression library on the surface of said eukaryotic, preferably mammalian cells; and (f) isolating from said first population of eukaryotic, preferably mammalian cells a cell, wherein said cell is selected for the capability of the antibody displayed on its surface of specifically binding said antigen of interest or a fragment or antigenic determinant thereof.

Also described is a method of isolating a cell expressing an antibody specifically binding an antigen of interest, said method comprising the steps of: (a) selecting from a population of isolated B cells a sub-population of B cells by selecting B cells for their capability of specifically binding said antigen of interest; (b) generating an expression library, wherein each member of said expression library encodes an antibody comprising at least one variable region (VR), by (i) generating a multitude of DNA molecules, wherein said generating comprises the step of amplifying a pool of DNA molecules from said sub-population of B cells, wherein each of said DNA molecules of said pool of DNA molecules encodes one of said at least one variable region (VR); and (ii) cloning said multitude of DNA molecules into an expression vector; (c) introducing said expression library into a first population of eukaryotic, preferably mammalian cells; (d) displaying antibodies of said expression library on the surface of said eukaryotic, preferably mammalian cells; and (e) isolating from said first population of eukaryotic, preferably mammalian cells a cell, wherein said cell is selected for the capability of the antibody displayed on its surface of specifically binding said antigen of interest or a fragment or antigenic determinant thereof.

The use of alphaviral expression libraries allows for an extraordinarily high screening efficiency. Thus, subject of the invention is a method of isolating a cell expressing an antibody specifically binding an antigen of interest, said method comprising the steps of: (a) selecting from a population of isolated B cells a sub-population of B cells by selecting B cells for their capability of specifically binding said antigen of interest; (b) generating an alphaviral expression library, wherein each member of said alphaviral expression library encodes an antibody comprising at least one variable region (VR), by (i) generating a multitude of DNA molecules, wherein said generating comprises the step of amplifying a pool of DNA molecules from said sub-population of B cells, wherein each of said DNA molecules of said pool of DNA molecules encodes one of said at least one variable region (VR); and (ii) cloning said multitude of DNA molecules into an alphaviral expression vector; (c) introducing said alphaviral expression library into a first population of eukaryotic, preferably mammalian cells; (d) displaying antibodies of said alphaviral expression library on the surface of said eukaryotic, preferably mammalian cells; and (e) isolating from said first population of eukaryotic, preferably mammalian cells a cell, wherein said cell is selected for the capability of the antibody displayed on its surface of specifically binding said antigen of interest or antigenic determinant thereof.

Also described is a method of isolating a cell expressing an antibody specifically binding an antigen of interest, said method comprising the steps of: (a) selecting from a population of isolated B cells a sub-population ofB cells by selecting B cells for their capability of specifically binding said antigen of interest; (b) generating an alphaviral expression library, said generating comprising the steps of: (i) generating a multitude of DNA molecules encoding antibodies, said generating a multitude of DNA molecules comprising the steps of: (1) amplifying from said sub-population of B cells a first pool of DNA molecules encoding HCVRs; (2) amplifying from said sub-population of B cells a second pool of DNA molecules encoding LCVRs; and (3) linking specimens of said first and of said second pool of DNA molecules to each other by a DNA encoding a linker region (LR); (ii) cloning a specimen of said multitude of DNA molecules into an alphaviral expression vector; wherein each member of said alphaviral expression library encodes an antibody comprising a signal peptide, a HCVR, a LCVR and a transmembrane region, wherein said HCVR and said LCVR are linked to each other by said linker region; (c) introducing said alphaviral expression library into a first population of eukaryotic, preferably mammalian cells; (d) displaying antibodies of said alphaviral expression library on the surface of said eukaryotic, preferably mammalian cells; and (e) isolating from said first population of eukaryotic, preferably mammalian cells a cell, wherein said cell is selected for the capability of the antibody displayed on its surface of specifically binding said antigen of interest or a fragment or antigenic determinant thereof.

Also described is a method of isolating a cell expressing an antibody specifically binding an antigen of interest, said method comprising the steps of: (a) selecting from a population of isolated B cells a sub-population of B cells by selecting B cells for their capability of specifically binding said antigen of interest; (b) generating an alphaviral expression library, said generating comprising the steps of: (i) generating a multitude of DNA molecules encoding antibodies, said generating comprising the steps of: (1) isolating RNA from said sub-population of B cells; (2) transcribing said RNA to cDNA; (3) amplifying from said cDNA said first pool of DNA molecules using a first mixture of oligonucleotides comprising at least two oligonucleotides capable of amplifying HCVR coding regions; (4) amplifying from said cDNA said second pool of DNA molecules using a second mixture of oligonucleotides comprising at least two oligonucleotides capable of amplifying LCVR coding regions; and (5) linking specimens of said first and said second pool of DNA molecules to each other by a DNA encoding said linker region; (ii) cloning a specimen of said multitude of DNA molecules into an alphaviral expression vector; wherein each member of said alphaviral expression library encodes an antibody comprising a signal peptide, a HCVR, a LCVR and a transmembrane region, wherein said HCVR and said LCVR are linked to each other by said linker region; (c) introducing said alphaviral expression library into a first population of eukaryotic, preferably mammalian cells; (d) displaying antibodies of said alphaviral expression library on the surface of said eukaryotic, preferably mammalian cells; and (e) isolating from said first population of eukaryotic, preferably mammalian cells a cell, wherein said cell is selected for the capability of the antibody displayed on its surface of specifically binding said antigen of interest or a fragment or antigenic determinant thereof.

Also described is a method of producing an antibody specifically binding an antigen of interest, said method comprising the steps of: (a) isolating a cell expressing an antibody according to any one of the methods above; (b) obtaining RNA from said isolated cell; (c) synthesizing cDNA encoding said antibody from said RNA; (d) cloning said cDNA into an expression vector, preferably an alphaviral expression vector; (e) generating a fusion construct encoding a fusion product comprising said antibody and said purification tag; (f) expressing said fusion product in a cell, preferably a mammalian cell; and (g) purifying said fusion product.

Also described is a method of producing an antibody specifically binding an antigen of interest, said method comprising the steps of: (a) isolating a cell expressing an antibody according to any one of the methods above; (b) obtaining RNA from said cell; (c) synthesizing cDNA form said RNA; (d) amplifying from said cDNA a DNA encoding VRs of said antibody expressed by said cell; (e) generating an expression construct comprising said DNA, wherein said expression construct is encoding at least one VR of said antibody expressed by said cell; (f) expressing said expression construct in a cell.

The description also discloses an expression vector for displaying polypeptides, preferably antibodies, on the surface of a eukaryotic, preferably mammalian cell; an expression vector, preferably an alphaviral expression vector, wherein said expression vector comprises DNA elements encoding a signal peptide, a transmembrane region and, preferably, a detection tag, and wherein further preferably said expression vector, preferably said alphaviral expression vector, comprises a restriction site allowing the cloning, preferably the orientation specific cloning, of DNA molecules encoding said polypeptides, preferably said antibody variable regions, into said expression vector;
an expression library comprising said expression vector, wherein preferably said expression library is an alphaviral expression library and said expression vector is an alphaviral expression vector; and
a eukaryotic, preferably mammalian, cell comprising said expression vector, preferably said alphaviral expression vector, or comprising at least one specimen of said expression library, preferably of said alphaviral expression library.

All embodiments described herein shall refer to all aspects of the invention and may be combined in any possible combination.

### DETAILED DESCRIPTION OF THE INVENTION

**"Animal"**: As used herein, the term "animal" refers to any organism comprising an immune system capable of producing antibodies. Preferred animals in the context of the invention are fish, amphibians, birds, reptiles, and mammals, preferably artiodactyls, rodents and primates. In a preferred embodiment said animal is selected from the group consisting of sheep, elk, deer, donkey, mule deer, mink, horse, cattle, pig, goat, dog, cat, rat, hamster, guinea pig, and mouse. In a further preferred embodiment said animal is a mouse, a rat or, most preferably, a primate. In a further preferred embodiment said animal is a non-human primate or a human, most preferably a human. In a further preferred embodiment said animal is a humanized mouse, e.g. as described as a source for humanized antibodies in (Lonberg

(2005), Nature Biotechnology 23(9):1117-1125). In a further preferred embodiment the animal is a humanized mouse or a human, preferably a human.

**"Antibody":** As used herein, the term "antibody" refers to a molecule, preferably a protein, which is capable of specifically binding an antigen, typically and preferably by binding an epitope or antigenic determinant or said antigen. The term antibody refers to whole antibodies, preferably of the IgG, IgA, IgE, IgM, or IgD class, more preferably of the IgG class, most preferably IgG I, IgG2, IgG3, and IgG4, and antigen-binding fragments thereof, including single chain antibodies, wherein further preferably said whole antibodies comprise either a kappa or a lambda light chain. The term "antibody" also refers to antigen binding antibody fragments, preferably to proteolytic fragments and their recombinant analogues, most preferably to Fab, Fab' and F(ab')2, Fd, and Fv. The term "antibody" further encompasses proteins comprising at least one, preferably two variable regions. Preferred antibodies are single chain antibodies, preferably scFvs, disulfide-linked Fvs (sdFv) and fragments comprising either a light chain variable region (LCVR) or a heavy chain variable region (HCVR). In the context of the invention the term "antibody" also refers to recombinant antibodies, preferably to recombinant proteins consisting of a single polypeptide, wherein said polypeptide comprises at least one variable region, preferably two variable regions, most preferably at least one, preferably one, HCVR and at least one, preferably one LCVR. In the context of the invention recombinant antibodies may further comprise functional elements, such as, for example, a linker region, a transmembrane region, a signal peptide or hydrophobic leader sequence, a detection tag and/or a purification tag.

**"Fv":** The term Fv refers to the smallest proteolytic fragment of an antibody capable of binding an antigen and to recombinant analogues of said fragment.

**"single chain antibody":** A single chain antibody is an antibody consisting of a single polypeptide. Preferred single chain antibodies consist of a polypeptide comprising a single VR, preferably a single HCVR. More preferred single chain antibodies are scFv, wherein said scFv consist of a single polypeptide comprising exactly one HCVR and exactly one LCVR, wherein said HCVR and said LCVR are linked to each other by a linker region, wherein preferably said linker region consists of at least 15, preferably of 15 to 20 amino acids (Bird et al. (1988) Science; 242(4877):423-426). Further preferred single chain antibodies are scFv, wherein said scFv are encoded by a coding region, wherein said coding region, in 5' to 3' direction, comprises in the following order: (1) a light chain variable region (LCVR) consisting of light chain framework (LFR) 1, complementary determining region (LCDR) 1, LFR 2, LCDR 2, LFR3, LCDR3 and LFR4 from a κ or λ light chain; (2) a flexible linker (L), and (3) a heavy chain variable region (HCVR) consisting of framework (HFR) 1, complementary determining region (HCDR) 1, HFR 2, HCDR 2, HFR3, HCDR3 and HFR4. Alternatively, single chain antibodies are scFv, wherein said scFv are encoded by a coding region, wherein said coding region, in 5' to 3' direction, comprises in the following order: (1) a heavy chain variable region (HCVR) consisting of framework (HFR) 1, complementary determining region (HCDR) 1, HFR 2, HCDR 2, HFR3, HCDR3 and HFR4; (2) a flexible linker (L), and (3) a light chain variable region (LCVR) consisting of light chain framework (LFR) 1, complementary determining region (LCDR) 1, LFR 2, LCDR 2, LFR3, LCDR3 and LFR4 from a κ or λ light chain.

**"diabody":** The term "diabody" refers to an antibody comprising two polypeptide chains, preferably two identical polypeptide chains, wherein each polypeptide chain comprises a HCVR and a LCVR, wherein said HCVR and said LCVR are linked to each other by a linker region, wherein preferably said linker region comprises at most 10 amino acids (Huston et al. (1988), PNAS 85(16):587958-83; Holliger et al. (1993), PNAS 90(14):6444-6448, Hollinger & Hudson, 2005, Nature Biotechnology 23(9):1126-1136; Arndt et al. (2004) FEBS Letters 578(3):257-261). Preferred linker regions of diabodies comprise 0, 1, 2, 3, 4, 5, 6, 7, 8, 9, or 10 amino acids.

**"species specific antibody":** The term "species specific antibody" refers to an antibody, preferably to a recombinant antibody, comprising variable and preferably also constant regions of only one single animal species. Preferred species specific antibodies are mouse antibodies, rat antibodies and human antibodies, most preferably human antibodies.

**"human antibodies"** and **"fully human antibodies":** As used herein, the term "human antibody" refers to an antibody, preferably a recombinant antibody, essentially having the amino acid sequence of a human immunoglobulin, or a fragment thereof, and includes antibodies isolated from human immunoglobulin libraries. In the context of the invention "human antibodies" may comprise a limited number of amino acid exchanges as compared to the sequence of a native human antibody. Such amino acid exchanges can, for example, be caused by cloning procedures. However, the number of such amino acid exchanges in human antibodies of the invention is preferably minimized; most preferably, the amino acid sequence of human antibodies is at least 95 %, more preferably at least 96 %, still more preferably 97 %, still more preferably 98 %, still more preferably 99 % and most preferably 100 % identical to that of native human antibodies. Preferred recombinant human antibodies differ from native human antibodies in at most 20, 15, 10, 9, 8, 7, 6, 5, 4, 3, 2, or 1 amino acid. Very preferably, differences in the amino acid sequence of recombinant human antibodies and native human antibodies are eliminated my means of molecular cloning, and thus, most preferably, the amino acid sequence of recombinant human antibodies and native human antibodies are identical. Such antibodies are also referred to as "fully human antibodies".

Preferred recombinant human antibodies comprise at least one, preferably one, heavy chain variable region and at least one, preferably one, heavy chain constant region, wherein said at least one heavy chain variable region is at least 95 %, more preferably at least 96 %, still more preferably 97 %, still more preferably 98 %, still more preferably 99 % and most preferably 100 % identical to a native human heavy chain variable region; and wherein said at least one heavy chain constant region is at least 95 %, more preferably at least 96 %, still more preferably 97 %, still more preferably 98 %, still more preferably 99 % and most preferably 100 % identical to a native human heavy chain constant region.

Further preferred recombinant human antibodies comprise at least one, preferably one, light chain variable region and at least one, preferably one, light chain constant region, wherein said at least one light chain variable region is at least 95 %, more preferably at least 96 %, still more preferably 97 %, still more preferably 98 %, still more preferably 99 % and most preferably 100 % identical to a native human light chain variable region; and wherein said at least one light chain constant region is at least 95 %, more preferably at least 96 %, still more preferably 97 %, still more preferably 98 %, still more preferably 99 % and most preferably 100 % identical to a native human light chain constant region.

Preferred human antibodies comprise least one, preferably one, heavy chain variable region and at least one, preferably one, heavy chain constant region, at least one, preferably one, light chain variable region and at least one, preferably one, light chain constant region, wherein said at least one light chain variable region is at least 95 %, more preferably at least 96 %, still more preferably 97 %, still more preferably 98 %, still more preferably 99 % and most preferably 100 % identical to a native human light chain variable region; and wherein said at leat one heavy chain variable region, said at least one heavy chain constant region, said at least one light chain constant region and said at least one light chain constant region is at least 95 %, more preferably at least 96 %, still more preferably 97 %, still more preferably 98 %, still more preferably 99 % and most preferably 100 % identical to the respective native human regions.

**"humanized antibodies":** As used herein, the term "humanized antibody" refers to antibodies wherein the antigen-binding parts of the antibody are derived from a non-human species and the remaining parts of the humanized antibody comprise or preferably entirely consist of a human amino acid sequence. The generation of humanized antibodies is within the skill of the artisan. The basic technology for the generation of humanized antibodies is, for example, disclosed in GB 2188638 B, Riechmann et al. (1988) Nature 332:323-327, and Foote and Winter (1992) Mol. Biol. 224:487-499. Preferred humanized antibodies are mouse antibodies, wherein the constant regions, more preferably the constant regions and the VR framework regions are exchanged by the corresponding human sequences ("CDR grafting").

**"monoclonal antibody":** As used herein, the term "monoclonal antibody" refers to an antibody population comprising only one single antibody species, i.e. antibodies having an identical amino acid sequence.

**"constant region (CR)":** The term "constant region" refers to a light chain constant region (LCCR) or a heavy chain constant region (HCCR) of.an antibody. Typically and preferably, said CR comprises one to four immunoglobulin domains characterized by disulfide stabilized loop structures. Preferred CRs are CRs, preferably kappa CRs or lambda CRs, of immunoglobulins, preferably of human immunoglobulins, wherein further preferably said immunoglobulins, preferably said human immunoglobulins, are selected from the group consisting of IgG1, IgG2, IgG3, IgG4, IgA, IgE, IgM, and IgD. Very preferred CRs are human CRs comprising or consisting of an amino acid sequence available from public databases, including, for example the Immunogenetic Information System (http://imgt.cines.fr/).

**light chain constant region (LCCR):** The LCCR, more specifically the kappa LCCR or the lambda LCCR, typically represents the C-terminal half of a native kappa or lambda light chain of an native antibody. A LCCR typically comprises about 110 amino acids representing one immunoglobulin domain.

**heavy chain constant region (HCCR):** The constant region of a heavy chain comprises about three quarters or more of the heavy chain of an antibody and is situated at its C-terminus. Typically the HCCR comprises either three or four immunoglobulin domains.

**"variable region (VR)":** Refers to the variable region or variable domain of an antibody, more specifically to the heavy chain variable region (HCVR) or to the light chain variable region (LCVR). Typically and preferably, a VR comprises a single immunoglobulin domain. Preferred VRs are VRs of immunoglobulins, preferably of human immunoglobulins, wherein further preferably said immunoglobulins, preferably said human immunoglobulins, are selected from the group consisting of IgG1, IgG2, IgG3, IgG4, IgA, IgE, IgM, and IgD. VRs of various species are known in the art. Preferred VRs are human VRs, wherein said human VRs exhibit at least 80 %, preferably at least 90 %, more preferably at least 95 %, most preferably at least 99 % sequence identity with any known human VR sequence, preferably with any human VR sequence available from public databases, most preferably with any human VR available from the Immunogenetics Information System (http://imgt.cines.fr/).

**"light chain variable region (LCVR)":** Light chain variable regions are encoded by rearranged nucleic acid molecules and are either a kappa LCVR or a lambda LCVR. In the context of the invention preferred kappa LCVRs are human kappa LCVRs, preferably human kappa LCVRs which are encoded by a DNA which can be amplified from human B cells using a primer combination of any one of SEQ ID NO:49 to 52 with any one of SEQ ID NO:53 to 56, and further preferably, PCR conditions described in Example 3.

In the context of the invention preferred lambda LCVRs are human lambda LCVRs, preferably human lambda LCVRs which are encoded by a DNA which can be amplified from human B cells using a primer combination of any one of SEQ ID NO:57 to 65 with any one of SEQ ID NO:66 to 68, and further preferably, PCR conditions described in Example 3.

**"heavy chain variable region (HCVR)":** Heavy chain variable regions are encoded by rearranged nucleic acid molecules. In the context of the invention preferred HCVRs are human HCVRs, preferably human HCVRs which are encoded by a DNA which can be amplified from human B cells using a primer combination of any one of SEQ ID NO:42 to 47 with SEQ ID NO:48 and, further preferably, PCR conditions described in Example 3.

**"antibody coding region":** As used herein, the term "antibody coding region" refers to any DNA encoding an antibody or an element thereof. Preferably, "antibody coding regions" refers to a DNA encoding a CR, preferably a HCCR or LCCR, or a VR, preferably a HCVR or a LCVR, of an antibody. Very preferred antibody coding regions are DNA fragments representing human antibody coding regions, preferably human VR coding regions, most preferably human VR coding regions which can be amplified from human B cells using any combination of primers of SEQ ID NO:42 to 68 and, further preferably, PCR conditions described in Example 3.

Preferred antibody coding regions are human kappa LCVR coding regions, preferably human kappa LCVR coding regions which can be amplified from human B cells using a primer combination of any one of SEQ ID NO:49 to 52 with any one of SEQ ID NO:53 to 56, and further preferably, PCR conditions described in Example 3.

Further preferred antibody coding regions are human lambda LCVR coding regions, preferably human lambda LCVR coding regions which can be amplified from human B cells using a primer combination of any one of SEQ ID NO:57 to 65 with any one of SEQ ID NO:66 to 68, and further preferably, PCR conditions described in Example 3.

Further preferred antibody coding regions are human HCVR coding regions, preferably human HCVR coding regions which can be amplified from human B cells using a primer combination of any one of SEQ ID NO:42 to 47 with SEQ ID NO:48 and, further preferably, PCR conditions described in Example 3.

**"antigen":** As used herein, the term "antigen" refers to a molecule which is bound by an antibody. Typically, an antigen is recognized by the immune system and/or by a humoral immune response and can have one or more epitopes, preferably B-cell epitopes, or antigenic determinants. The term antigen refers to protein and non-protein antigens. In the context of the invention, the term antigen shall also refer to haptens.

**"hapten":** The term hapten refers to a small molecule which is not recognized by the immune system in free form but which is recognized by the immune system when bound to a carrier, preferably to an immunogenic carrier. Preferred haptens are peptides, preferably peptides of protein antigens, wherein said peptides of protein antigens most preferably consist of 2 to 200, preferably 2 to 100, and most preferably of 2 to 50 amino acids. In a preferred embodiment said peptides of protein antigens consist of about 6 to about 30 amino acids. Further preferred haptens are selected from (a) opioids; (b) morphine derivatives, preferably selected from codeine, fentanyl, heroin, morphium and opium; (c) stimulants, preferably selected from amphetamine, cocaine, MDMA (methylenedioxymethamphetamine), methamphetamine, methylphenidate and nicotine; (d) hallucinogens, preferably LSD, mescaline, psilocybin, and cannabinoids.

**"antigen of interest":** The application provides methods for the selection of cells expressing antibodies with a desired specificity and to methods of producing such antibodies, i.e. the antibodies of the invention are capable of binding an antigen of interest. Typically and preferably, said antigen of interest is a protein antigen, a non-protein antigen or a hapten. The antigen of interest is preferably selected from the group consisting of (a) antigen of a microorganism or of a pathogen, (b) tumor antigen, (c) self antigen, and (d) allergen. Very preferably, said antigen of interest is a hapten.

**"fragment of the antigen of interest":** The term "fragment of an antigen of interest" refers to a fragment of an antigen, preferably of a polypeptide, comprising at least one antigenic determinant of said antigen. In a preferred embodiment a fragment of the antigen of interest is a polypeptide consisting of a stretch, preferably a consecutive stretch, of amino acids derived from said antigen of interest, wherein said polypeptide can be bound by an antibody. Typically and preferably, said fragment comprises at least 80, preferably at least 90, more preferably at least 95, still more preferably at least 99 and most preferably 100 % sequence identity with said antigen of interest. Typically and preferably, a fragment of the antigen of interest is a polypeptide consisting of 6 to 1000, preferably 6 to 500, more preferably 6 to 300, still more preferably 6 to 200, still more preferably 6 to 100 amino acids. Very preferred are fragments consisting of about 10, 20, 30, 40, 50, 60, 70, 80, 90 or 100 amino acids.

**"antigen of a microorganisms or pathogen":** An antigen of a microorganism or pathogen preferably is an antigen of infectious virus, infectious bacteria, parasites or infectious fungi. Such antigens include the intact microorganism or pathogen as well as natural isolates and fragments or derivatives thereof and also synthetic or recombinant compounds which are identical to or similar to natural microorganism antigens and induce an immune response specific for that microorganism. A compound is similar to an antigen of a microorganism or pathogen if it induces an immune response (humoral and/or cellular) to a natural microorganism antigen. Examples of infectious viruses, bacteria, and infectious fungi that are microbial antigen as used herein, are described in WO03/024481 (page 23 last paragraph to page 25 third paragraph).

**"tumor antigen":** A tumor antigen is a compound, such as a peptide, associated with a tumor or cancer and which can be bound by an antibody. Tumor antigens can be prepared from cancer cells either by preparing crude extracts of cancer cells, for example, as described in Cohen, et al., Cancer Research, 54:1055 (1994), by partially purifying the antigens, by recombinant technology or by de novo synthesis of known antigens. Tumor antigens include antigens that are antigenic portions of or are a whole tumor or cancer polypeptide. Such antigens can be isolated or prepared recombinantly or by any other means known in the art. Cancers or tumors include, but are not limited to, biliary tract cancer; brain cancer; breast cancer; cervical cancer; choriocarcinoma; colon cancer; endometrial cancer; esophageal cancer; gastric cancer; intraepithelial neoplasms; lymphomas; liver cancer; lung cancer (e.g. small cell and non-small cell); melanoma; neuroblastomas; oral cancer; ovarian cancer; pancreas cancer; prostate cancer; rectal cancer; sarcomas; skin cancer; testicular cancer; thyroid cancer; and renal cancer, as well as other carcinomas and sarcomas.

**"self antigen":** As used herein, the term "self antigen" refers, with respect to an animal, to proteins encoded by the DNA of said animal and products generated by proteins or RNA encoded by the DNA of said animal. Preferably, the tem "self antigen", as used herein, refers to proteins encoded by the human genome or DNA and products generated by proteins or RNA encoded by the human genome or DNA are defined as self. In one embodiment, self antigens are proteins that result from a combination of two or more self-molecules or fragments of self-molecules and proteins that have a sequence identity of at least 95 %, preferably at least 97 %, more preferably at least 99 % are also considered to be self antigens.

**"Allergens":** The term "allergen", as used herein, also encompasses "allergen extracts" and "allergenic epitopes" which are capable of inducing an allergic reaction of the immune system of an animal. Preferred allergens are pollen (e.g. grass, ragweed, birch and mountain cedar); house dust and dust mites; mammalian epidermal allergens and animal danders; mold and fungus; insect bodies and insect venom; feathers; food; and drugs (e.g., penicillin).

**"antigenic determinant":** As used herein, the term "antigenic determinant" is meant to refer to that portion of an antigen that is specifically recognized by B-lymphocytes. B-lymphocytes respond to foreign antigenic determinants by antibody production.

**"specifically binding" (antibody** / **antigen):** The specificity of an antibody relates to the antibody's capability of specifically binding an antigen. The specificity of this interaction between the antibody and the antigen (affinity) is characterized by a binding constant or, inversely, by a dissociation constant (Kd), It is to be understood that the apparent affinity of an antibody to an antigen in a multivalent interaction depends on the structure of the antibody and of the antigen, and on the actual assay conditions. The apparent affinity of an antibody to an antigen in a multivalent interaction may be significantly higher than in a monovalent interaction due to avidity. Thus, affinity is preferably determined under conditions favoring monovalent interactions. Kd can be determined by methods known in the art. Kd of a given combination of antibody and antigen is preferably determined by ELISA, most preferably by an ELISA essentially as described in Example 7, wherein a constant amount of immobilized antigen is contacted with a serial dilution of a known concentration of a purified antibody, preferably a monovalent antibody, for example scFv or Fab fragment. Kd is then determined as the concentration of the antibody where half-maximal binding is observed. Alternatively, Kd of a monovalent interaction of an antibody and an antigen is determined by Biacore analysis as the ratio of on rate (kₒₙ) and off rate (k_{off}.). Lower values of Kd indicate a stronger binding of the antibody to the antigen than higher values of Kd. Thus, in the context of the application, an antibody is considered to be "specifically binding an antigen (of interest)", when the dissociation constant (Kd), preferably determined as described above, and further preferably determined in a monovalent interaction, is at most 1 mM (<= 10⁻³ M), preferably at most 1 µM (<= 10⁻⁶M), most preferably at most 1 nM (<= 10⁻⁹M). Very preferred are antibodies capable of binding an antigen with a Kd of less than 1 nM (< 10⁻⁹ M, "subnanomolar"), wherein further preferably Kd is determined in a monovalent interaction. Further preferred antibodies are capable of binding an antigen with a Kd of 0.01 to 10 nM, more preferably of 0.01 to 5 nM, still more preferably of 0.01 to 3 nM, most preferably of 0.1 to 2 nM, wherein further preferably Kd is determined in a monovalent interaction. Still further preferred antibodies are capable of binding an antigen with a Kd of 0.1 to 50 nM, more preferably of 1.0 to 50 nM, still more preferably of 1.0 to 30 nM, most preferably of 0.1 to 2 nM, wherein further preferably Kd is determined in a monovalent interaction.

**"specifically binding"(antibody displayed on a cell** / **antigen):** With respect to an antibody displayed on a mammalian cell the specificity of the binding of an antigen is preferably determined in an fluorescence assay essentially as set forth herein in Example 4, wherein the intensity of a fluorescence signal is correlated with the amount of antigen bound by a cell displaying said antibody. Antibodies displayed on mammalian cells are regarded as specifically binding an antigen, when the intensity of the fluorescence signal is higher than the signal detected for control cells. Preferably, said signal is at least two times higher than that of control cells.

**"B-cell":** As used herein, the term "B-cell" refers to a cell produced in the bone marrow of an animal expressing membrane-bound antibody specific for an antigen. Following interaction with the antigen it differentiates into a plasma cell producing antibodies specific for the antigen or into a memory B-cell.

**"Antigen-specific B cell":** As used herein, the term "antigen-specific B cell" refers to a B cell which expresses antibodies that are able to distinguish between the antigen of interest and other antigens and which specifically bind to that antigen of interest with high or low affinity but which do not bind to other antigens.

**"Memory B-cell":** As used herein, "memory B-cell" refers to a B-cell sub-type that is formed following a primary contact with the antigen of interest. When a B-cell is activated, by specifically recognizing the antigen of interest, it proliferates to form antibody producing plasma cells and long-lived memory B cells. These memory B cells are specific for the antigen of interest that stimulated their production. If this antigen of interest is encountered again, memory B cells can recognize it and quickly proliferate.

**"immunizing":** As used herein the term immunizing means administering to an animal the antigen of interest, a fragment or antigenic determinant thereof, preferably together with an adjuvant in a dose capable of inducing a detectable immune response, preferably a B-cell response.

**"Tag":** The term tag, preferably a purification or detection tag, refers to a polypeptide segment that can be attached to a second polypeptide to provide for purification or detection of the second polypeptide or provides sites for attachment of the second polypeptide to a substrate. In principle, any peptide or protein for which an antibody or other specific binding agent is available can be used as an affinity tag. Tags include haemagglutinin tag, myc tag, poly-histidine tag, protein A, glutathione S transferase, Glu-Glu affinity tag, substance P, FLAG peptide, streptavidine binding peptide, or other antigenic epitope or binding domain (mostly taken from US06686168).

**"expression library":** The term expression library refers to a multitude of expression vectors of the same type, wherein individual expression vectors expresses a different polypeptide, e.g. a different antibody. Preferred expression libraries are viral expression libraries, most preferably alphaviral expression libraries. Alphaviral expression libraries are preferred because of their capability of self-replication. Furthermore, alphaviral expression libraries allow to display about one single antibody species per cell, wherein about each individual cell displays a distinct antibody species. Very preferred alphaviral expression libraries are Sindbis-based libraries as described, for example, in WO1999/025876A1 and Koller et al. 2001 (Nature Biotech 19:851-855).

**"Multiplicity of infection (MOI)":** The term multiplicity of infection refers to the ratio between the number of infectious virus particles in a viral, preferably alphaviral, expression library and the number of cells exposed to the virus.

The application provides a method of generating, selecting and isolating a cell expressing an antibody of desired specificity. In more detail, the application provides a method of isolating a cell expressing an antibody specifically binding an antigen of interest, said method comprising the steps of: (a) selecting from a population of isolated B cells a sub-population of B cells by selecting B cells for their capability of specifically binding said antigen of interest; (b) generating an alphaviral expression library, wherein each member of said alphaviral expression library encodes an antibody comprising at least one variable region (VR), by (i) generating a multitude of DNA molecules, wherein said generating comprises the step of amplifying a pool of DNA molecules from said sub-population of B cells, wherein each of said DNA molecules of said pool of DNA molecules encodes one of said at least one variable region (VR); and (ii) cloning a specimen of said multitude of DNA molecules into an alphaviral expression vector; (c) introducing said alphaviral expression library into a first population of mammalian cells; (d) displaying antibodies of said alphaviral expression library on the surface of said mammalian cells; and (e) isolating from said first population of mammalian cells a cell, wherein said cell is selected for the capability of the antibody displayed on its surface of specifically binding said antigen of interest or a fragment or antigenic determinant thereof.

Furthermore, the application provides for a method of isolating a cell expressing an antibody specifically binding an antigen of interest, said method comprising the steps of: (a) selecting from a population of isolated B cells a sub-population of B cells by selecting B cells for their capability of specifically binding said antigen of interest; (b) generating an expression library, preferably an alphaviral expression library, said generating comprising the steps of: (i) generating a multitude of DNA molecules encoding antibodies, said generating a multitude of DNA molecules comprising the steps of: (1) amplifying from said sub-population of B cells a first pool of DNA molecules encoding HCVRs; (2) amplifying from said sub-population of B cells a second pool of DNA molecules encoding LCVRs; and (3) linking specimens of said first and of said second pool of DNA molecules to each other by a DNA encoding a linker region (LR); (ii) cloning said multitude of DNA molecules into an expression vector, preferably into an alphaviral expression vector; wherein each member of said expression library, preferably of said alphaviral expression library, encodes an antibody comprising a signal peptide, a HCVR, a LCVR and a transmembrane region, wherein said HCVR and said LCVR are linked to each other by said linker region; (c) introducing said expression library, preferably said alphaviral expression library, into a first population of cells, preferably mammalian cells; (d) displaying antibodies of said expression library, preferably of said alphaviral expression library, on the surface of said cells, preferably mammalian cells; and (e) isolating from said first population of cells, preferably mammalian cells, a cell, wherein said cell is selected for the capability of the antibody displayed on its surface of specifically binding said antigen of interest or a fragment or antigenic determinant thereof.

Moreover, the application provides for method of isolating a cell expressing an antibody specifically binding an antigen of interest, said method comprising the steps of: (a) selecting from a population of B cells a sub-population of B cells by selecting B cells for their capability of specifically binding said antigen of interest; (b) generating an expression library, preferably an alphaviral expression library, said generating comprising the steps of: (i) generating a multitude of DNA molecules encoding antibodies, said generating comprising the steps of: (1) isolating RNA from said sub-population ofB cells; (2) transcribing said RNA to cDNA; (3) amplifying from said CDNA said first pool of DNA molecules using a first mixture of oligonucleotides comprising at least two oligonucleotides capable of amplifying HCVR coding regions; (4) amplifying from said cDNA said second pool of DNA molecules using a second mixture of oligonucleotides comprising at least two oligonucleotides capable of amplifying LCVR coding regions; and (5) linking specimens of said first and said second pool of DNA molecules to each other by a DNA encoding said linker region; (ii) cloning said multitude of DNA molecules into an expression vector, preferably an alphaviral expression vector; wherein each member of said expression library, preferably of said alphaviral expression library encodes an antibody comprising a signal peptide, a HCVR, a LCVR and a transmembrane region, wherein said HCVR and said LCVR are linked to each other by said linker region; (c) introducing said expression library, preferably said alphaviral expression library, into a first population of cells, preferably mammalian cells; (d) displaying antibodies of said expression library, preferably of said alphaviral expression library, on the surface of said cells, preferably of said mammalian cells; and (e) isolating from said first population of cells, preferably of mammalian cells, a cell, wherein said cell is selected for the capability of the antibody displayed on its surface of specifically binding said antigen of interest or a fragment or antigenic determinant thereof.

In a preferred embodiment each antibody encoded by said expression library, preferably by said alphaviral expression library, further comprises a signal peptide and a transmembrane region.

In a further preferred embodiment said antibody specifically binding said antigen of interest is a humanized or human antibody, preferably a human antibody. In a further preferred embodiment said antibody specifically binding said antigen of interest is a single chain antibody, preferably a scFv. Thus, the antibody displayed on the surface of said cell is preferably expressed as a scFv comprising a transmembrane region. In a preferred embodiment said at least one VR comprised by said antibody is a heavy chain variable region (HCVR) and a light chain variable region (LCVR). Thus, in a further preferred embodiment each member of said expression library, preferably said alphaviral expression library, encodes an antibody, wherein said antibody is expressed as fusion protein consisting of a single polypeptide, wherein said polypeptide comprises a signal peptide, a HCVR, a LCVR, and a transmembrane region. Typically and preferably, cDNA encoding variable regions is synthesized from RNA obtained from said sub-population of antigen specific B cells, cloned and expressed in an expression vector, preferably an alphaviral expression vector, wherein the variability of antigen-specific antibodies is increased by randomly linking different light and heavy chain variable regions. This is achieved by separately amplifying DNA molecules encoding HCVRs and LCVRs and linking them together by a DNA encoding a linker region (LR). Therefore, in a preferred embodiment said generating an expression library, preferably an alphaviral expression library, comprises the steps of: (a) generating a multitude of DNA molecules encoding antibodies, said generating comprising the steps of: (i) amplifying from said sub-population of B cells a first pool of DNA molecules encoding HCVRs; (ii) amplifying from said sub-population of B cells a second pool of DNA molecules encoding LCVRs; and (iii) linking specimens of said first and of said second pool of DNA molecules to each other by a DNA encoding a linker region (LR); (b) cloning a specimen of said multitude of DNA molecules into an expression vector, preferably into an alphaviral expression vector; wherein each member of said expression library, preferably of said alphaviral expression library, encodes an antibody comprising a signal peptide, a HCVR, a LCVR and a transmembrane region, wherein said HCVR and said LCVR are linked to each other by said linker region.

In a further preferred embodiment said generating a multitude of DNA molecules comprises the steps of: (a) isolating RNA from said sub-population of B cells; (b) transcribing said RNA to cDNA; and (c) amplifying from said cDNA a pool of DNA molecules using a mixture of oligonucleotides comprising at least two oligonucleotides capable of amplifying VR coding regions.

In a further preferred embodiment said generating a multitude of DNA molecules comprises the steps of: (a) isolating RNA from said sub-population ofB cells; (b) transcribing said RNA to cDNA; (c) amplifying from said cDNA said first pool of DNA molecules using a first mixture of oligonucleotides comprising at least two oligonucleotides capable of amplifying HCVR coding regions; (d) amplifying from said cDNA said second pool of DNA molecules using a second mixture of oligonucleotides comprising at least two oligonucleotides capable of amplifying LCVR coding regions; and (e) linking specimens of said first and said second pool of DNA molecules to each other by a DNA encoding said linker region (LR). In a preferred embodiment the order of these elements from N- to C-terminus of said antibody is LCVR-LR-HCVR or HCVR-LR-LCVR, most preferably said order is HCVR-LR-LCVR.

The cloning of variable regions is a standard procedure generally known in the art and has been described for various species, including humans, non-human primates, mouse, rabbit, and chicken. For review see Barbas III et al. (eds.), Phage Display - A Laboratory manual, Cold Spring Habour Press, 2001, in particular the chapter Andris-Widhopf et al., Generation of Antibody Libraries: PCR Amplification and Assembly of Light- and Heavy-chain Coding Sequences, therein. Andris-Widhopf et al. discloses sequences of oligonucleotides capable of amplifying variable region coding regions (VR coding regions), preferably HCVR coding regions or LCVR coding regions, of the afore mentioned species . Furthermore, oligonucleotides capable of amplifying HCVR coding regions or LCVR coding regions, preferably human HCVR coding regions or LCVR coding regions, can be designed by the artisan by comparing known sequences of antibody coding regions which are available from databases such as, for example, Immunogenetics (http://imgt.cines.fr/), Kabat (www.kabatdatabase.com), and Vbase (http://vbase.mrc-cpe.cam.ac.uk/), and by identifying consensus sequences suitable for primer design. Based on general knowledge in molecular biology, on the afore mentioned manual (Barbas III et al. (eds.) Phage Display - A Laboratory manual, Cold Spring Habour Press, 2001) and the references cited therein, the artisan is able to design oligonucleotides capable of amplifying HCVR coding regions or LCVR coding regions, wherein preferably said primers comprise suitable restriction sites for the cloning of the amplified products and wherein preferably said oligonucleotides also encode said linker region. Further Strategies for amplifying and cloning VRs are described in Sblattero and Bradbury (1998) Immunotechnology 3:271-278 and Weitkamp et al. (2003), J. Immunol. Meth. 275:223-237.

Preferred oligonucleotides encode restriction sites (RS1 and RS2) to allow for cloning of the assembled coding regions in the orientation RS1-LCVR-LR-HCVR-RS2 or RSI-HCVR-LR-LCVR-RS2, preferably RS1-LCVR-LR-HCVR-RS2. In a preferred embodiment, said restriction sites are distinct from one another and at least one of them generates a single-stranded overhang ("sticky end"), thus allowing for directional cloning. More preferably, said RS are eight or more base pairs long and recognized by "rare cutting" restriction enzymes selected from but not limited to the list of Asc1, Fse1, Not1, Pac1, Pme1, Sfi1 and Swa1. Most preferably, the RS are recognition sequences for Sfi1 (which cuts the sequence 5'-GGCCNNNNNGGCC-3'), and the sequences of RS1 and RS2 are, respectively, 5'-GGCCCAGGCGGCC-3' and 5'-GGCCAGGCCGGCC-3'. Primers suitable for the generation of libraries of scFv cDNAs of the format Sfi1-LCVR-GGSSRSSSSGGGGSGGGG-HCVR-Sfi1 have been described (Barbas, C.F., III, Burton, D. R., Scott, J. K. and Silverman, G. J. (2001) Phage Display. A Laboratory Manual. Cold Spring Harbor Laboratory Press, 9.24-9.26) and are listed below.

The human HCVR, human kappa LCVR and human lambda LCVR coding regions are amplified by PCR with mixtures of specific sense and antisense primers annealing in the framework 1 and 4 regions, respectively. The principal set of primers is described here: Sblattero D, Bradbury A. (1998) A definitive set of oligonucleotide primers for amplifying human V regions. Immunotechnology. 3, 271-278. As an alternative to the use of a specific mix of antisense primers for the amplification of HCVR, kappa LCVR and lambda LCVR coding sequences, one antisense primer annealing in the gamma, kappa and lambda constant region can be used, respectively.

It has surprisingly been found that the efficiency of the subsequent cloning of specific VR coding regions can be enhanced by pre-amplifying the transcriptome of said sub-population of B cells, preferably by using the template switch protocol as described by Zhu et al. 2001, Biotechniques 30(4):892-897, wherein single stranded cDNA is synthesized with the CDS oligonucleotide SEQ ID NO:32 and the SMART II oligonucleotide SEQ ID NO:33 as switch template. However, the pre-amplification of the transcriptome needs to be balanced against the possible loss of certain rare cDNA species and the possible accumulation of sequence errors.

Thus, in a preferred embodiment, said transcribing of said RNA to cDNA comprises the steps of pre-amplifying the transcriptome of said sub-population of B cells, wherein preferably said pre-amplifying comprises the steps of: (a) selectively transcribing polyadenylated mRNA contained in said RNA to single stranded cDNA; and (b) amplifying double stranded cDNA from said single stranded cDNA. In a further preferred embodiment said selectively transcribing is performed using the oligonucleotides of SEQ ID NO:32 and SEQ ID NO:33. In a further preferred embodiment said amplifying double stranded cDNA is performed using the oligonucleotides of SEQ ID NO:33 and SEQ ID NO:34, wherein preferably the number of PCR cycles is less than 20, more preferably less than 15, still more preferably 10 to 14, and most preferably 14.

In principle said linker region may consist of any polypeptide comprising suitable length and flexibility to accommodate appropriate folding and assembly of the heavy and light chain variable regions. In a preferred embodiment said linker region consists of 5 to 30, preferably 5 to 22, more preferably 5 to 20, and most preferably of 18 amino acid. It is known to the artisan, that the length of the linker regions influences the structure and, thus, the immunological characteristics of the resulting antibody, in particular of the resulting single chain antibody. Linker regions of less than 15 amino acids in length typically lead to the formation of so called "diabodies", whereas linker regions comprising at least 15 amino acid residues typically lead to the formation of scFv (Huston et al. (1988), PNAS 85(16):587958-83; Holliger et al. (1993), PNAS 90(14):6444-6448, Hollinger & Hudson, 2005, Nature Biotechnology 23(9):1126-1136). Thus, in a further preferred embodiment said linker region consists of 15 to 20, most preferably of 18 amino acid residues. In a very preferred embodiment said linker region comprises or further preferably consists of SEQ ID NO:107.

In a further preferred embodiment said linker region is encoded by an oligonucleotide contained in said mixture of oligonucleitodes, preferably in said first mixture of oligonucleotides, and/or in said second mixture of oligonucleotides.

Said linking specimens of said first and said second pool of DNA molecules to each other by a DNA encoding said linker region may be performed by ligating said DNA molecules with said DNA encoding said linker region. Typically and preferably, said linking is performed by PCR overlap extension using an overlap in the sequence of the oligonucleotides encoding said linker region. Thus, in a further preferred embodiment a first part of said linker region is encoded by an oligonucleotide contained in said first mixture of oligonucleotides and a second part of said linker region is encoded by an oligonucleotide contained in said second mixture of oligonucleotides, wherein preferably said oligonucleotide encoding said first part of said linker region and said oligonucleotide encoding said second part of said linker region comprise an overlap, wherein further preferably said overlap is at least 3, preferably at least 10, more preferably at least 20, and most preferably 24 nucleotides in length, wherein still further preferably said overlap is at most 50, preferably at most 40, more preferably at most 30, and most preferably at most 24 nucleotides in length.

In a further preferred embodiment said linking of said specimens of said first pool of DNA molecules and of said second pool of DNA molecules to each other is performed by PCR using the oligonucleotides depicted in SEQ ID NO:69 and SEQ ID NO:70 as primers.

Typically and preferably, the resulting multitude of DNA molecules encoding antibodies, preferably human single chain antibodies, most preferably human scFv, are about 750-800 bp in length and further preferably flanked by two Sfi1 restriction sites.

In one embodiment said pool of DNA molecules, preferably said first and/or said second pool of DNA molecules is either generated by pooling DNA molecules obtained in independent PCR reactions, each reaction performed with a different pair of oligonucleotides capable of amplifying VR coding regions, wherein preferably the oligonucleotides contained in an individual reaction are in an equimolar ratio. Thus, said mixture of oligonucleotides, said first mixture of oligonucleotides and/or said second mixture of oligonucleotides comprises or preferably consists of exactly one pair of oligonucleotides capable of amplifying VR coding regions, preferably HCVR coding regions or LCVR coding regions. The artisan may consider to standardize the concentration of DNA molecules generated in different reactions and with different pairs of oligonucleotides in said pool of DNA molecules, preferably said first and/or said second pool of DNA molecules, to the same concentration. The artisan may further consider to adapt in said pool of DNA molecules, preferably said first and/or said second pool of DNA molecules the ratio of DNA molecules encoding certain VR to the frequency of the corresponding VR coding regions in the genome of said B cells.

Typically and preferably said generating of said pool of DNA molecules, preferably said first and/or said second pool of DNA molecules is performed in a single reaction using more than one pair of oligonucleotides in said reaction. In a preferred embodiment said mixture of oligonucleotides, preferably said first mixture of oligonucleotides, comprises at least two oligonucleotides capable of amplifying human HCVR coding regions. In a further preferred embodiment said mixture of oligonucleotides, preferably said first mixture of oligonucleotides, comprises at least two, preferably all, oligonucleotides selected from the group consisting of SEQ ID NO:42 to 48. In a further preferred embodiment said mixture of oligonucleotides, preferably said second mixture of oligonucleotides, comprises at least two oligonucleotides capable of amplifying kappa LCVR coding regions, preferably human LCVR coding regions. In a further preferred embodiment said mixture of oligonucleotides, preferably said second mixture of oligonucleotides, comprises at least two oligonucleotides capable of amplifying kappa LCVR coding regions, wherein preferably said mixture of oligonucleotides, preferably said second mixture of oligonucleotides, comprises at least two, preferably all, oligonucleotides selected from the group consisting of SEQ ID NO:49 to 56.

In a preferred embodiment said mixture of oligonucleotides, preferably said second mixture of oligonucleotides, comprises at least two oligonucleotides capable of amplifying lambda LCVR coding regions, preferably human lambda LCVR coding regions. In a preferred embodiment said mixture of oligonucleotides, preferably said second mixture of oligonucleotides, comprises at least two oligonucleotides capable of amplifying lambda LCVR coding regions, wherein further preferably said mixture of oligonucleotides, preferably said second mixture of oligonucleotides, comprises at least two, preferably all, oligonucleotides selected from the group consisting of SEQ ID NO:57 to 68.

In a further preferred embodiment said mixture of oligonucleotides, said first mixture of oligonucleotides or said second mixture of oligonucleotides comprise a total amount of primers capable of amplifying VR coding regions, wherein all forward primers and all reverse primers contained in said total amount are in an equimolar ratio.

In a further preferred embodiment said antibody encoded by said expression library, preferably by said alphaviral expression library, comprises exactly one VR and a transmembrane region, wherein preferably said exactly one VR is a HCVR.

In a further preferred embodiment said antibody encoded by said expression library, preferably by said alphaviral expression library, comprises said HCVR, said LCVR and said linker region (LR), in an order selected from: (a) LCVR-LR-HCVR; and (b) HCVR-LR-LCVR; wherein preferably said order is LCVR-LR-HCVR.

To ensure cell surface display of said antibody, said antibody is expressed with a signal peptide directing said antibody to the secretory pathway through the endoplasmic reticulum of said cell, preferably of said mammalian cell, wherein preferably said signal peptide is located at the N-terminus of said antibody, and wherein further preferably said signal peptide is cleaved off said antibody during the processing and transport in said cell, preferably in said mammalian cell. Furthermore, said antibody is expressed with a transmembrane region anchoring said antibody in the cell membrane. Very preferably, said transmembrane region is located at the C-terminus of said antibody and causes said antibody to remain attached to the outer surface of said cell. The anchoring of said antibody in the cell membrane can also be achieved, for example, by GPI-linking (Moran & Caras 1991, The Journal of Cell Biology 115(6):1595-1600).

Thus, in a preferred embodiment said cloning a specimen of said multitude of DNA molecules into an expression vector, preferably into an alphaviral expression vector, comprises the steps of: (a) generating a DNA construct encoding said antibody comprising a signal peptide, a HCVR, a LCVR and a transmembrane region, by linking a specimen of said multitude of DNA molecules to a first DNA element encoding said transmembrane region; and (b) functionally linking said DNA construct to a second DNA element encoding said signal peptide directing said antibody to the secretory pathway, wherein preferably said functionally linking is performed in such a way that said signal peptide is linked to the N-terminus of said antibody.

Signal peptides directing a protein to the secretory pathway of a eukaryotic cell are generally known in the art and are disclosed, for example, in Nielsen et al. (1997), Protein Engineering, 10:1-6. In one embodiment, the signal peptide is derived from a secretory or type I transmembrane protein. In a preferred embodiment, the signal peptide is derived from a secretory protein such as member of the serum protein family (albumin, transferrin, lipoproteins, immunoglobulins), an extracellular matrix protein (collagen, fibronectin, proteoglycans), a peptide hormone (insulin, glucagon, endorphins, enkephalins, ACTH), a digestive enzyme (trypsin, chymotrypsin, amylase, ribonuclease, deoxyribonuclease) or a milk protein (casein, lactalbumin). In a more preferred embodiment, the signal peptide is derived from an immunoglobulin, preferably a light chain variable region. In a further preferred embodiment said signal peptide is a mouse Ig kappa light chain signal peptide, and wherein preferably said signal peptide comprises or further preferably consists of SEQ ID NO:105.

In one embodiment, said transmembrane region is derived from an integral membrane protein. In a preferred embodiment, said transmembrane region is an internal stop-transfer membrane-anchor sequence derived from a type I transmembrane protein (Do et al. (1996), Cell 85:369-78; Mothes et al. (1997), Cell 89:523-533) such as a cell adhesion molecule (integrins, mucins, cadherins), a lectin (Sialoadhesin, CD22, CD33), or a receptor tyrosin kinase (insulin receptor, EGF receptor, FGF receptor, PDGF receptor). In a more preferred embodiment, said transmembrane region is derived from a receptor tyrosine kinase, more preferably from human platelet-derived growth factor receptor (PDGFR), most preferably from hPDGFR B chain (accession number NP_002600). In a very preferred embodiment said transmembrane region is derived from human PDGFR beta chain, wherein preferably said transmembrane region comprises or further preferably consists of SEQ ID NO:106.

It is advantageous to express said antibody as a polypeptide further comprising a tag allowing the detection of cells expressing said antibody and the quantification of the expression level. Thus, in a further preferred embodiment said antibody further comprises a detection tag, wherein preferably said detection tag is HA, and wherein further preferably said detection tag comprises or still further preferably consists of SEQ ID NO:108.

In a very preferred embodiment said antibody encoded by said expression library, preferably by said alphaviral expression library, comprises a signal peptide (SP), a HCVR, a LCVR, a linker region (LR) and a transmembrane region (TM), wherein the order of said elements from the N- to the C-terminus of said antibody is: SP-LCVR-LR-HCVR-TM. In a further preferred embodiment said antibody encoded by said expression library, preferably by said alphaviral expression library, comprises a signal peptide (SP), a HCVR, a LCVR, a linker region (LR), a transmembrane region (TM) and a detection tag (TAG), wherein the order of said elements from the N- to the C-terminus of said antibody is: SP-LCVR-LR-HCVR-TAG-TM.

The multiplicity of assembled VR coding regions, preferably in the format Sfi1-LCVR-GGSSRSSSSGGGGSGGGG-HCVR-Sfi1, is then cloned into an expression vector, preferably into a viral expression vector, most preferably into an alphaviral expression library, creating an antibody expression library. In a preferred embodiment said expression library is a viral expression library, preferably a viral expression library derived from an RNA virus, wherein further preferably said RNA virus is a member of the Togaviridae, wherein still further preferably said RNA virus is an alphavirus. In a more preferred embodiment said expression library is an alphaviral expression library, wherein preferably said alphaviral expression library is derived from an alphavirus selected from the group of: (a) Sindbis virus; (b) Semliki forest virus; and (c) Venezuelan equine encephalitis virus. In a very preferred embodiment said alphaviral expression library is derived from Sindbis virus.

Alphaviruses, including Sindbis virus, can function in a broad range of host cells, including mammalian, avian, amphibian; reptilian and insect cells. Their genome comprises elements capable of directing expression of proteins, including heterologous proteins, encoded by nucleic acids of said viral genome in large amounts.

In one embodiment, said expression library is based on a single Sindbis RNA replicon. However, expression of structural and non-structural viral proteins can also be separated, and the structural proteins can be provided either by a packaging cell line or by a helper virus replicon (Bredenbeek PJ, Frolov I, Rice CM, Schlesinger S. (1993) Sindbis virus expression vectors: packaging of RNA replicons by using defective helper RNAs. J Virol. 67, 6439-6446). In a preferred embodiment, said expression library is based on two separate Sindbis RNA replicons, one encoding the nonstructural proteins plus said antibody, the other encoding the structural proteins. A Sindbis based alphaviral expression systems useful in the context of the invention has been described in detail in WO1999/025876A1.

In one embodiment said expression library comprises an expression vector, wherein said expression vector is a viral expression vector, wherein preferably said viral expression vector is an alphaviral expression vector, wherein further preferably said alphaviral expression vector is derived from Sindbis virus. In a preferred embodiment said expression vector comprises DNA elements encoding said signal peptide, said transmembrane region and, optionally, said detection tag in the desired order and further comprises a restriction site allowing the cloning, preferably the orientation specific cloning, of said multitude of DNA molecules into said expression vector. In a preferred embodiment said expression vector, preferably said alphaviral expression vector, comprises a DNA encoding a signal peptide, preferably mouse Ig kappa light chain signal peptide and a transmembrane region, preferably a transmembrane region derived from human PDGFR beta chain. In a very preferred embodiment said expression vector comprises nucleotides 4 to 282 of SEQ ID NO:1. In a still more preferred embodiment said expression vector is an alphaviral expression vector derived from Sindbis virus, wherein said alphaviral expression vector comprises or preferably consists ofpDel-SP-TM (SEQ ID NO:38).

In a further preferred embodiment said expression vector, preferably said alphaviral expression vector, comprises a DNA encoding a signal peptide, preferably mouse Ig kappa light chain signal peptide, a transmembrane region, preferably a transmembrane region derived from human PDGFR beta chain, and a detection tag, preferably HA. In a very preferred embodiment said expression vector, preferably said alphaviral expression vector, comprises 4 to 312 of SEQ ID NO:40. In a still further preferred embodiment said expression vector is an alphaviral expression vector derived from Sindbis virus, wherein said alphaviral expression vector comprises or preferably consists of pDel-SP-HA-TM (SEQ ID NO:39).

In a further preferred embodiment said population of isolated B cells is derived from an animal exhibiting an increased titer of antibodies specifically binding said antigen of interest. The titer of antibodies binding an antigen of interest in the blood of an animal can be determined by methods generally known in the art, e.g. by ELISA. Thus in a preferred embodiment said titer, preferably said titer in the blood of said animal, is at least 5 times, preferably at least 10 times, most preferably at least 20 times higher than in the average population of said animal, and wherein further preferably said titer can be competed away by said antigen of interest or fragment or antigenic determinant thereof.

In a further preferred embodiment said animal is or has been exposed to said antigen of interest or to a fragment or antigenic determinant thereof, wherein preferably said exposure is by way of natural exposure, infection with a pathogen or immunization. In a further preferred embodiment said animal is or has been infected by a pathogen, wherein said pathogen comprises said antigen of interest or a fragment or antigenic determinant thereof.

In a further preferred embodiment said population of isolated B cells is derived from an animal immunized with an immunogenic composition, wherein said immunogenic composition comprises or alternatively consists of: (a) said antigen of interest; (b) a fragment of said antigen of interest; and (c) an antigenic determinant of said antigen of interest. Any immunogenic composition known in the art may be used in the context of the invention. Generally preferred are compositions generating a strong immune response. Preferred immunogenic compositions are compositions comprising a virus-like particle (VLP), preferably a VLP of a RNA bacteriophage, more preferably a VLP of RNA bacteriophages Qbeta, AP205 or fr, most preferably a VLP of RNA bacteriophage Qbeta, and said antigen of interest or an antigenic determinant thereof. Immunogenic compositions useful in the context of the invention are disclosed in WO2006/097530A2, WO2006/097530A2, WO2006/045796A2, WO2006/032674A1, WO2006/027300A2, WO2005/117963A1, WO2006/063974A2, WO2004/084939A2, WO2004/085635A1, WO2005/068639A2, WO2005/108425A1, W02005/117983A2, WO2005/004907A1, WO2004/096272A2, WO2004/016282A1, WO2004/009124A2, WO2003/039225A2, WO2004/007538A2, WO2003/040164A2, WO2003/031466A2, WO2004/009116A2, and WO2003/024481A2.

In a further preferred embodiment, said immunizing of said animal is performed with an immunogenic composition, wherein the immunogenicity of said immunogenic composition is enhanced by an immunostimulatory substance, preferably by an immunostimulatory oligonucleotide, most preferably by an unmethylated CpG-containing oligonucleotide as disclosed, for example, in WO2003/024481A2, WO2005/004907A1 and WO2004/084940A1 . In a very preferred embodiment said unmethylated CpG-containing oligonucleotide is G10 (SEQ ID NO:54 of WO2005/004907A1) .

It is within the skill of the artisan to find a dosage and a mode of administration of said immunogenic compositions resulting in high antibody titers. In a preferred embodiment said immunizing of said animal with said immunogenic composition is performed by administering said immunogenic compositions to said animal at least three times, preferably three to six times, in intervals of at least one week, preferably in intervals of two weeks up to three months. In a further preferred embodiment said immunizing of said animal is performed by administering at least 100 µg, preferably 200 to 1000 µg of said immunogenic composition to said animal per single administration. In a further preferred embodiment said immunogenic composition comprises an adjuvant, preferably Freund's complete or incomplete adjuvant or alum.

In a further preferred embodiment said population of isolated B cells is derived from a source selected from: (a) blood; (b) secondary lymphoid organs, preferably spleen or lymph node; (c) bone marrow; and (d) tissue comprising memory B cells. Most preferably said source is blood. In a further preferred embodiment said population of isolated B cells comprises or preferably consists of peripheral blood mononuclear cells (PBMCs).

In a preferred embodiment, said animal is a mammal or a bird. In a preferred embodiment, said animal is selected from the group consisting of: (a) human; (b) mouse; (c) rabbit; and (d) chicken. In a very preferred embodiment, said animal is a mammal, preferably a rat, a mouse or a human. In a further preferred embodiment said animal a humanized mouse or a human, most preferably a human.

The efficiency of the screening for and cloning of antigen specific antibodies can be significantly increased by enriching antigen specific B cells. Methods for selecting from said population of isolated B cells a sub-population of B cells by selecting B cells for their capability of specifically binding said antigen of interest are generally known in the art. These methods are based on the interaction of antigen-specific B cells contained in said population of isolated B cells with the antigen of interest. In a preferred embodiment said selecting from said population of isolated B cells a sub-population of B cells comprises the steps of: (a) contacting said population of isolated B cells with said antigen of interest or a fragment or antigenic determinant thereof; and (b) selecting B cells specifically binding said antigen of interest or fragment or antigenic determinant thereof.

Preferred methods for selecting from said population of isolated B cells a sub-population of B cells are the binding of B cells to an antigen-covered carrier and FACS sorting and as described in WO2004/102198A2 . Thus, in one embodiment said selecting from said population of isolated B cells a sub-population of B cells comprises the steps of: (a) coating a carrier with said antigen of interest or fragment or antigenic determinant thereof; (b) contacting said population of isolated B cells with said carrier and allowing said B cells to bind to said carrier via said antigen of interest or fragment or antigenic determinant thereof; and (c) removing unbound B cells, wherein preferably said carrier comprises or further preferably consists of beads, wherein still further preferably said beads are paramagnetic beads.

In a preferred embodiment, said selecting from said population of isolated B cells a sub-population of B cells comprises is performed by FACS sorting, wherein preferably said selecting from said population of isolated B cells a sub-population of B cells comprises the steps of: (a) contacting said population of isolated B cells with said antigen of interest or fragment or antigenic determinant thereof, wherein said antigen of interest or fragment or antigenic determinant thereof is labeled with a fluorescence dye; and (b) separating B cells bound to said antigen of interest or fragment or antigenic determinant thereof by FACS sorting.

In a further preferred embodiment said fluorescence dye is selected from the group consisting of (a) PerCP, allophycocyanin (APC), (b) texas red, (c) rhodamine, (d) Cy3, (e) Cy5, (f) Cy5.5, (f) Cy7, (g) Alexa Fluor Dyes, preferably Alexa 647 nm or Alexa 546 nm (h) phycoerythrin (PE), (i) green fluorescent protein (GFP), (j) a tandem dye (e.g. PE-Cy5), and (k) fluorescein isothiocyanate (FITC). In a very preferred embodiment said fluorescence dye is Alexa 647 nm or Alexa 546 nm. In the context of the invention labeling of a compound, preferably of said antigen of interest or fragment or antigenic determinant thereof, with said fluorescence dye is performed by any method known in the art, preferably by direct labeling said compound by coupling said fluorescence dye to said compound, wherein said coupling may be effected via a covalent as well as a non-covalent bound. Alternatively, labeling of a compound, preferably of said antigen of interest or fragment or antigenic determinant thereof, with said fluorescence dye is performed indirectly by binding to said compound a second compound, preferably an antibody, wherein said second compound comprises said fluorescence dye.

In one preferred embodiment said antigen of interest or fragment or antigenic determinant thereof is coupled to a VLP, preferably to a VLP of a RNA bacteriophage, most preferably to a VLP of bacteriophage Qbeta, wherein said antigen of interest or fragment or antigenic determinant thereof is labeled with said fluorescence dye by binding an anti-VLP antibody to said VLP, wherein said anti-VLP antibody is labeled with said fluorescence dye, wherein preferably said anti-VLP antibody is directly labeled by said fluorescence dye or biotin/steptavidin-fluorescence-labeled.

In one preferred embodiment said antigen of interest or fragment or antigenic determinant thereof is coupled to a VLP, preferably to a VLP of a RNA bacteriophage, most preferably to a VLP of bacteriophage Qbeta, wherein said antigen of interest or fragment or antigenic determinant thereof is labeled with said fluorescence dye by binding an antibody directed against said antigen of interest or fragment or antigenic determinant thereof to said antigen of interest or fragment or antigenic determinant thereof, wherein said antibody directed against said antigen of interest or fragment or antigenic determinant thereof is labeled with said fluorescence dye, wherein preferably said antibody directed against said antigen of interest or fragment or antigenic determinant thereof is directly labeled by said fluorescence dye or biotin/steptavidin-fluorescence-labeled.

If the cloning of a certain type of immunoglobulin is intended, said sub-population of B cells may, besides the capability of said cells of specifically binding said antigen of interest, be further selected for additional markers which are specific for those types of B cells expressing immunoglobulins the cloning of which is intended. Alternatively, certain undesired types of B cells predominantly expressing undesired types of immunoglobulins may be excluded. Additionally, vitality markers such as, for example, PI (propidium iodide) oder 7-AAD (7-Amino-actinomycin) may be applied to select for vital cells. Further additionally or alternatively, cell death or apoptosis markers, such as, for example, YO-PRO-1 or Annexin V may be applied to sort out dead or apoptotic cells.

Furthermore, it is advantageous to include in said selecting from said population of isolated B cells a sub-population of B cells a positive selection for the presence of a B-cell specific marker, preferably for CD19 or B220.

In a further embodiment said selecting from said population of isolated B cells a sub-population of B cells comprises the steps of: (a) contacting said population of isolated B cells with said antigen of interest or a fragment or antigenic determinant thereof; (b) selecting B cells specifically binding said antigen of interest or fragment or antigenic determinant thereof; and (c) selecting said B cells for at least one additional parameter, wherein preferably said selection for said at least one additional parameter is (i) a positive selection for a parameter selected from presence of a B-cell specific marker, preferably CD19 or B220, and vitality of said B cells; and/or (ii) a negative selection for a parameter selected from: presence of IgM antibodies; presence of IgD antibodies, presence of cell death markers, and presence of apoptosis markers.

Typically and preferably, the cloning of immunoglobulins of the IgG class is intended and, thus, said selecting from said population of isolated B cells a sub-population of B cells further comprises the step of selecting for class switched B cells, preferably for IgM- and/or IgD-negative B cells, most preferably for IgM- and IgD-negative B cells.

In a preferred embodiment, said selecting from said population of isolated B cells a sub-population of B cells comprises the steps of: (a) contacting said population of isolated B cells with said antigen of interest or fragment or antigenic determinant thereof, wherein said antigen of interest or fragment or antigenic determinant thereof is labeled with a first fluorescence dye, wherein preferably said fluorescence dye is Alexa 647 nm, Alexa 488 or Alexa 546 nm; (b) contacting the cells of said population of isolated B cells with anti-IgM and/or anti-IgD antibodies, wherein said anti-IgM and/or anti-IgD antibodies are labeled with a second and/or a third fluorescence dye, wherein said second and/or said third fluorescence dye emits fluorescence at a wavelength which is different from the wavelength of the fluorescence emitted by said first fluorescence dye; and (c) separating B cells bound to said antigen of interest or fragment or antigenic determinant thereof but not bound to said anti-IgM and/or not bound to said anti-IgD antibodies by FACS sorting.

For the efficiency of the subsequent screening process it is very advantageous though not absolutely essential, that each cell, expressing and displaying an antibody on its surface comprises about one, preferably exactly one, single antibody species, wherein preferably each cell comprises a different antibody species. This is scenario is generally referred to as "one antibody per cell format".

A one antibody per cell format can be achieved, for example, by using a viral expression library, preferably an alphaviral expression library, and by choosing a low ratio of expression vectors per number of eukaryotic, preferably mammalian cells, when introducing said expression library into said first population of said cells. Thus, in a preferred embodiment said expression library is a viral expression library, preferably an alphaviral expression library, most preferably an alphaviral expression library derived from Sindbis virus, and said introducing said expression library into a first population of eukaryotic, preferably mammalian cells is performed by infecting said eukaryotic, preferably mammalian cell with said viral expression library, preferably with said alphaviral expression library, wherein further preferably said infecting is performed at a multiplicity of infection of at most 10, preferably at most 1, more preferably at most 0.2, and most preferably at most 0.1. In a very preferred embodiment said multiplicity of infection is 0.1.

Alternatively, a one antibody per cell format can be achieved by transfection of a plasmid library to said eukaryotic, preferably mammalian cells, wherein the transfection rate is maintained at a high level by co-transfecting a second plasmid which is not expressed in said cells. Thus, in a further embodiment said introducing said expression library, preferably a plasmid library, into a first population of eukaryotic, preferably mammalian cells is performed by transfecting said cells with said expression vectors, preferably with said expression plasmids, wherein the ration between the number of said expression vectors, preferably of said expression plasmids, and the number of said eukaryotic, preferably mammalian cells is chosen to result in approximately one expression vector, preferably one expression plasmid, per eukaryotic, preferably mammalian cell, wherein preferably the transfection rate is maintained at a high level by co-transfecting a second plasmid which is not expressed in said eukaryotic cell.

In a further embodiment said isolating of said cell is performed by FACS sorting. In a preferred embodiment said isolating of said cell comprises the steps of: (a) staining said first population of eukaryotic, preferably mammalian cells with said antigen of interest or fragment or antigenic determinant thereof, wherein said antigen of interest or fragment or antigenic determinant thereof is labeled with a fluorescence dye; and (b) separating an individual cell specifically binding said antigen of interest, or fragment or antigenic determinant thereof, by means of FACS sorting. The use of said detection tag as a component of said antibody displayed on the surface of said eukaryotic, preferably mammalian cells allows to further select only cells expressing and/or displaying an antibody. Thus, in a preferred embodiment said antibody further comprises a detection tag, wherein preferably said detection tag is HA, and said isolating of said individual cell comprises the steps of: (a) staining said first population of eukaryotic, preferably mammalian cells with a compound specifically binding to said detection tag, wherein said compound is labeled with a first fluorescence dye; (b) staining said first population of eukaryotic, preferably mammalian cells with said antigen of interest or fragment or antigenic determinant thereof, wherein said antigen of interest or fragment or antigenic determinant thereof is labeled with a second fluorescence dye, wherein said second fluorescence dye emits fluorescence at a wavelength which is different from the wavelength of the fluorescence emitted by said first fluorescence dye; and (c) separating an individual cell specifically binding said detection tag and said antigen of interest, or fragment or antigenic determinant thereof, by means of FACS sorting.

In a further embodiment said separating an individual cell specifically binding said antigen of interest, or fragment or antigenic determinant thereof, by means of FACS sorting comprises the step of further selecting said cell at least one additional parameter, wherein preferably said at least one additional parameter is selected from (i) a positive selection for vitality of said cell or the presence of a detection tag; and/or (ii) a negative selection for a parameter selected from: presence of IgM antibodies; presence of IgD antibodies, presence of cell death markers, and presence of apoptosis markers. Negative selection may also include negative selection for the binding of one or more, preferably one, undesired antigen(s). It is within the skill of the artisan to include undesired antigen(s), preferably in an unlabelled format, in the screen in order to out-compete cells expressing an antibody binding said undesired antigen(s).

In a further preferred embodiment said method further comprises the steps of: (a) cultivating at least one, preferably exactly one, of said individual cells in the presence of a second population of eukaryotic, preferably mammalian cells; (b) verifying the capability of said second population of eukaryotic, preferably mammalian cells of specifically binding said antigen of interest, or fragment or antigenic determinant thereof. In a further preferred embodiment said verifying comprises the steps of: (a) staining said second population of eukaryotic, preferably mammalian cells with said antigen of interest, or fragment or antigenic determinant thereof, wherein said antigen of interest, or fragment or antigenic determinant thereof, is labeled with a fluorescence dye; and (b) detecting cells specifically binding said antigen of interest, or fragment or antigenic determinant thereof, by FACS analysis

In a further preferred embodiment said first population of eukaryotic, preferably mammalian cells and/or, preferably and, said second population of eukaryotic, preferably mammalian cells comprises or preferably consists of cells selected from: (a) BHK 21 cells, preferably ATCC No. CCL-10; (b) Neuro-2a cells; and (c) HEK-293T cells, preferably ATCC No. CRL-11268. In a very preferred embodiment said first population of eukaryotic, preferably mammalian cells and/or, preferably and, said second population of eukaryotic, preferably mammalian cells comprises or preferably consists of BHK 21 cells, wherein further preferably said expression library is an alphaviral expression library, wherein still further preferably said alphaviral expression library is derived from Sindbis virus.

The method of the invention is by no means limited to the nature of the antigen of interest. Therefore said antigen of interest used in the method of the invention may be any antigen of known or yet unknown provenance. In one embodiment, the antigen of interest is a recombinant antigen or a synthetic peptide. In another embodiment, the antigen or antigenic determinant is isolated from a natural source. Preferred antigens of interest used in the present invention can be synthesized or recombinantly expressed and coupled to VLPs, or fused to VLPs using recombinant DNA techniques. Exemplary procedures describing the attachment of antigens to virus-like particles are disclosed in WO00/32227, in WO01/85208 and in WO02/056905 .

In a preferred embodiment said antigen of interest is selected from the group consisting of: (a) allergen; (b) self-antigen; (c) tumor antigen; (d) antigen of a pathogen; and (e) hapten.

In a further preferred embodiment said antigen of interest is an allergen, preferably an allergen selected from the group consisting of: (a) pollen allergen, preferably Bet v I (birch pollen allergen); (b) house dust allergen, preferably Der p I (House dust mite allergen); (c) cat allergen, preferably Fel d1 (d) bee venom phospholipase A2; (e) 5 Dol m V (white-faced hornet venom allergen); (f) and an immunogenic fragments of (a) to (e).

In a further preferred embodiment said antigen of interest is a self antigen, preferably a self-antigen selected from the group consisting of: (a) IL-6; (b) granulocyte macrophages colony stimulating factor (GMCSF); (c) IL-1 alpha; (d) IL-1 beta; (e) IL-5; (f) IL-15; (g) IL-23; (h) tumor necrosis factor (TNF) alpha; (i) receptor activator of nuclear factor kappaB ligand (RANKL); (j) Ghrelin; (k) GIP; (1) adiponectin receptor, (m) amyloid beta, preferably amyloid beta peptide (Aβ1-42); (n) lymphotoxins, preferably Lymphotoxin α (LT α), or Lymphotoxin β (LT β); (o) vascular endothelial growth factor (VEGF) and vascular endothelial growth factor receptor (VEGF-R); (p) MIF; (q) MCP-1; (r) SDF-1; (s) Rank-L; (t) M-CSF; (u) Angiotensin II; (v) Endoglin; (w) Eotaxin; (x) BLC; (y) CCL21; (z) IL-13; (aa) IL-17; (bb) IL-8; (cc) Bradykinin; (dd) Resistin; (ee) LHRH; (ff) GHRH; (gg) GIH; (hh) CRH; (ii) TRH; (jj) Gastrin; (kk) Interferon a; (11) Interferon γ; (mm) EGF-R; and (nn) fragments of (a) to (mm) which can be used to elicit immunological responses.

In a further preferred embodiment said antigen of interest is a tumor antigen, wherein preferably said tumor antigen is selected from the group consisting of: (a) MelanA; (b) HER2 / ErbB-2 (breast cancer); (c) GD2 (neuroblastoma); (d) EGF-R (malignant glioblastoma); (e) CEA (medullary thyroid cancer); (f) CD52 (leukemia); (g) human melanoma protein gp100; (h) tyrosinase and tyrosinase related proteins, preferably TRP-1 and TRP-2; (i) NA17-A nt protein; (j) MAGE-3 protein; and (k) NY-ESO-1.

In a further preferred embodiment said antigen of interest is an antigen of a pathogen, wherein preferably said pathogen is selected from the group consisting of: (a) hepatitis B virus; (b) influenza A virus; (c) HIV; (d) Hepatitis C virus; (e) rotavirus; (f) polio virus; (g) encephalitis virus; (h) West-Nile virus; (i) SARS virus; (j) Ebola virus; (k) Measles virus; (l) RSV; (m) Toxoplasma; (n) Plasmodium falciparum; (o) Plasmodium ovale; (p) Plasmodium malariae; and (q) Chlamydia.

In a further preferred embodiment said antigen of interest is an antigen of a pathogen, wherein preferably said antigen of interest is selected from the group consisting of: (a) hepatitis B virus preS1 protein; (b) influenza A virus M2 protein; and (b) influenza A virus HA protein.

In a further preferred embodiment said antigen of interest is a hapten, preferably a hapten selected from the group consisting of haptens of: (a) opioids; (b) morphine derivatives, preferably selected from codeine, fentanyl, heroin, morphium and opium; (c) stimulants, preferably selected from amphetamine, cocaine, MDMA (methylenedioxymethamphetamine), methamphetamine, methylphenidate and nicotine; (d) hallucinogens, preferably LSD, mescaline, psilocybin, and cannabinoids. In a very preferred embodiment said antigen of interest is a hapten of nicotine or of a nicotine derivative.

Said individual cell displaying said antibody of interest can then be used to clone and to recombinantly express antibodies comprising the variable regions of said antibody displayed on said cell using methods generally known in the art (see for example Weitkamp et al., 2003, J. Immunol. Meth. 275, 223-237). In principle, it is possible to express said antibodies in any know form (for different forms of antibodies see Hollinger & Hudson (2005), Nature Biotechnology 23(9)), preferably as IgG, most preferably as fully human IgG.

One possibility of producing recombinant antibodies specifically binding said antigen of interest is to express said antibody as a fusion product comprising a purification tag. For example, the expression of single chain antibodies as an Fc-fusion has been described in Ray et al. (2001), Clin. Exp. Immunol. 125(1):94-101 and Ono et al. (2003), J. Biosci. Bioeng. 95(3):231-238). The description therefore provides for a method of producing an antibody specifically binding an antigen of interest said method comprising the steps of: (a) isolating a cell expressing an antibody according to the method described above; (b) obtaining RNA from said isolated cell; (c) synthesizing cDNA encoding said antibody from said RNA; (d) cloning said cDNA into an expression vector; (e) generating a fusion construct encoding a fusion product comprising said antibody and said purification tag; (f) expressing said fusion product in a cell; and (g) purifying said fusion product. In a further preferred embodiment said antibody comprises at least one VR, preferably a LCVR and a HCVR, and a purification tag, wherein preferably said at least one VR, more preferably said LCVR and said LCVR, are derived from the same of said individual cell. In a preferred embodiment said synthesizing of said cDNA comprises the step of synthesizing single stranded cDNA from said RNA, wherein preferably said single stranded cDNA is synthesized using SEQ ID NO:35 as a primer. In a further preferred embodiment said synthesizing of said cDNA further comprises the step of amplifying said cDNA from said single stranded cDNA, wherein preferably said amplifying is performed using the oligonucleotides of SEQ ID NO:35 and SEQ ID NO:36 as primers. In a still further preferred embodiment said purification tag is Fc, preferably human Fc, and wherein further preferably said purification tag comprises or still further preferably consists of SEQ ID NO:109. In a very preferred embodiment said expression vector is pCEP-SP-Sfi-Fc (SEQ ID NO:37). In a further preferred embodiment said expressing of said fusion product is performed in mammalian cells, preferably in HEK-293T cells. Antibodies comprising a purification tag can be expressed an purified using standard procedures and are preferably used to test the specificity of said antibody for said antigen of interest of fragment or antigenic determinant thereof by determining the binding constant of said antibody to said antigen of interest or fragment or antigenic determinant thereof, wherein said testing is preferably performed by ELISA, most preferably by ELISA essentially as described in Example 7.

The description further provides a method of producing an antibody specifically binding an antigen of interest by expressing said antibody as an immunoglobulin, preferably as a species specific immunoglobulin, most preferably as a mouse, rat, rabbit chicken or human immunoglobulin, most preferably as a fully human immunoglobulin. One embodiment of the invention is a method of producing an antibody specifically binding an antigen of interest, said method comprising the steps of: (a) isolating a cell expressing an antibody according to the method described above; (b) obtaining RNA from said cell; (c) synthesizing cDNA form said RNA; (d) amplifying from said cDNA a DNA encoding VRs of said antibody expressed by said cell; (e) generating an expression construct comprising said DNA, wherein said expression construct is encoding at least one VR of said antibody expressed by said cell; (f) expressing said expression construct in a cell. In a preferred embodiment, said method comprising the steps of: (a) isolating a cell expressing an antibody according to the method described above; (b) obtaining RNA from said cell; (c) synthesizing cDNA form said RNA; (d) amplifying from said cDNA a first DNA encoding a HCVR of said antibody expressed by said cell; (e) generating a first expression construct comprising said first DNA, wherein said first expression construct is encoding a heavy chain immunoglobulin comprising a heavy chain constant region (HCCR) and said HCVR; (f) amplifying from said cDNA a second DNA encoding a LCVR of said antibody expressed by said cell; (g) generating a second expression construct comprising said second DNA, wherein said second expression construct is encoding a light chain immunoglobulin comprising a light chain constant region (LCCR) and said LCVR; (h) expressing said first expression construct and said second expression construct in a cell. In a further preferred embodiment said HCCR, said HCVR, said LCCR and said LCVR are derived from human.

In a further preferred embodiment said expression construct, said first expression construct and/or said second expression construct are further encoding a hydrophobic leader sequence, preferably a species specific hydrophobic leader sequence, most preferably a human hydrophobic leader sequence. In a further preferred embodiment said first expression construct is further encoding a human heavy chain hydrophobic leader sequence. In a further preferred embodiment said second expression construct is further encoding a human light chain hydrophobic leader sequence, wherein said human light chain hydrophobic leader sequence is selected from the group consisting of (a) human kappa light chain hydrophobic leader sequence; and (b) human lambda light chain hydrophobic leader sequence.

In a further preferred embodiment said synthesizing of said cDNA comprises the step of synthesizing single stranded cDNA from said RNA, wherein preferably said single stranded cDNA is synthesized using SEQ ID NO:35 as a primer. In a further preferred embodiment said synthesizing of said cDNA further comprises the step of amplifying said cDNA from said single stranded cDNA, wherein preferably said amplifying is performed using the oligonucleotides of SEQ ID NO:35 and SEQ ID NO:36 as primers.

In a further preferred embodiment said HCCR is a human HCCR, preferably a human HCCR selected from the group consisting of: (a) human gamma 1 HCCR; (b) human gamma 2 HCCR; (c) human gamma 4 HCCR; and (d) human heavy chain Fd regions, preferably gamma 2 Fd region. In a further preferred embodiment said LCCR is a human LCCR, preferably a human LCCR selected from the group consisting of: (a) human kappa LCCR; and (b) human lambda LCCR.

In a further preferred embodiment said amplifying of said first DNA is performed with HCVR specific primers, wherein preferably said HCVR specific primers are SEQ ID NO:102 and SEQ ID NO:103.

In a further preferred embodiment said amplifying of said second DNA is performed with LCVR specific primers, wherein preferably said LCVR specific primers are selected from kappa LCVR specific primers and lambda LCVR specific primers. In a further preferred embodiment said LCVR specific primers are kappa LCVR specific primers, wherein preferably said kappa LCVR specific primers are a combination of any one selected from SEQ ID NO:92 or 93 with SEQ ID NO:94. In a further preferred embodiment said LCVR specific primers are lambda LCVR specific primers, wherein preferably said lambda LCVR specific primers are a combination of any one selected from SEQ ID NO:95 to 99 with any one of SEQ ID NO:100 or 101.

In a further preferred embodiment said LCCR is a human kappa LCCR and wherein said LCVR is a human kappa LCVR. In a further preferred embodiment said LCCR is a human lambda LCCR and wherein said LCVR is a human lambda LCVR.

In principle, immunoglobulins comprising a heavy and a light chain can be recombinantly produced by expressing two different expression vectors in the same cell. Alternatively, expression constructs encoding said light chain and said heavy chain can be cloned into a single expression vector. Thus, in one embodiment said expressing of said first expression construct and of said second expression construct comprises expressing said first expression construct as part of a first expression vector and expressing said second expression construct as part of a second expression vector, wherein said first expression vector and said second expression vector are co-transfected to said cell. In a preferred embodiment said expressing of said first expression construct and of said second expression construct comprises expressing said first expression construct and said second expression construct as part of the same expression vector, wherein preferably said expression vector is pCB15 (SEQ ID NO:104).

For the expression of species specific, preferably human, antibodies expression cassettes are produced encoding HCCRs or LCCRs of said species, preferably of humans, and the corresponding leader sequences and comprising a restriction site allowing to insert the corresponding VR coding regions. In a preferred embodiment said generating said first expression construct comprises the step of cloning said first DNA into a first expression cassette, wherein said first expression cassette is encoding said HCCR, and, preferably, said HCCR hydrophobic leader sequence, wherein further preferably said first expression cassette comprises or still more preferably consists of a sequence selected from SEQ ID NO:117 to 120. In a further preferred embodiment said generating said second expression construct comprises the step of cloning said second DNA into a second expression cassette, wherein said second expression cassette is encoding said LCCR, and, preferably, said LCCR hydrophobic leader sequence, and wherein further preferably said second expression cassette comprises or still more preferably consists of a sequence selected from SEQ ID NO:121 or 122.

In one embodiment said antibody is expressed in a form selected from: (a) single chain antibody, preferably scFv; (b) diabody; (c) Fab fragment; (d) F(ab')2 fragment; and (e) whole antibody, preferably selected from IgG, IgA, IgE, IgM, and IgD; wherein preferably said antibody is a human antibody, most preferably a fully human antibody.

In a preferred embodiment said antibody is a Fab fragment, wherein preferably said first expression cassette comprises or preferably consists of SEQ ID NO:120 and wherein further preferably said second expression cassette comprises or preferably consists of SEQ ID NO:121. In a further preferred embodiment said antibody is a Fab fragment, where in said first expression vector comprises or preferably consists of SEQ ID NO:85 and wherein said second expression vector comprises or preferably consists of said SEQ ID NO:71.

In a further preferred embodiment said antibody is a Fab fragment, wherein preferably said first expression cassette comprises or preferably consists of SEQ ID NO:120 and wherein further preferably said second expression cassette comprises or preferably consists of SEQ ID NO:122. In a further preferred embodiment said antibody is a Fab fragment and said first expression vector comprises or preferably consists of SEQ ID NO:85 and said second expression vector comprises or preferably consists of said SEQ ID NO:110.

In another embodiment said antibody is expressed as a whole antibody of the IgG class, preferably as IgG1, IgG2, IgG3, or IgG4; wherein preferably said antibody is a human antibody, most preferably a fully human antibody.

In a preferred embodiment said antibody is a IgG1, and wherein preferably said first expression cassette comprises or preferably consists of SEQ ID NO:118 and wherein further preferably said second expression cassette comprises or preferably consists of SEQ ID NO:121. In a further preferred embodiment said first expression vector comprises or preferably consists of SEQ ID NO:88 and wherein said second expression vector comprises or preferably consists of said SEQ ID NO:71. In a further preferred embodiment said antibody is a IgG1, and wherein preferably said first expression cassette comprises or preferably consists of SEQ ID NO:118 and wherein further preferably said second expression cassette comprises or preferably consists of SEQ ID NO:122. In a further embodiment said first expression vector comprises or preferably consists of SEQ ID NO:88 and wherein said second expression vector comprises or preferably consists of said SEQ ID NO:110.

In a further preferred embodiment said antibody is a IgG2, and wherein preferably said first expression cassette comprises or preferably consists of SEQ ID NO:117 and wherein further preferably said second expression cassette comprises or preferably consists of SEQ ID NO:121. In a further preferred embodiment said first expression vector comprises or preferably consists of SEQ ID NO:78 and wherein said second expression vector comprises or preferably consists of said SEQ ID NO:71. In a further preferred embodiment said antibody is a IgG2, and wherein preferably said first expression cassette comprises or preferably consists of SEQ ID NO:117 and wherein further preferably said second expression cassette comprises or preferably consists of SEQ ID NO:122. In a further preferred embodiment said first expression vector comprises or preferably consists of SEQ ID NO:78 and wherein said second expression vector comprises or preferably consists of said SEQ ID NO:110.

In a further preferred embodiment said antibody is a IgG4, and wherein preferably said first expression cassette comprises or preferably consists of SEQ ID NO:119 and wherein further preferably said second expression cassette comprises or preferably consists of SEQ ID NO:121. In a further preferred embodiment said first expression vector comprises or preferably consists of SEQ ID NO:90 and wherein said second expression vector comprises or preferably consists of said SEQ ID NO:71. In a further preferred embodiment said antibody is a IgG4, and wherein preferably said first expression cassette comprises or preferably consists of SEQ ID NO:119 and wherein further preferably said second expression cassette comprises or preferably consists of SEQ ID NO:122. In a further preferred embodiment said first expression vector comprises or preferably consists of SEQ ID NO:90 and wherein said second expression vector comprises or preferably consists of said SEQ ID NO:71.

Said expressing of said antibody may be performed in any eukaryotic expression system known in the art. Typically and preferably, said expressing of said antibody is performed in eukaryotic cells, wherein further preferably said eukaryotic, cells are selected from yeast cells, insect cells and mammalian cells. In a preferred embodiment said expressing of said antibody is performed in mammalian cells, wherein preferably said mammalian cells are selected from HEK-293T cells, CHO cells, COS cells. Very preferably said mammalian cells are HEK-293T cells.

The description discloses an expression vector for displaying polypeptides, preferably antibodies, most preferably single chain antibodies, on the surface of a eukaryotic, preferably mammalian cell; and an expression vector, preferably a viral expression vector, more preferably alphaviral expression vector, most preferably an expression vector derived from Sindbis virus, wherein said expression vector comprises DNA elements encoding a signal peptide, a transmembrane region and, preferably, a detection tag, and wherein further preferably said expression vector comprises a restriction site allowing the cloning, preferably the orientation specific cloning, of DNA molecules encoding said polypeptides, preferably said antibody variable regions, into said expression vector. In a further preferred embodiment said expression vector comprises said DNA elements and said restriction site in an orientation allowing the expression of a fusion protein comprising from the N- to the C-terminus said signal peptide, said polypeptide, preferably said detection tag, and said transmembrane region.

In a preferred embodiment said signal peptide is mouse Ig kappa light chain signal peptide. In a further preferred embodiment said transmembrane region is derived from human PDGFR beta chain. In a further preferred embodiment said signal peptide is mouse Ig kappa light chain signal peptide and said transmembrane region is derived from human PDGFR beta chain. In a very preferred embodiment said expression vector comprises nucleotides 4 to 282 of SEQ ID NO:1. In a still more preferred embodiment said expression vector is an alphaviral expression vector derived from Sindbis virus, wherein said alphaviral expression vector comprises or preferably consists of pDel-SP-TM (SEQ ID NO:38).

In a further preferred embodiment said detection tag is HA. In a further preferred embodiment said signal peptide is mouse Ig kappa light chain signal peptide, said transmembrane region is derived from human PDGFR beta chain and said detection tag is HA. In a very preferred embodiment said expression vector comprises nucleotides 4 to 312 of SEQ ID NO:40. In a still further preferred embodiment said expression vector is an alphaviral expression vector derived from Sindbis virus, wherein said alphaviral expression vector comprises or preferably consists of pDel-SP-HA-TM (SEQ ID NO:39).

The description further discloses an expression library, preferably to an expression library expressing antibodies, wherein further preferably said antibodies are single chain antibodies, wherein still further preferably said single chain antibodies are human single chain antibodies, said expression library comprising said expression vector. In a preferred embodiment, said expression library comprises nucleotides 4 to 282 of SEQ ID NO:1 or, further preferably, said expression library comprises SEQ ID NO:38. In a further preferred embodiment, said expression library comprises nucleotides 4 to 312 of SEQ ID NO:40 or, further preferably, said expression library comprises SEQ ID NO:39.

The description further discloses an eukaryotic, preferably mammalian cell comprising said expression vector or comprising at least one specimen of said expression library.

### EXAMPLES

### EXAMPLE 1

### Construction of pDel-SP-TM, a Sindbis-based viral vector allowing cell surface display of single-chain antibodies

A DNA fragment (SEQ ID NO:1) encoding a mouse Ig kappa signal peptide (SEQ ID NO:105), two Sfi1 restriction sites and the transmembrane region of the human platelet-derived growth factor receptor beta chain (PDGFR; SEQ ID NO:106) was assembled from six overlapping oligonucleotides. Briefly, the oligonucleotides SPTM-2 (5'-CCT GCT ATG GGT ACT GCT GCT CTG GGT TCC AGG TTC CAC TGG TGA CTA TGA GGC CCA GGC GGC CGG TAC-3', SEQ ID NO:26), SPTM-3 (5'-CCT CCT GCG TGT CCT GGC CCA CAG CAT TGC GGC CGG CCT GGC CGC TAG CGG TAC CGG CCG CCT GGG CCT C-3', SEQ ID NO:27), SPTM-4 (5'-GGC CAG GAC ACG CAG GAG GTC ATC GTG. GTG CCA CAC TCC TTG CCC TTT AAG GTG GTG GTG ATC TCA GCC-3', SEQ ID NO:28) and SPTM-5 (5'-CAT GAT GAG GAT GAT AAG GGA GAT GAT GGT GAG CAC CAC CAG GGC CAG GAT GGC TGA GAT CAC CAC CAC C-3' SEQ ID NO:29) were mixed at a final concentration of 0.1 µM each in a 100 µl polymerase chain reaction (PCR) and cycled 20 times (20 sec at 94 °C; 20 sec at 60 °C; 40 sec at 72 °C) in the presence of 2.5 units Taq DNA polymerase (Invitrogen) under the manufacturer's recommended reaction conditions. 1 µl of this reaction was then mixed with the oligonucleotides SPTM-1 (5'-GAG TCT AGA GCC ACC ATG GAG ACA GAC ACA CTC CTG CTA TGG GTA CTG CT GCT C-3', SEQ ID NO:30) and SPTM-6 (5'-CTC GGG CCC CTA ACG TGG CTT CTT CTG CCA AAG CAT GAT GAG GAT GAT AAG GGA G-3', SEQ ID NO:31) at a final concentration of 0.1 µM each in a second 100 µl PCR reaction and cycled for another 20 cycles as above. The resulting 285 bp DNA fragment was digested with the restriction endonucleases XbaI and ApaI, purified by agarose gel electrophoresis, and ligated into the XbaI/ApaI digested Sindbis virus expression vector pDelSfi, yielding the scFv display vector pDel-SP-TM (SEQ ID NO:38).

For the construction of pDel-SP-HA-TM (SEQ ID NO:39), a 315 bp DNA fragment (SP-HA-TM Linker, SEQ ID NO:40), which in addition encodes a haemagglutinin (HA) tag between the SfiI sites and the TM region, was assembled and cloned. The whole procedure was identical to the one for pDel-SP-TM (SEQ ID NO:38), except that the oligo SPTM-3 (SEQ ID NO:27) was replaced by the oligo SPTM-3HA (5'-CCT CCT GCG TGT CCT GGC CCA CAG CAT TAG AGG CAT AAT CTG GCA CGT CGT AAG GAT AGC GGC CGG CCT GGC CGC TAG CGG TAC CGG CCG CCT GGG CCT G-3', SEQ ID NO:41).

### EXAMPLE 2

### Isolation of Qβ-specific human memory B cells from peripheral blood mononuclear cells

Peripheral blood mononuclear cells (PBMC) were isolated from 20 ml of heparinized blood of a Qβ-vaccinated volunteer by a standard Ficoll-HypaqueTM Plus ((Amersham Biosciences) gradient method. PBMC were stained with Alexa 647 nm-labeled Qβ (4 µg/ml), FITC-labeled mouse anti-human IgM (1.5 µg/ml) (Jackson ImmunoResearch Laboratories), FITC-labeled mouse anti-human IgD (diluted 1:50) (BD Biosciences Pharmingen), and PE-labeled mouse anti-human CD 19 (diluted 1:100) (BD Biosciences Pharmingen). After 30 min cells were washed, filtered and stained with propidium iodide (PI) to exclude dead cells. 230 Qβ-specific memory B cells (Qβ-, CD19-positive, IgM-, IgD-, PI-negative) were sorted on a FACSVantage SE flow cytometer (Becton Dickinson) and used for library construction.

### EXAMPLE 3

### Construction of a single-chain antibody cell surface display library from Qβ-specific human memory B cells

Total RNA was isolated from 230 Qβ-specific human memory B cells using TRI reagent (Molecular Research, Inc.). Single-stranded cDNA was produced with PowerScriptTM reverse transcriptase (Clontech) using the template switch protocol (Zhu et al. 2001 Biotechniques 30(4):892-7), with the CDS oligonucleotide (5'-AAG CAG TGG TAA CAA CGC AGA GTA CTT TTT TTT TTT TTT TTT TTT TTT TTT TTT TVN-3', SEQ ID NO:32) as primer, and the SMART II oligonucleotide (5'-d[AAG CAG TGG TAA CAA CGC AGA GTA CGC] r[GGG]-3', SEQ ID NO:33) as switch template. The cDNA was bulk-amplified by 14 cycles of PCR, using the Advantage2 polymerase mix (Clontech) and an anchor primer (5'-AAG CAG TGG TAT CAA CGC AGA GT-3', SEQ ID NO:34) in a total volume of 200 µl. Double-stranded cDNA was purified with the Qiaquick PCR purification kit (Qiagen).

A single-chain antibody library was then produced essentially as described (Phage Display: A Laboratory Manual, Cold Spring Harbor Laboratory Press, 2001) using the pre-amplified ds-cDNA as template. Briefly, heavy chain variable region coding sequences were amplified with an equimolar mix of 6 sense primers (HSCVH1-FL, SEQ ID NO:42; HSCVH2-FL, SEQ ID NO:43; HSCVH3a-FL, SEQ ID NO:44; HSGVH4a-FL, SEQ ID NO:45; HSCVH4-FL, SEQ ID NO:46; and HSCVH35-FL, SEQ ID NO:47) plus an antisense constant region primer (HSCG1234-B; SEQ ID NO:48); the κ light chain variable region. coding sequences were amplified with an equimolar mix of 4 sense primers (HSCK1-F, SEQ ID NO:49; HSCK24-F, SEQ ID NO:50; HSCK3-F, SEQ ID NO:51; and HSCK5-F, SEQ ID NO:52) plus an equimolar mix of 4 antisense primers (HSCJK14o-B, SEQ ID NO:53; HSCJK2o-B, SEQ ID NO:54; HSGJK3o-B SEQ ID NO:55; and HSCJKL5o-B, SEQ ID NO:56); and the λ light chain variable region coding sequences were amplified with an equimolar mix of 9 sense primers (HSCLam1a, SEQ ID NO:57; HSCLamlb, SEQ ID NO:58; HSCLam2, SEQ ID NO:59; HSCLam3, SEQ ID NO:60; HSCLam4, SEQ ID NO:61; HSCLam6, SEQ ID NO:62; HSCLam78, SEQ ID NO:63; HSCLam9, SEQ ID NO:64; and HSCLam10 SEQ ID NO:65) plus an equimolar mix of 3 antisense primers (HSCJLam1236, SEQ ID NO:66; HSCJLam4, SEQ ID NO:67 and HSCJLam57, SEQ ID NO:68).

The scFv coding regions were assembled by PCR overlap extension of the VH PCR product with either the the Vκ PCR product or the Vλ PCR product using the primers RSC-F (SEQ ID NO:69) and RSC-B (SEQ ID NO:70). The resulting ∼750-800 bp PCR products encoded a 5' light chain variable region (either κ or λ) and a 3' heavy chain variable region, linked by an 18 amino acid flexible linker, and flanked by two SfiI restriction sites. The κ- and λ-containing scFv fragments were pooled in equimolar ratio, digested with the restriction endonuclease SfiI, purified by agarose gel electrophoresis and cloned into SfiI-digested pDel-SP-TM (SEQ ID NO:38). The resulting library consisted of approximately 10⁶ independent transformants. DNA was isolated from pooled colonies using the HiSpeed Plasmid Maxi Kit (Qiagen).

As a measure of library quality, individual clones were sequenced to ascertain diversity and overall structural organization of the single-chain antibodies, as well as their in frame fusion to the N-terminal Ig κ signal peptide and C-terminal PDGFR transmembrane region. Of the six scFv clones that were sequenced each corresponded to a different scFv, indicating that the library is diverse (SEQ ID NOs:2-7). Further, all six clones were fused in-frame to both signal peptide and transmembrane region. In addition, most of the clones displayed an intact open reading frame, with only one clone having an in-frame stop codon in the heavy chain variable region as a result of a point mutation. This is likely to be a PCR mutation resulting from the extensive amplification during library construction. In conclusion, the scFv cell surface display library was diverse and predominantly consisted of functional antibodies that can be expected to be displayed on the cell surface.

The plasmid library was converted into a Sindbis virus library as follows. For in vitro transcription, 5 µg of the library plasmid was linearized, half with the restriction endonuclease NotI (Roche), the other half with PacI (New England Biolabs). 5 µg of the helper plasmid pDHEB (Bredenbeek et al. 1993 J Virol. 67(11):6439-6446), encoding the Sindbis virus structural proteins, was linearized with the restriction endonuclease EcoRI. All restriction digests were then extracted with phenol-chloroform, ethanol precipitated, and resuspended in RNase-free H₂O at a concentration of 0.5 µg/µl. 1 µg of the linearized library and of the helper plasmid were subjected to SP6 RNA polymerase-mediated in vitro transcription in a volume of 20 µl, using the mMessage mMachineTM kit (Ambion). The transcribed library RNA was co-electroporated with an equimolar amount of helper RNA into 10⁷ BHK cells. 18 hours post transfection, cell supernatant was harvested and the viral titer determined to be approximately 10⁷ per ml. This Sindbis virus based cell surface display library was then used to isolate Qβ-specific single-chain antibodies.

### EXAMPLE 4

### Identification of cells displaying Qβ-specific single-chain antibodies by fluorescence-activated cell sorting

Sixty million subconfluent (80 %) baby hamster kidney (BHK) cells were infected with the single-chain antibody library derived from Qβ-specific variable domains or an empty viral vector as a negative control at a multiplicity of infection (MOI) of 0.2. After 5 hours, cells were detached with cell dissociation buffer (Sigma), washed and stained. Half of the cells were stained with Alexa 647 nm-labeled Uβ (4 µg/ml) for 30 min. The remaining cells were stained with Alexa 546 nm-labeled Qβ (4 µg/ml) and an anti-sindbis serum from rabbit (diluted 1:6000) for 30 min, followed by staining with Cy5-labeled donkey anti-rabbit IgG (1 µg/ml) (Jackson ImmunoResearch Laboratories) for 20 min. All cells were then washed, filtered and stained with propidium iodide (PI) to exclude dead cells. Single cell sorting was performed on a FACS Vantage SE flow cytometer (Becton Dickinson) for, respectively, Alexa 647 nm-positive, PI-negative and, Alexa 546 nm-positive, sindibis-positive, PI-negative cells. In total, 480 cells were sorted, 264 from the Alexa 647 nm sorting, and 216 from the Alexa 546 nm sorting.

Each cell was sorted into a well of a 24-well plate containing 50 % confluent BHK feeder cells. Upon virus spread (2-3 days post sorting), the infected cells were tested by FACS analysis for Qβ binding. On day 2 post sorting, 228 wells showed typical signs of viral infection, 199 of which bound Qβ. On day 3 post sorting, another 48 wells showed clear viral infection with 39 of them binding Qβ.

### EXAMPLE 5

### Rescue of cDNA encoding Qβ-specific single-chain antibodies

To obtain cDNAs encoding Qβ-specific single-chain antibodies, RT-PCR was performed using supernatants from BHK cells, each containing monoclonal recombinant Sindbis virus. For the viral RNA isolation, 140 µl of viral supernatant and the QIAamp Viral RNA Kit (Qiagen) were used. The procedure was performed according to manufacturer's protocol and the RNA was dissolved in 30 µl RNase-free H₂O. For the cDNA synthesis 8 µl of the viral RNA were used per reaction. The 1st strand cDNA was synthesized in a 20 µl reaction containing 20 pmoles LPP2 primer (5'- ACA AAT TGG ACT AAT CGA TGG C-3', SEQ ID NO:35), using PowerScriptTM reverse transcriptase (Clontech) according to the manufacturer's recommendations.

Single-chain antibody cDNAs were PCR amplified from 2 µl 1st strand cDNA in 100 µl reactions with the primers pDel-seq (5'-GAG CAA AAG AGC ATT CCA AG-3', SEQ ID NO:36) and LPP2 (SEQ ID NO:35), using the Advantage 2 Polymerase mix (Clontech) according to the manufacturer's recommendations. The PCR reaction was performed with one cycle of 1 min at 95 °C followed by 30 cycles of 20 sec at 95 °C, 20 sec at 56 °C, 90 sec at 72 °C. The resulting PCR products were analyzed on an agarose gel and the ∼750-800 bp bands isolated using the QIAquick gel extraction Kit (Qiagen) according to manufacturer's protocol. Each gel-purified PCR product was then subjected to sequencing using the primers pDel-seq and LPP2 (∼100-200 ng per sequencing reaction).

A total of 14 PCR products were sequenced, the scFv coding regions assembled and the sequences predicted for the displayed scFvs determined (SEQ ID NOs:8-21). With the exception of one clone, each of the single-chain antibodies had an open reading frame and was fused in-frame to both signal peptide and transmembrane region, as was to be expected. ScFv-Qb#18 (SEQ ID NO:18) had a frame shift at the beginning of the heavy chain variable region followed by an early termination, leading to a protein lacking not only most of the heavy chain V region, but also the transmembrane region. Such a protein is expected to be secreted and should not be selected by our cell surface display strategy. Thus, it seems likely that the mutation was introduced during the gene rescue PCR amplification.

The sequence diversity was significantly reduced compared to prior to the screen. While there were no two scFvs with identical sequence, many were clearly closely related. Significantly, there were several scFvs where one of the two variable regions were identical. For instance, scFv-Qb#2 (SEQ ID NO:8), scFv-Qb#3 (SEQ ID NO:9), scFv-Qb#4 (SEQ ID NO:10) and scFv-Qb#6 (SEQ ID NO:12) share the same heavy chain variable region. Similarly, the light chain variable regions of scFv-Qb#2 (SEQ ID NO:8), scFv-Qb#5 (SEQ ID NO:11) and scFv-Qb#7 (SEQ ID NO:13) are almost identical and differ by only one or a few amino acids.

### EXAMPLE 6

### Construction, expression, and purification of the Qβ-specific scFv-Fc fusion proteins

Synthetic constructs were produced allowing for the eukaryotic expression of fusion proteins carrying an N-terminal human scFv fused to a C-terminal human Fc-γ1 domain. Thus, PCR products corresponding to scFv-Qb#2 (SEQ ID NO:8), scFv-Qb#3 (SEQ ID NO:9), scFv-Qb#5 (SEQ ID NO:11) and scFv-Qb#8 (SEQ ID NO:14) were digested with the restriction endonuclease SfiI (New England Biolabs) and cloned into the expression vector pCEP-SP-Sfi-Fc (SEQ ID NO:37). This vector is a derivative of the episomal mammalian expression vector pCEP4 (Invitrogen, cat. no. V044-50), carrying the Epstein-Barr Virus replication origin (oriP) and nuclear antigen (encoded by the EBNA-1 gene) to permit extrachromosomal replication, and contains a puromycin selection marker in place of the original hygromycin B resistance gene. The resulting plasmids, pCEP/scFvQb#2-Fc, pCEP/scFvQb#3-Fc, pCEP/scFvQb#5-Fc and pCEP/scFvQb#8-Fc drive expression of scFv-Fc domain fusion proteins (SEQ ID NOs:22-25) under the control of a CMV promoter.

Expression of the fusion constructs was done in HEK-293T cells. One day before transfection, 10⁷ HEK-293T cells were plated onto a 14 cm tissue culture plate for each protein to be expressed. Cells were then transfected with the respective scFv-Fc fusion construct using Lipofectamin Plus (Invitrogen) according to the manufacturer's recommendations, incubated one day, and replated on three 14 cm dishes in the presence of 1 µg/ml puromycin. After 3 days of selection, puromycin-resistant cells were transferred to six Poly-L-Lysine coated 14 cm plates and grown to confluency. Medium was then replaced by serum-free medium and supernatants containing the respective scFv-Fc fusion protein was collected every 3 days and filtered through a 0.22 µM Millex GV sterile filter (Millipore).

For each of the scFv-Fc fusion proteins, the consecutive harvests were pooled and applied to a protein A-sepharose column. The column was washed with 10 column volumes of phosphate-buffered saline (PBS), and bound protein eluted with 0.1 M Glycine pH 3.6. 1 ml fractions were collected in tubes containing 0.1 ml of 1 M Tris pH 7.5 for neutralization. Protein-containing fractions were analyzed by SDS-PAGE and pooled. The buffer was exchanged with PBS by dialysis using 10'000 MWCO Slide-A-Lyzer dialysis cassettes (Pierce). The purified proteins in PBS were then filtered through 0.22 µM Millex GV sterile filters (Millipore) and aliquotted. Working stocks were kept at 4°C, whereas aliquots for long-term storage were flash-frozen in liquid nitrogen and kept -80°C.

### EXAMPLE 7

### Verification of Qβ-specific binding of scFv-Fc fusion proteins by ELISA

ELISA plates (96 well MAXIsorb, NUNC immuno plate 442404) were coated with Qβ at a concentration of 2 µg/ml in coating buffer (0.1 M NaHCO₃, pH 9.6), over night at 4 °C. Alternatively, ELISA plates were coated with 2 µg/ml of an irrelevant control protein. The plates were then washed with wash buffer (PBS/0.05 % Tween) and blocked for 1 h at 37 °C with 3 % BSA in wash buffer. The plates were then washed again and incubated with serially diluted scFv-Qb#2-Fc (SEQ ID NO:8), scFv-Qb#3-Fc (SEQ ID NO:9), scFv-Qb#5-Fc (SEQ ID NO:11) and scFv-Qb#8-Fc (SEQ ID NO:14) (either serum-free tissue culture supernatant or purified scFv-Fc fusion proteins). Plates were incubated at 37 °C for 1 h and then extensively washed with wash buffer. Bound specific scFv-Fc fusion proteins were then detected by a 30 minute incubation with a HRPO-labeled, Fcγ-specific, goat anti-human IgG antibody (Jackson ImmunoResearch Laboratories 109-035-098). After extensive washing with wash buffer, plates were developed with OPD solution (1 OPD tablet, 25 µl OPD buffer and 8ul H2O2) for 5 to 10 minutes and the reaction was stopped with 5 % H₂SO₄ solution. Plates were then read at OD 450 nm on an ELISA reader (Biorad Benchmark). Half-maximal binding of purified scFv-Fc fusion proteins was observed at picomolar concentrations (scFv-Qb#2-Fc, 51 pM; scFv-Qb#3-Fc, 35 pM; scFv-Qb#5-Fc, 52 pM; scFv-Qb#8-Fc, 163 pM), suggesting that the antibodies are of very high affinity.

### ExAMPLE 8

### Construction of vectors allowing for expression of human antibodies as whole IgG or Fab.

pCMV-LC (SEQ ID NO:71), a vector allowing for the expression of natural human antibody κ light chains, was generated as follows. First, a DNA segment encoding an Ig κ light chain signal peptide was assembled from the 4 oligonucleotides SP-kappa-1 (5'-GGC TAG CGC CAC CAT GGA CAT GAG GGT CCC CGC TCA GCT CCT GGG GCT C-3', SEQ ID NO:72), SP-kappa-2 (5'-CAG GAG CTG AGC GGG GAC CCT CAT GTC CAT GGT GGC GCT AGC CAG CT-3', SEQ ID NO:73), SP-kappa-3 (5'-CTG CTA CTC TGG CTC CGA GGT GCC AGA TGT GAC ATC GAG CTC CTG CA-3', SEQ ID NO:74) and SP-kappa-4 (5'-GGA GCT CGA TGT CAC ATC TGG CAC CTC GGA GCC AGA GTA GCA GGA GCC C-3', SEQ ID NO:75), by annealing the complementary oligonucleotides SP-kappa-1 and -2, and SP-kappa-3 and -4, respectively. The two resulting double stranded DNA fragments SP-kappa-1/2 and SP-kappa-3/4 were cloned into the vector pCMV-Script (Stratagene) digested with the restriction endonucleases SacI and PstI, yielding pCMV-kappa-leader. Second, the human κ light chain constant region was amplified from human spleen cDNA using the primers C-kappa-F (5'-GAG GAG GAT ATC AAA CGA ACT GTG GCT GCA CCA TC-3', SEQ ID NO:76) and C-kappa-B (5'-GAG GAG GGT ACC GTT TAA ACC TAA CAC TCT CCC CTG TTG AAG CTC TTT GTG ACG GGC GAA CTC AGG CC-3', SEQ ID NO:77). The resulting 359 bp PCR product was digested with the restriction endonucleases EcoRV and KpnI and cloned into the vector pCMV-Script, yielding pCMV-C-kappa. Third, after the correct sequence of both plasmids was verified, pCMV-kappa-leader and pCMV-C-kappa were digested with the restriction endonucleases EcoRV and KpnI. The 343 bp fragment excised from pCMV-C-kappa, corresponding to the κ light chain constant region, was then ligated into the 4282 bp pCMV-kappa-leader vector fragment, yielding the light chain expression vector pCMV-LC (SEQ ID NO:71). DNA fragments encoding light chain variable regions can be cloned into pCMV-LC via the restriction endonucleases SacI and EcoRV and expressed as part of natural κ light chains.

pCMV-LC-lambda (SEQ ID NO:110), a vector allowing for the expression of natural human antibody λ light chains, was generated as follows. First, a DNA segment encoding an Ig λ light chain signal peptide was assembled from the 4 oligonucleotides SP-lambda-1 (5'-GGC TAG CGC CAC CAT GGC CTG GGC TCT GCT CCT CCT CAC CCT CCT-3', SEQ ID NO:111), SP-lambda-2 (5'-GTG AGG AGG AGC AGA GCC CAG GCC ATG GTG GCG CTA GCC AGC T-3', SEQ ID NO:112), SP-lambda-3 (5'-CAC TCA GGG CAC AGG GTC CTG GGC CCA GTC TGA GCT CCT GCA-3', SEQ ID NO:113) and SP-lambda-4 (5'-GGA GCT CAG ACT GGG CCC AGG ACC CTG TGC CCT GAG TGA GGA GG-3', SEQ ID NO:114), by annealing the complementary oligonucleotides SP-lambda-1 and -2, and SP-lambda-3 and -4, respectively. The two resulting double stranded DNA fragments SP-lambda-1/2 and SP-lambda-3/4 were closed into the vector pCMV-Script (Stratagene) digested with the restriction endonucleases SacI and PstI, yielding pCMV-lambda-leader. Second, the human λ light chain constant region was amplified from human spleen cDNA using the primers C-lambda-F (5'-GAG GAG GAT ATC CTA GGT CAG CCC AAG GCT GCC CC-3', SEQ ID NO:115) and C-lambda-B (5'-GAG GAG GGT ACC GTT TAA ACC TAT GAA CAT TCT GTA GGG GC-3', SEQ ID NO:116). The resulting 356 bp PCR product was digested with the restriction endonucleases EcoRV and KpnI and cloned into the vector pCMV-Script, yielding pCMV-C-lambda. Third, after the correct sequence of both plasmids was verified, pCMV-lambda-leader and pCMV-C-lambda were digested with the restriction endonucleases EcoRV and KpnI. The 340 bp fragment excised from pCMV-C-lambda, corresponding to the λ light chain constant region, was then ligated into the 4273 bp pCMV-lambda-leader vector fragment, yielding the light chain expression vector pCMV-LC-lambda. DNA fragments encoding lambda light chain variable regions can be cloned into pCMV-LC-lambda via the restriction endonucleases SacI and EcoRV and expressed as part of natural λ light chains.

pCMV-HC (SEQ ID NO:78), a vector allowing for the expression of natural human antibody γ2 heavy chains, was generated as follows. First, a DNA segment encoding an Ig heavy chain signal peptide was assembled from the 4 oligonucleotides SP-heavy-1 (5'-CGG CGC GCC ACC ATG GAC TGG ACC TGG AGG ATC CTC TT-3' SEQ ID NO:79), SP-heavy-2 (5'-ACC AAG AAG AGG ATC CTC CAG GTC CAG TCC ATG GTG GCG CGC CGA GCT-3' SEQ ID NO:80), SP-heavy-3 (5'-CTT GGT GGC AGC AGC CAC AGG AGC CCA CTC CCA GAT GCA ACT GC-3' SEQ ID NO:81) and SP-heavy-4 (5'-TCG AGC AGT TGC ATC TGG GAG TGG GCT CCT GTG GCT GCT GCC-3' SEQ ID NO:82), by annealing the complementary oligonucleotides SP-heavy-1 and -2, and SP-heavy-3 and -4, respectively. The two resulting double stranded DNA fragments SP-heavy-1/2 and SP-heavy-3/4 were cloned into the vector pCMV-Script (Stratagene) digested with the restriction endonucleases SacI and XhoI, yielding pCMV-heavy-leader. Second, the human γ2 heavy chain constant region was amplified from human spleen cDNA using the primers C-gamma2-FL (5'-GAG GAG CTC GAG GCC TCC ACC AAG GGC CCA TCG GTC TTC CCC CTG GCG CCC TGC TCC AGG AGC ACC TCC-3' SEQ ID NO:83) and C-gamma2-B (5'-GAG GAG GGT ACC TTA ATT AAT CAT TTA CCC GGA GAC AGG GAG-3' SEQ ID NO:84). The resulting 1013 bp PCR product was digested with the restriction endonucleases XhoI and KpnI and cloned into the vector pCMV-Script, yielding pCMV-C-gamma2. Third, after the correct sequence of both plasmids was verified, pCMV-heavy-leader and pCMV-C-gamma2 were digested with the restriction endonucleases XhoI and KpnI. The 999 bp fragment excised from pCMV-C-gamma2, corresponding to the γ2 heavy chain constant region, was then ligated into the 4258 bp pCMV-gamma2-leader vector fragment, yielding the heavy chain expression vector pCMV-HC. DNA fragments encoding heavy chain variable regions can be cloned into pCMV-HC via the restriction endonucleases XhoI and ApaI and expressed as part of natural γ2 heavy chains. Cotransfection of a pCMV-LC (SEQ ID NO:71) with a pCMV-HC (SEQ ID NO:78) expression construct will allow for the production of whole IgG2.

pCMV-Fd (SEQ ID NO:85), a vector allowing for the expression of human γ2 heavy chain Fd regions, was generated as follows. The human γ2 heavy chain Fd region was amplified from the plasmid pCMV-C-gamma2 using the primers C-gamma2-F (5'-GAG GAG CTC GAG GCC TCC ACC AAG GGC CCA TCG-3', SEQ ID NO:86) and Fd-gamma2-B (5'-GAG GAG GGT ACC TTA ATT AAT CAT TTG CGC TCA ACT GTC TTG TC-3', SEQ ID NO:87). The resulting 338 bp PCR product was digested with the restriction endonucleases XhoI and KpnI and cloned into the vector pCMV-gamma2-leader, yielding pCMV-Fd (SEQ ID NO:85). DNA fragments encoding heavy chain variable regions can be cloned into pCMV-Fd via the restriction endonucleases XhoI and ApaI and expressed as part of γ2 heavy chain Fd regions. Cotransfection of a pCMV-LC (SEQ ID NO:71) with a pCMV-Fd (SEQ ID NO:85) expression construct will allow for the production of Fab fragments.

pCMV-HC-g1 (SEQ ID NO:88), a vector allowing for the expression of natural human antibody γ1 heavy chains, was generated as follows. The human γ1 heavy chain constant region was amplified from human bone marrow cDNA using the primers C-gamma1-F (5'-CAA GGG CCC ATC GGT CTT CCC CCT GGC ACC CTC-3', SEQ ID NO:89) and C-gamma2-B (SEQ ID NO:84). The resulting 1005 bp PCR product was digested with the restriction endonucleases ApaI and KpnI and used to replace the Fd coding region in pCMV-Fd (SEQ ID NO:85), yielding pCMV-HC-g1 (SEQ ID NO:88). DNA fragments encoding heavy chain variable regions can be cloned into pCMV-HC-g1 (SEQ ID NO:88) via the restriction endonucleases XhoI and ApaI and expressed as part of natural γ1 heavy chains. Cotransfection of a pCMV-LC (SEQ ID NO:71) with a pCMV-HC-g1 (SEQ ID NO:88) expression construct will allow for the production of whole IgG1.

pCMV-HC-g4 (SEQ ID NO:90), a vector allowing for the expression of natural human antibody γ4 heavy chains, was generated by nested PCR as follows. The human γ4 heavy chain constant region was pre-amplified from human spleen cDNA using the primers C-gamma2-F (SEQ ID NO:86) and C-gamma4-B2 (5'-AGC GGG GGC TTG CCG GCC CTG-3', SEQ ID NO:123). The resulting 1021bp PCR product was then reamplified with the primers C-gamma2-FL (SEQ ID NO:83) and C-gamma4-B (5'-GAG GAG GGT ACC TTA ATT AAC CGG CCC TGG CAC TCA TTT ACC CA-3', SEQ ID NO:91). The resulting 1029bp PCR product was digested with the restriction endonucleases XhoI and Pac1 and used to replace the Fd coding region in pCMV-Fd (SEQ ID NO:85), yielding pCMV-HC-g4 (SEQ ID NO:90). DNA fragments encoding heavy chain variable regions can be cloned into pCMV-HC-g4 (SEQ ID NO:90) via the restriction endonucleases XhoI and ApaI and expressed as part of natural γ4 heavy chains. Cotransfection of a pCMV-LC (SEQ ID NO:71) or pCMV-LC-lambda (SEQ ID NO:110) with a pCMV-HC-g4 (SEQ ID NO:90) expression construct will allow for the production of whole IgG4.

### EXAMPLE 9

### Construction, expression, and purification of fully human Qβ-specific IgG and Fab.

The heavy and light chain variable region coding segments of scFv-Qb#2 (SEQ ID NO:8), scFv-Qb#3 (SEQ ID NO:9), scFv-Qb#5 (SEQ ID NO:11) and scFv-Qb#8 (SEQ ID NO:14) were amplified by PCR using variable region-specific transfer primers (SEQ ID NO:92-103). Specifically, the light chain variable regions were amplified as follow, wherein VL stands for lambda light chain variable region, VK stands for kappa light chain variable region, and VH stands for heavy chain variable region: VL-Qb#2 was amplified with the primers VL-SacI-F (SEQ ID NO:95) and VL-EcoR5-B1 (SEQ ID NO:100); VK-Qb#3 with the primers VK-SacI-F (SEQ ID NO:92) and VK-EcoR5-B2 (SEQ ID NO:94); VL-Qb#5 with the primers VL-SacI-F (SEQ ID NO:95) and VL-EcoR5-B2 (SEQ ID NO:101); VL-Qb#8 with the primers VL-SacI-F3 (SEQ ID NO:98) and VL-EcoR5-B2 (SEQ ID NO:101). The heavy chain variable region coding segments VH-Qb#2, VH-Qb#3, VH-Qb#5 and VH-Qb#8 were all amplified with the primers VH-XhoI-F (SEQ ID NO:102) and VH-ApaI-B (SEQ ID NO:103).

The resulting light chain variable region PCR products were digested with the restriction enzymes SacI and EcoR5, purified by agarose gel electrophoresis, and ligated into SacI-EcoR5 digested pCMV-LC (SEQ ID NO:71), yielding the light chain expression vectors pCMV-Qb#2-LC, pCMV-Qb#3-LC, pCMV-Qb#5-LC and pCMV-Qb#8-LC. Similarly, the heavy chain variable region PCR products were digested with the restriction enzymes XhoI and ApaI, gel purified, and ligated into XhoI-ApaI digested pCMV-HC (SEQ ID NO:78), yielding the γ2 heavy chain expression vectors pCMV-Qb#2-HC, pCMV-Qb#3-HC, pCMV-Qb#5-HC and pCMV-Qb#8-HC, as well as into XhoI-ApaI digested pCMV-Fd (SEQ ID NO:85), yielding the γ2 Fd region expression vectors pCMV-Qb#2-Fd, pCMV-Qb#3-Fd, pCMV-Qb#5-Fd and pCMV-Qb#8-Fd.

As demonstrated in Example 8, co-expression of each of the pCMV-LC expression constructs with the corresponding pCMV-HC or pCMV-Fd expression construct in principle allows for the production of, respectively, whole IgG or Fab fragments. However, to increase yields and facilitate large-scale production of antibodies, heavy and light chain coding regions were first combined into a single, EBNA-based expression vector, pCB15 (SEQ ID NO:104). For instance, for expression of antibody Qb#2 as a whole IgG, the light chain coding region was excised from pCMV-Qb#2-LC by digestion with the restriction enzymes NheI and PmeI, the resulting 735 bp fragment purified by agarose gel electrophoresis, and then ligated into NheI-PmeI digested pCB15, yielding pCB15-Qb#2-LC. The Qb#2 heavy chain coding region was then excised from pCMV-Qb#2-HC by digestion with AscI and PacI, the resulting 1433 bp fragment gel-purified, and then ligated into AscI-PacI digested pCB15-Qb#2-LC, yielding the whole IgG expression vector pCB15-Qb#2-IgG2.

For expression as a Fab fragment, the Qb#2 Fd coding region was excised from pCMV-Qb#2-Fd by digestion with AscI and PacI, the resulting 758 bp fragment gel-purified, and then ligated into AscI-PacI digested pCB15-Qb#2-LC, yielding the Fab expression vector pCB15-Qb#2-Fab. The whole IgG expression vectors pCB15-Qb#3-IgG2, pCB15-Qb#5-IgG2 and pCB15-Qb#8-IgG2, and the Fab expression vectors pCB15-Qb#3-Fab, pCB15-Qb#5-Fab and pCB15-Qb#8-Fab were generated in exactly the same way as the Qb#2 expression vectors.

Expression of whole IgG and Fab fragments was done in HEK-293T cells, exactly as described for the scFv-Fc fusion proteins (Example 6), with expression levels in the range of 20 to 50 mg/L. Both whole IgG and Fab fragments were purified by applying protein-containing cell supernatants to affinity columns (IgG: protein G; Fab: goat anti-human F(ab')2). The columns were washed with 10 column volumes of phosphate-buffered saline (PBS), and bound protein eluted with 0.1 M Glycine pH 3.6. 1 ml fractions were collected in tubes containing 0.1 ml of 1 M Tris pH 7.5 for neutralization. Protein-containing fractions were analyzed by SDS-PAGE and pooled. The buffer was exchanged with PBS by dialysis using 10'000 MWCO Slide-A-Lyzer dialysis cassettes (Pierce). The purified proteins in PBS were then filtered through 0.22 µM Millex GV sterile filters (Millipore) and aliquoted. Working stocks were kept at 4 °C, whereas aliquots for long-term storage were flash-frozen in liquid nitrogen and kept -80 °C.

The binding properties of purified Qβ-specific IgG2 and Fab immunoglobulins were analyzed by ELISA essentially as described in Example 7. For most of the immunoglobulins, half-maximal binding was observed at picomolar concentrations, suggesting that they are of very high affinity (see Table 1).

**Table 1: Range of concentrations of half-maximal binding to Qβ.**

| | **IgG2** | **Fab** |
|---|---|---|
| Qβ#2 | 25 - 56 pM | 261 - 454 pM |
| Qβ#3 | 15 - 37 pM | 77 - 239 pM |
| Qβ#5 | 21 - 54 pM | 82 - 236 pM |
| Qβ#8 | 456 - 1'097 pM | > 10'000 pM |

### EXAMPLE 10

### Isolation of human pathogen-specific human monoclonal antibodies: anti-preS1 antibodies

Peripheral blood mononuclear cells (PBMC) were isolated from 20 ml of heparinized blood of a fr-preS1 (21-47) (SEQ ID NO:124) vaccinated volunteer by a standard Ficoll-Hypaque™ Plus (Amersham Biosciences) gradient method. PBMC were stained with: (1) Qβ-preS1 (21-47) (1 µg/ml) in combination with a Alexa 488 nm-labeled Qβ-specific mouse mAb; (2) Alexa 647 nm-labeled Qβ (3 µg/ml); (3) PE-labeled mouse anti-human IgM (diluted 1:50; BD Biosciences Pharmingen), mouse anti-human IgD (diluted 1:100; BD Biosciences Pharmingen), mouse anti-human CD14 (diluted 1:50; BD Biosciences Pharmingen), and mouse anti-human CD3 (diluted 1:50; BD Biosciences Pharmingen) antibodies; and (4) PE-TexasRed-labeled mouse anti-human CD19 antibody (diluted 1:50; Caltag Laboratories). After staining, cells were washed, filtered and preS1 (21-47)-specific B cells (FL1-positive, FL2-negative, FL3-positive, FL4-negative) were sorted on a FACSVantage SE flow cytometer (Becton Dickinson).

Construction of a Sindbis-based scFv cell surface display library was done exactly as described in Example 3. Cells displaying preS1 (21-47)-specific scFv antibodies were isolated essentially as described in Example 4, using Qβ-preS1 (21-47) as a bait. A total of six preS1 (21-47)-specific antibodies were identified: A124, C032, E040, J058, L023 and L025. ScFv-Fc fusion proteins were cloned, expressed and purified as described in Examples 5 and 6. The binding properties of purified scFv-Fc fusion proteins were analyzed by ELISA essentially as described in Example 7. Half-maximal binding was observed at concentrations in the low nanomolar range (A124, 6.9 nM; C032, 5.6 nM; E040, 1.2 - 1.9 nM; J058, 7.5 nM; L023, 2.2 nM; and L025, 1.0 nM), suggesting that the antibodies are of high affinity. Antibodies A124, E040, J058, L023, and L025 were converted to whole human IgG2, and expressed and purified as described in Example 9.

### EXAMPLE 11

### Isolation of hapten-specific human monoclonal antibodies: anti-Nicotin antibodies

Peripheral blood mononuclear cells (PBMC) are isolated from 20 ml of heparinized blood of a Qβ-Nicotin-vaccinated volunteer by a standard Ficoll-HypaqueTM Plus (Amersham Biosciences) gradient method. PBMC are stained with: (1) Qβ-Nicotin (4 µg/ml) in combination with a Nicotin-specific mouse mAb and FITC-labeled goat anti-mouse antibody (Jackson ImmunoResearch Laboratories); (2) Alexa 647nm-labeled Qβ (4 µg/ml); (3) PE-labeled mouse anti-human IgM, IgD, CD14 and CD3 antibodies as described in Example 10; and (4) PE-TexasRed-labeled mouse anti-human CD19 antibody as described in Example 10. After staining, cells are washed, filtered and Nicotin-specific B cells (FL1-positive, FL2-negative, FL3-positive, FL4-negative) are sorted on a FACSVantage SE flow cytometer (Becton Dickinson).

Construction of a Sindbis-based scfv cell surface display library is done exactly as described in Example 3. Cells displaying Nicotin-specific scFv antibodies are isolated essentially as described in Example 4, using Qβ-Nicotin as a bait. Nicotin-specific antibodies are cloned, expressed and purified as described in Examples 5, 6 and 9.

### EXAMPLE 12

### Isolation of self antigen-specific human monoclonal antibodies: anti-ghrelin antibodies

Peripheral blood mononuclear cells (PBMC) are isolated from 20 ml of heparinized blood of a Qβ-ghrelin (24-31)-vaccinated volunteer by a standard Ficoll-HypaqueTM Plus (Amersham Biosciences) gradient method. PBMC are stained with: (1) Qβ-ghrelin (24-31) (4 µg/ml) in combination with a Alexa 488nm-labeled Qβ-specific mouse mAb; (2) Alexa 647nm-labeled Qβ (4µg/ml); (3) PE-labeled mouse anti-human IgM, IgD, CD14 and CD3 antibodies as described in Example 10; and (4) PE-TexasRed-labeled mouse anti-human CD19 antibody as described in Example 10. After staining, cells are washed, filtered and ghrelin (24-31)-specific B cells (FL1-positive, FL2-negative, FL3-positive, FL4-negative) are sorted on a FACSVantage SE flow cytometer (Becton Dickinson).

Construction of a Sindbis-based scFv cell surface display library is done exactly as described in Example 3. Cells displaying ghrelin (24-31)-specific scFv antibodies are isolated essentially as described in Example 4; using Qβ-ghrelin (24-31) as a bait. Ghrelin (24-31)-specific antibodies are cloned, expressed and purified as described in Examples 5, 6 and 9.

### EXAMPLE 13

### Isolation of allergen-specific human monoclonal antibodies: anti-Fel d1 antibodies

In principle, Fel d1-specific B cells can be isolated from a cat-allergic individual. Alternatively, Fel d1-specific B cells are isolated from a Fel d1-vaccinated volunteer. Thus, peripheral blood mononuclear cells (PBMC) are isolated from 20 ml of heparinized blood by a standard Ficoll-Hypaque™ Plus (Amersham Biosciences) gradient method. PBMC are stained with: (1) Qβ-Fel d1 (4 µg/ml) in combination with a Alexa 488nm-labeled Qβ-specific mouse mAb; (2) Alexa 647nm-labeled Qβ (3 µg/ml); (3) PE-labeled mouse anti-human IgM, IgD, CD14 and CD3 antibodies as described in Example 10; and (4) PE-TexasRed-labeled mouse anti-human CD19 antibody as described in Example 10. After staining, cells are washed, filtered and Fel d1-specific B cells (FL1-positive, FL2-negative, FL3-positive, FL4-negative) are sorted on a FACSVantage SE flow cytometer (Becton Dickinson).

Construction of a Sindbis-based scFv cell surface display library is done exactly as described in Example 3. Cells displaying Fel d1-specific scFv antibodies are isolated essentially as described in Example 4, using Qβ-Fel dl as a bait. Fel dl-specific antibodies are cloned, expressed and purified as described in Examples 5, 6 and 9.

## Claims

1. A method of isolating a cell expressing an antibody specifically binding an antigen of interest, said method comprising the steps of:
(a) selecting from a population of isolated B cells a sub-population of B cells by selecting B cells for their capability of specifically binding said antigen of interest;
(b) generating an alphaviral expression library, wherein each member of said alphaviral expression library encodes an antibody comprising at least one variable region (VR) by
(i) generating a multitude of DNA molecules, wherein said generating comprises the step of amplifying a pool of DNA molecules from said sub-population of B cells, wherein each of said DNA molecules of said pool of DNA molecules encodes one of said at least one variable region (VR); and
(ii) cloning a specimen of said multitude of DNA molecules into an alphaviral expression vector;
(c) introducing said alphaviral expression library into a first population of mammalian cells;
(d) displaying antibodies of said alphaviral expression library on the surface of said mammalian cells; and
(e) isolating from said first population of mammalian cells a cell, wherein said cell is selected for the capability of the antibody displayed on its surface of specifically binding said antigen of interest or antigenic determinant thereof.

2. The method of claim 1, wherein each antibody encoded by said alphaviral expression library further comprises:
(a) a signal peptide, wherein preferably said signal peptide is a mouse Ig kappa light chain signal peptide or comprises or consists of SEQ ID NO: 105; and
(b) a transmembrane region, wherein preferably said transmembrane region is derived from human PDGFR beta chain, or comprises or consists of SEQ ID NO: 106; and,
optionally,
(c) a detection tag, wherein preferably said detection tag is HAor comprises or consists of SEQ ID NO: 108.

3. The method of any one of claims 1 or 2, wherein said generating an alphaviral expression library comprises the steps of:
(a) generating a multitude of DNA molecules encoding antibodies, said generating comprising the steps of:
(i) amplifying from said sub-population of B cells a first pool of DNA molecules encoding HCVRs;
(ii) amplifying from said sub-population of B cells a second pool of DNA molecules encoding LCVRs; and
(iii) linking specimens of said first and of said second pool of DNA molecules to each other by a DNA encoding a linker region (LR);
(b) cloning a specimen of said multitude of DNA molecules into an alphaviral expression vector;
wherein each member of said alphaviral expression library encodes an antibody comprising a signal peptide, a HCVR, a LCVR and a transmembrane region, wherein said HCVR and said LCVR are linked to each other by said linker region.

4. The method of any one of claims 1 to 3, wherein said generating a multitude of DNA molecules comprises the steps of:
(a) isolating RNA from said sub-population of B cells;
(b) transcribing said RNA to cDNA; and
(c) amplifying from said cDNA a pool of DNA molecules using a mixture of oligonucleotides comprising at least two oligonucleotides capable of amplifying VR coding regions.

5. The method of any one of claims 1 to 4, wherein said generating a multitude of DNA molecules comprises the steps of:
(a) isolating RNA from said sub-population of B cells;
(b) transcribing said RNA to cDNA;
(c) amplifying from said cDNA said first pool of DNA molecules using a first mixture of oligonucleotides comprising at least two oligonucleotides capable of amplifying HCVR coding regions;
(d) amplifying from said cDNA said second pool of DNA molecules using a second mixture of oligonucleotides comprising at least two oligonucleotides capable of amplifying LCVR coding regions; and
(e) linking specimens of said first and said second pool of DNA molecules to each other by a DNA encoding said linker region.

6. The method of any one of claims 4 or 5,
(a) wherein said mixture of oligonucleotides, preferably said first mixture of oligonucleotides comprises at least two oligonucleotides capable of amplifying HCVR coding regions or comprises at least two oligonucleotides selected from the group consisting of SEQ ID NO:42 to 48; and/or
(b) wherein said mixture of oligonucleotides, preferably said second mixture of oligonucleotides, comprises at least two oligonucleotides capable of amplifying kappa LCVR coding regions or comprises at least two oligonucleotides selected from the group consisting of SEQ ID NO:49 to 56; and/or
(c) wherein said mixture of oligonucleotides, preferably said second mixture of oligonucleotides, comprises at least two oligonucleotides capable of amplifying lambda LCVR coding regions or comprises at least two oligonucleotides selected from the group consisting of SEQ ID NO:57 to 68.

7. The method of any one of claims 1 to 6 wherein said antibody encoded by said alphaviral expression library comprises said HCVR, said LCVR and said linker region (LR), in an order selected from:
(a) LCVR-LR-HCVR; and
(b) HCVR-LR-LCVR.

8. The method of any of claims 3 to 7, wherein said linking of said specimens of said first pool of DNA molecules and of said second pool of DNA molecules to each other is performed by PCR using the oligonucleotides depicted in SEQ ID NO:69 and SEQ ID NO:70 as primers.

9. The method of any one of claims 1 to 8, wherein said cloning a specimen of said multitude of DNA molecules into an alphaviral expression vector comprises the steps of:
(a) generating a DNA construct encoding said antibody comprising a HCVR, a LCVR and a transmembrane region by linking a specimen of said multitude of DNA molecules to a first DNA element encoding said transmembrane region; and
(b) functionally linking said DNA construct to a second DNA element encoding a signal peptide directing said antibody to the secretory pathway.

10. The method of any one of claims 1 to 9, wherein
(a) said alphaviral expression vector is derived from Sindbis virus,
(b) said alphaviral expression vector is derived from Sindbis virus and comprises:
(i) nucleotides 4 to 282 of SEQ ID NO: 1; or
(ii) nucleotides 4 to 312 of SEQ ID NO :40; and/or
(c) said alphaviral expression vector is derived from Sindbis virus and comprises or consists of:
(i) SEQ ID NO: 38 (pDel-SP-TM), or
(ii) SEQ ID NO:39 (pDel-SP-HA-TM).

11. The method of any one of claims 1 to 10, wherein said selecting from said population of isolated B cells a sub-population of B cells comprises the steps of:
(a) contacting said population of isolated B cells with said antigen of interest or antigenic determinant thereof, wherein preferably said antigen of interest or antigenic determinant thereof is labeled with a fluorescence dye; and
(b) selecting B cells specifically binding said antigen of interest or antigenic determinant thereof, preferably by FACS sorting.

12. The method of any one of claims 1 to 11, wherein said selecting from said population of isolated B cells a sub-population of B cells comprises the steps of:
(a) contacting said population of isolated B cells with said antigen of interest or antigenic determinant thereof, wherein said antigen of interest or antigenic determinant thereof is labeled with a first fluorescence dye;
(b) contacting the cells of said population of isolated B cells with anti-IgM and/or anti-IgD antibodies, wherein said anti-IgM and/or anti-IgD antibodies are labeled with a second fluorescence dye; and
(c) selecting B cells bound to said antigen of interest or antigenic determinant thereof but not bound to said anti-IgM and/or not bound to said anti-IgD antibodies by FACS sorting.

13. The method of any one of claims 1 to 12, wherein said introducing said alphaviral expression library into a first population of mammalian cells is performed by infecting said mammalian cell with said alphaviral expression library.

14. The method of any one of claims 1 to 13, wherein said isolating of said individual cell is performed by FACS sorting,
wherein preferably said isolating of said individual cell comprises the steps of:
(a) staining said first population of mammalian cells with said antigen of interest or antigenic determinant thereof, wherein said antigen of interest or antigenic determinant thereof is labeled with a fluorescence dye; and
(b) separating an individual cell specifically binding said antigen of interest or antigenic determinant thereof, by means of FACS sorting;
and wherein further preferably said method further comprises the steps of:
(a) cultivating at least one of said individual cell in the presence of a second population of mammalian cells;
(b) verifying the capability of said second population of mammalian cells of specifically binding said antigen of interest or antigenic determinant thereof.

15. The method of any one of claims 1 to 14, wherein said first population of mammalian cells and/or said second population of mammalian cells comprises or consists of cells selected from:
(a) BHK 21 cells;
(b) Neuro-2a cells; and
(c) HEK-293T cells.

16. The method of any of claims 1 to 15,
(a) wherein the antibody expressed by the cell to be isolated is a single chain antibody,
(b) wherein said population of isolated B cells used for the selecting of claim 1 step (a) comprises or consists of peripheral blood mononuclear cells (PBMCs); and/or
(c) wherein each member of said alphaviral expression library encodes an antibody comprising a heavy chain variable region (HCVR) and a light chain variable region (LCVR).

## Patentansprüche

1. Verfahren zum Isolieren einer Zelle, die einen spezifisch an ein Antigen von Interesse bindenden Antikörper exprimiert, wobei das Verfahren die Schritte umfasst:
(a) Auswählen einer Sub-Population von B-Zellen aus einer Population von isolierten B-Zellen durch Auswählen der B-Zellen hinsichtlich ihrer Fähigkeit zur spezifischen Bindung an das Antigen von Interesse;
(b) Erzeugen einer alphaviralen Expressionsbibliothek, wobei jedes Mitglied der alphaviralen Expressionsbibliothek einen Antikörper kodiert, der mindestens eine variable Region (VR) umfasst, durch
(i) Erzeugen einer Vielzahl von DNA-Molekülen, wobei das Erzeugen den Schritt des Amplifizierens eines Pools von DNA-Molekülen aus der Sub-Population von B-Zellen umfasst, wobei jedes der DNA-Moleküle des Pools von DNA-Molekülen eine der mindestens einen variablen Region (VR) kodiert; und
(ii) Klonieren einer Probe der Vielzahl von DNA-Molekülen in einen alphaviralen Expressionsvektor;
(c) Einführen der alphaviralen Expressionsbibliothek in eine erste Population von Säugerzellen;
(d) Präsentieren von Antikörpern der alphaviralen Expressionsbibliothek auf der Oberfläche der Säugerzellen; und
(e) Isolieren einer Zelle aus der ersten Population von Säugerzellen, wobei die Zelle hinsichtlich der Fähigkeit des auf ihrer Oberfläche präsentierten Antikörpers zur spezifischen Bindung des Antigens von Interesse oder antigener Determinanten hiervon ausgewählt wird.

2. Verfahren nach Anspruch 1, wobei jeder Antikörper, der durch die alphavirale Expressionsbibliothek kodiert wird, weiterhin umfasst:
(a) ein Signalpeptid, wobei vorzugsweise das Signalpeptid ein Signalpeptid der leichten Kette von Ig kappa aus der Maus ist oder SEQ ID NO:105 umfasst oder daraus besteht; und
(b) eine Transmembranregion, wobei die Transmembranregion vorzugsweise aus der humanen PDGFR-beta-Kette abgeleitet ist oder SEQ ID NO:106 umfasst oder daraus besteht; und gegebenenfalls
(c) einen Nachweis-Tag, wobei vorzugsweise der Nachweis-Tag HA ist oder SEQ ID NO:108 umfasst oder daraus besteht.

3. Verfahren nach einem beliebigen der Ansprüche 1 oder 2, wobei das Erzeugen einer alphaviralen Expressionsbibliothek die Schritte umfasst:
(a) Erzeugen einer Vielfalt von Antikörper-kodierenden DNA-Molekülen, wobei das Erzeugen die Schritte umfasst:
(i) Amplifizieren eines ersten Pools von DNA-Molekülen, die HCVRs kodieren, aus der Sub-Population von B-Zellen;
(ii) Amplifizieren eines zweiten Pools von DNA-Molekülen, die LCVRs kodieren, aus der Sub-Population von B-Zellen; und
(iii) Verknüpfen von Proben des ersten und des zweiten Pools von DNA-Molekülen miteinander durch eine DNA, die eine Linkerregion (LR) kodiert;
(b) Klonieren einer Probe der Vielzahl von DNA-Molekülen in einen alphaviralen Expressionsvektor;
wobei jedes Mitglied der alphaviralen Expressionsbibliothek einen Antikörper kodiert, der ein Signalpeptid, eine HCVR, eine LCVR und eine Transmembranregion umfasst, wobei die HCVR und die LCVR über die Linkerregion miteinander verknüpft sind.

4. Verfahren nach einem beliebigen der Ansprüche 1 bis 3, wobei das Erzeugen einer Vielzahl von DNA-Molekülen die Schritte umfasst:
(a) Isolieren von RNA aus der Sub-Population von B-Zellen;
(b) Transkribieren der RNA zu cDNA; und
(c) Amplifizieren eines Pools von DNA-Molekülen aus der cDNA unter Verwendung einer Mischung von Oligonukleotiden, umfassend mindestens zwei Oligonukleotide, die zur Amplifikation der VR-kodierenden Regionen in der Lage sind.

5. Verfahren nach einem beliebigen der Ansprüche 1 bis 4, wobei das Erzeugen einer Vielzahl von DNA-Molekülen die Schritte umfasst:
(a) Isolieren von RNA aus der Sub-Population von B-Zellen;
(b) Transkribieren der RNA zu cDNA; und
(c) Amplifizieren eines ersten Pools von DNA-Molekülen aus der cDNA unter Verwendung einer ersten Mischung von Oligonukleotiden, umfassend mindestens zwei Oligonukleotide, die zur Amplifikation der HCVR-kodierenden Regionen in der Lage sind;
(d) Amplifizieren des zweiten Pools von DNA-Molekülen aus der cDNA unter Verwendung einer zweiten Mischung von Oligonukleotiden, umfassend mindestens zwei Oligonukleotide, die zur Amplifikation der LCVR-kodierenden Regionen in der Lage sind; und
(e) Verknüpfen von Proben des ersten und des zweiten Pools von DNA-Molekülen miteinander durch eine DNA, die die Linkerregion kodiert.

6. Verfahren nach einem beliebigen der Ansprüche 4 oder 5,
(a) wobei die Mischung von Oligonukleotiden, vorzugsweise die erste Mischung von Oligonukleotiden, mindestens zwei Oligonukleotide umfasst, die zur Amplifikation der HCVR-kodierenden Regionen in der Lage sind, oder mindestens zwei Oligonukleotide umfasst, die ausgewählt sind aus der Gruppe bestehend aus SEQ ID NO:42 bis 48; und/oder
(b) wobei die Mischung von Oligonukleotiden, vorzugsweise die zweite Mischung von Oligonukleotiden, mindestens zwei Oligonukleotide umfasst, die zur Amplifikation der kappaLCVR-kodierenden Regionen in der Lage sind, oder mindestens zwei Oligonukleotide umfasst, die ausgewählt sind aus der Gruppe bestehend aus SEQ ID NO:49 bis 56; und/oder
(c) wobei die Mischung von Oligonukleotiden, vorzugsweise die zweite Mischung von Oligonukleotiden, mindestens zwei Oligonukleotide umfasst, die zur Amplifikation der lambdaLCVR-kodierenden Regionen in der Lage sind, oder mindestens zwei Oligonukleotide umfasst, die ausgewählt sind aus der Gruppe bestehend aus SEQ ID NO:57 bis 68.

7. Verfahren nach einem beliebigen der Ansprüche 1 bis 6, wobei der von der alphaviralen Expressionsbibliothek kodierte Antikörper die HCVR, die LCVR und die Linkerregion (LR) umfasst, in einer Reihenfolge ausgewählt aus:
(a) LCVR-LR-HCVR; und
(b) HCVR-LR-LCVR.

8. Verfahren nach einem beliebigen der Ansprüche 3 bis 7, wobei das Verknüpfen der Proben des ersten Pools von DNA-Molekülen und des zweiten Pools von DNA-Molekülen miteinander mittels PCR unter Verwendung der in SEQ ID NO:69 und SEQ ID NO:70 dargestellten Oligonukleotide als Primer durchgeführt wird.

9. Verfahren nach einem beliebigen der Ansprüche 1 bis 8, wobei das Klonieren einer Probe der Vielzahl von DNA-Molekülen in einen alphaviralen Expressionsvektor die Schritte umfasst:
(a) Erzeugen eines DNA-Konstrukts, das den Antikörper kodiert, der eine HCVR, eine LCVR und eine Transmembranregion umfasst, durch Verknüpfen einer Probe der Vielzahl von DNA-Molekülen an ein erstes DNA-Element, das die Transmembranregion kodiert; und
(b) funktionelles Verknüpfen des DNA-Konstrukts mit einem zweiten DNA-Element, das ein Signalpeptid kodiert, das den Antikörper in den sekretorischen Signalweg leitet.

10. Verfahren nach einem beliebigen der Ansprüche 1 bis 9, wobei
(a) der alphavirale Expressionsvektor vom Sindbis-Virus stammt,
(b) der alphavirale Expressionsvektor vom Sindbis-Virus stammt und umfasst:
(i) die Nukleotide 4 bis 282 von SEQ ID NO:1; oder
(ii) die Nukleotide 4 bis 312 von SEQ ID NO:40; und/oder
(c) der alphavirale Expressionsvektor vom Sindbis-Virus stammt und umfasst oder daraus besteht:
(i) SEQ ID NO:38 (pDel-SP-TM), oder
(ii) SEQ ID NO:39 (pDel-SP-HA-TM).

11. Verfahren nach einem beliebigen der Ansprüche 1 bis 10, wobei das Auswählen einer Sub-Population von B-Zellen aus der Population von isolierten B-Zellen die Schritte umfasst:
(a) Inkontaktbringen der Population von isolierten B-Zellen mit dem Antigen von Interesse oder einer antigenen Determinante hiervon, wobei vorzugsweise das Antigen von Interesse oder die antigene Determinante hiervon mit einem Fluoreszenzfarbstoff markiert ist; und
(b) Auswählen der B-Zellen, die spezifisch an das Antigen von Interesse oder die antigene Determinante hiervon binden, vorzugsweise durch FACS-Sortierung.

12. Verfahren nach einem beliebigen der Ansprüche 1 bis 11, wobei das Auswählen einer Sub-Population von B-Zellen aus der Population von isolierten B-Zellen die Schritte umfasst:
(a) Inkontaktbringen der Population von isolierten B-Zellen mit dem Antigen von Interesse oder einer antigenen Determinante hiervon, wobei vorzugsweise das Antigen von Interesse oder die antigene Determinante hiervon mit einem ersten Fluoreszenzfarbstoff markiert ist; und
(b) Inkontaktbringen der Zellen der Population von isolierten B-Zellen mit Anti-IgM- und/oder Anti-IgD-Antikörpern, wobei die Anti-IgM-und/oder Anti-IgD-Antikörper mit einem zweiten Fluoreszenzfarbstoff markiert sind; und
(c) Auswählen der B-Zellen, die an das Antigen von Interesse oder die antigene Determinante hiervon gebunden sind, aber nicht an die Anti-IgM-Antikörper und/oder nicht an die Anti-IgD-Antikörper gebunden sind, durch FACS-Sortierung.

13. Verfahren nach einem beliebigen der Ansprüche 1 bis 12, wobei das Einführen der alphaviralen Expressionsbibliothek in eine erste Population von Säugerzellen durch Infizieren der Säugerzellen mit der alphaviralen Expressionsbibliothek durchgeführt wird.

14. Verfahren nach einem beliebigen der Ansprüche 1 bis 13, wobei das Isolieren der individuellen Zellen durch FACS-Sortierung durchgeführt wird, wobei vorzugsweise das Isolieren der einzelnen Zellen die Schritte umfasst:
(a) Färben der ersten Population von Säugerzellen mit dem Antigen von Interesse oder der antigenen Determinante hiervon, wobei das Antigen von Interesse oder die antigene Determinante hiervon mit einem Fluoreszenzfarbstoff markiert ist; und
(b) Abtrennen der individuellen Zelle, die spezifisch das Antigen von Interesse oder die antigene Determinante hiervon bindet, durch FACS-Sortierung;
und wobei weiter bevorzugt das Verfahren weiter die Schritte umfasst:
(a) Kultivieren mindestens einer der individuellen Zellen in Gegenwart einer zweiten Population von Säugerzellen;
(b) Verifizieren der Fähigkeit der zweiten Population von Säugerzellen zur spezifischen Bindung des Antigens von Interesse oder der antigenen Determinante hiervon.

15. Verfahren nach einem beliebigen der Ansprüche 1 bis 14, wobei die erste Population von Säugerzellen und/oder die zweite Population von Säugerzellen Zellen umfasst oder aus Zellen besteht, ausgewählt aus:
(a) BHK-21-Zellen;
(b) Neuro-2a-Zellen; und
(c) HEK-293T-Zellen.

16. Verfahren nach einem beliebigen der Ansprüche 1 bis 15,
(a) wobei der Antikörper, der von der zu isolierenden Zelle exprimiert wird, ein einzelkettiger Antikörper ist,
(b) wobei die Population von isolierten B-Zellen, die zum Auswählen gemäß Anspruch 1 Schritt (a) verwendet wird, mononukleäre Zellen des peripheren Blutes *(Peripheral Blood Mononuclear Cells;* PBMCs) umfasst oder daraus besteht; und/oder
(c) wobei jedes Mitglied der alphaviralen Expressionsbibliothek einen Antikörper kodiert, der eine variable Region der schweren Kette (*Heavy Chain Variable Region*; HCVR) und eine variable Region der leichten Kette *(Light Chain Variable Region;* LCVR) umfasst.

## Revendications

1. Procédé d'isolement d'une cellule exprimant un anticorps se liant spécifiquement à un antigène d'intérêt, ledit procédé comprenant les étapes de :
(a) sélection parmi une population de lymphocytes B isolés d'une sous-population de lymphocytes B par la sélection de lymphocytes B pour leur capacité à se lier spécifiquement audit antigène d'intérêt ;
(b) génération d'une banque d'expression alphavirale, dans lequel chaque membre de ladite banque d'expression alphavirale code pour un anticorps comprenant au moins une région variable (VR) par
(i) la génération d'une multitude de molécules d'ADN, dans lequel ladite génération comprend l'étape d'amplification d'un mélange de molécules d'ADN à partir de ladite sous-population de lymphocytes B, dans lequel chacune desdites molécules d'ADN dudit mélange de molécules d'ADN code pour une de ladite au moins une région variable (VR) ; et
(ii) le clonage d'un spécimen de ladite multitude de molécules d'ADN dans un vecteur d'expression alphaviral ;
(c) introduction de ladite banque d'expression alphavirale dans une première population de cellules de mammifères ;
(d) présentation des anticorps de ladite banque d'expression alphavirale à la surface desdites cellules de mammifères ; et
(e) isolement à partir de ladite première population de cellules de mammifères d'une cellule, dans lequel ladite cellule est sélectionnée pour la capacité de l'anticorps présenté à sa surface de se lier spécifiquement audit antigène d'intérêt ou déterminant antigénique de celui-ci.

2. Procédé selon la revendication 1, dans lequel chaque anticorps codé par ladite banque d'expression alphavirale comprend en outre :
(a) un peptide signal, dans lequel de préférence ledit peptide signal est un peptide signal de chaîne légère kappa d'Ig de souris ou comprend ou consiste en SEQ ID NO : 105 ; et
(b) une région transmembranaire, dans lequel de préférence ladite région transmembranaire est dérivée de la chaîne bêta du PDGFR humain, ou comprend ou consiste en SEQ ID NO : 106 ; et
optionnellement,
(c) un marqueur de détection, dans lequel de préférence ledit marqueur de détection est la HA ou comprend ou consiste en SEQ ID NO : 108.

3. Procédé selon l'une quelconque des revendications 1 ou 2, dans lequel ladite génération d'une banque d'expression alphavirale comprend les étapes de :
(a) génération d'une multitude de molécules d'ADN codant pour des anticorps, ladite génération comprenant les étapes de :
(i) amplification à partir de ladite sous-population de lymphocytes B d'un premier mélange de molécules d'ADN codant pour des HCVR ;
(ii) amplification à partir de ladite sous-population de lymphocytes B d'un second mélange de molécules d'ADN codant pour des LCVR ; et
(iii) liaison de spécimens dudit premier et dudit second mélange de molécules d'ADN les uns avec les autres par un ADN codant pour une région lieur (LR) ;
(b) clonage d'un spécimen de ladite multitude de molécules d'ADN dans un vecteur d'expression alphaviral ;
dans lequel chaque membre de ladite banque d'expression alphavirale code pour un anticorps comprenant un peptide signal, une HCVR, une LCVR et une région transmembranaire, dans lequel ladite HCVR et ladite LCVR sont liées l'une à l'autre par ladite région lieur.

4. Procédé selon l'une quelconque des revendications 1 à 3, dans lequel ladite génération d'une multitude de molécules d'ADN comprend les étapes de :
(a) isolement de l'ARN à partir de ladite sous-population de lymphocytes B ;
(b) transcription dudit ARN en ADNc ; et
(c) amplification à partir dudit ADNc d'un mélange de molécules d'ADN en utilisant un mélange d'oligonucléotides comprenant au moins deux oligonucléotides capables d'amplifier les régions codantes des VR.

5. Procédé selon l'une quelconque des revendications **1** à **4,** dans lequel ladite génération d'une multitude de molécules d'ADN comprend les étapes de :
(a) isolement de l'ARN à partir de ladite sous-population de lymphocytes B ;
(b) transcription dudit ARN en ADNc ;
(c) amplification à partir dudit ADNc dudit premier mélange de molécules d'ADN en utilisant un premier mélange d'oligonucléotides comprenant au moins deux oligonucléotides capables d'amplifier les régions codantes des HCVR ;
(d) amplification à partir dudit ADNc dudit second mélange de molécules d'ADN en utilisant un second mélange d'oligonucléotides comprenant au moins deux oligonucléotides capables d'amplifier les régions codantes des LCVR ; et
(e) liaison des spécimens dudit premier groupe et dudit second groupe de molécules d'ADN les uns aux autres par un ADN codant pour ladite région lieur.

6. Procédé selon l'une quelconque des revendications **4** ou **5,**
(a) dans lequel ledit mélange d'oligonucléotides, de préférence ledit premier mélange d'oligonucléotides, comprend au moins deux oligonucléotides capables d'amplifier les régions codantes des HCVR ou comprend au moins deux oligonucléotides choisis dans le groupe consistant en SEQ ID NO : 42 à 48 ; et/ou
(b) dans lequel ledit mélange d'oligonucléotides, de préférence ledit second mélange d'oligonucléotides, comprend au moins deux oligonucléotides capables d'amplifier les régions codantes des LCVR kappa ou comprend au moins deux oligonucléotides choisis dans le groupe consistant en SEQ ID NO : 49 à 56 ; et/ou
(c) dans lequel ledit mélange d'oligonucléotides, de préférence ledit second mélange d'oligonucléotides, comprend au moins deux oligonucléotides capables d'amplifier les régions codantes des LCVR lambda ou comprend au moins deux oligonucléotides choisis dans le groupe consistant en SEQ ID NO : 57 à 68.

7. Procédé selon l'une quelconque des revendications 1 à 6, dans lequel ledit anticorps codé par ladite banque d'expression alphavirale comprend ladite HCVR, ladite LCVR et ladite région lieur (LR), dans un ordre choisi parmi :
(a) LCVR-LR-HCVR ; et
(b) HCVR-LR-LCVR.

8. Procédé selon l'une quelconque des revendications **3** à **7,** dans lequel ladite liaison desdits spécimens dudit premier mélange de molécules d'ADN et dudit second mélange de molécules d'ADN les uns aux autres est réalisée par PCR en utilisant les oligonucléotides représentés par SEQ ID NO : 69 et SEQ ID NO : 70 en tant qu'amorces.

9. Procédé selon l'une quelconque des revendications **1** à **8,** dans lequel ledit clonage d'un spécimen de ladite multitude de molécules d'ADN dans un vecteur d'expression alphaviral comprend les étapes de :
(a) génération d'une construction d'ADN codant pour ledit anticorps comprenant une HCVR, une LCVR et une région transmembranaire par liaison d'un spécimen de ladite multitude de molécules d'ADN à un premier élément d'ADN codant pour ladite région transmembranaire ; et
(b) liaison fonctionnelle de ladite construction d'ADN à un second élément d'ADN codant pour un peptide signal dirigeant ledit anticorps vers la voie sécrétoire.

10. Procédé selon l'une quelconque des revendications **1** à **9,** dans lequel
(a) ledit vecteur d'expression alphaviral est dérivé du virus Sindbis ;
(b) ledit vecteur d'expression alphaviral est dérivé du virus Sindbis et comprend :
(i) les nucléotides 4 à 282 de SEQ ID NO : 1 ; ou
(ii) les nucléotides 4 à 312 de SEQ ID NO : 40 ; et/ou
(c) ledit vecteur d'expression alphaviral est dérivé du virus Sindbis et comprend ou consiste en :
(i) SEQ ID NO : 38 (pDel-SP-TM), ou
(ii) SEQ ID NO : 39 (pDel-SP-HA-TM).

11. Procédé selon l'une quelconque des revendications **1** à **10,** dans lequel ladite sélection à partir de ladite population de lymphocytes B isolés d'une sous-population de lymphocytes B comprend les étapes de :
(a) mise en contact de ladite population de lymphocytes B isolés avec ledit antigène d'intérêt ou déterminant antigénique de celui-ci, dans lequel de préférence ledit antigène d'intérêt ou déterminant antigénique de celui-ci est marqué avec un colorant fluorescent ; et
(b) sélection des lymphocytes B se liant spécifiquement audit antigène d'intérêt ou déterminant antigénique de celui-ci, de préférence par la technique de tri par FACS.

12. Procédé selon l'une quelconque des revendications **1** à **11,** dans lequel ladite sélection à partir de ladite population de lymphocytes B isolés d'une sous-population de lymphocytes B comprend les étapes de :
(a) mise en contact de ladite population de lymphocytes B isolés avec ledit antigène d'intérêt ou déterminant antigénique de celui-ci, dans lequel ledit antigène d'intérêt ou déterminant antigénique de celui-ci est marqué avec un premier colorant fluorescent ;
(b) mise en contact des cellules de ladite population de lymphocytes B isolés avec des anticorps anti-IgM et/ou anti-IgD, dans lequel lesdits anticorps anti-IgM et/ou anti-IgD sont marqués avec un second colorant fluorescent ; et
(c) sélection des lymphocytes B liés audit antigène d'intérêt ou déterminant antigénique de celui-ci mais non liés auxdits anticorps anti-IgM et/ou non liés auxdits anticorps anti-IgD par la technique de tri par FACS.

13. Procédé selon l'une quelconque des revendications **1** à **12,** dans lequel ladite introduction de ladite banque d'expression alphavirale dans une première population de cellules de mammifères est réalisée par l'infection de ladite cellule de mammifère avec ladite banque d'expression alphavirale.

14. Procédé selon l'une quelconque des revendications **1** à **13,** dans lequel ledit isolement de ladite cellule individuelle est réalisé par la technique de tri par FACS, dans lequel, de préférence, ledit isolement de ladite cellule individuelle comprend les étapes de :
(a) coloration de ladite première population de cellules de mammifères avec ledit antigène d'intérêt ou déterminant antigénique de celui-ci, dans lequel ledit antigène d'intérêt ou déterminant antigénique de celui-ci est marqué avec un colorant fluorescent ; et
(b) séparation d'une cellule individuelle se liant spécifiquement audit antigène d'intérêt ou déterminant antigénique de celui-ci, au moyen de la technique de tri par FACS;
et dans lequel, de manière davantage préférée, ledit procédé comprend en outre les étapes de :
(a) culture d'au moins une de ladite cellule individuelle en présence d'une seconde population de cellules de mammifères ;
(b) vérification de la capacité de ladite seconde population de cellules de mammifères à se lier spécifiquement audit antigène d'intérêt ou déterminant antigénique de celui-ci.

15. Procédé selon l'une quelconque des revendications **1** à **14,** dans lequel ladite première population de cellules de mammifères et/ou ladite seconde population de cellules de mammifères comprend ou consiste en des cellules choisies parmi :
(a) des cellules BHK 21 ;
(b) des cellules Neuro-2a ; et
(c) des cellules HEK-293T.

16. Procédé selon l'une quelconque des revendications **1** à **15,**
(a) dans lequel l'anticorps exprimé par la cellule à isoler est un anticorps à chaîne unique,
(b) dans lequel ladite population de lymphocytes B isolés utilisés pour la sélection de l'étape (a) de la revendication 1 comprend ou consiste en des cellules mononucléées du sang périphérique (PBMCs) ; et/ou
(c) dans lequel chaque membre de ladite banque d'expression alphavirale code pour un anticorps comprenant une région variable de chaîne lourde (HCVR) et une région variable de chaîne légère (LCVR).


### SEQUENCE LISTING

<110> Cytos Biotechnology AG Bachmann, Martin Bauer, Monika Beerli, Roger
<120> SELECTION OF HUMAN MONOCLONAL ANTIBODIES BY EUKARYOTIC CELL DISPLAY
<130> P1060PC00
<150> EP 06123620.4 <151> 2006-11-07
<160> 124
<170> PatentIn version 3.3
<210> 1
   <211> 285
   <212> DNA
   <213> artificial sequence
<220>
   <223> chemically synthesized
<400> 1
   gagtctagag ccaccatgga gacagacaca ctcctgctat gggtactgct gctctgggtt      60
   ccaggttcca ctggtgacta tgaggcccag gcggccggta ccgctagcgg ccaggccggc     120
   cgcaatgctg tgggccagga cacgcaggag gtcatcgtgg tgccacactc cttgcccttt     180
   aaggtggtgg tgatctcagc catcctggcc ctggtggtgc tcaccatcat ctcccttatc     240
   atcctcatca tgctttggca gaagaagcca cgttaggggc ccgag                      285
<210> 2
   <211> 339
   <212> PRT
   <213> artificial sequence
<220>
   <223> chemically synthesized
<400> 2
<210> 3
   <211> 335
   <212> PRT
   <213> artificial sequence
<220>
   <223> chemically synthesized
<400> 3
   Met Glu Thr Asp Thr Leu Leu Leu Trp Val Leu Leu Leu Trp Val Pro
   1 5 10 15
   Gly Ser Thr Gly Asp Tyr Glu Ala Gln Ala Ala Glu Leu Val Leu Thr
   20 25 30
   Gln Pro Pro Ser Val Ser Gly Ala Pro Gly Gln Arg Val Thr Ile Ser
   35 40 45
   Cys Thr Gly Ser Ser Ser Asn Ile Gly Ala Gly Tyr Asp Val His Trp
   50 55 60
   Tyr Gln Gln Leu Pro Gly Thr Ala Pro Gln Leu Leu Ile Tyr Gly Asn
   65 70 75 80
   Ile Asn Arg Pro Ser Gly Val Pro Asp Arg Ser Ser Gly Ser Lys Ser
   85 90 95
   Gly Thr Ser Ala Ser Leu Ala Ile Thr Gly Leu Arg Ala Glu Asp Glu
   100 105 110
   Val Asp Tyr Tyr Cys Gln Ser Tyr Asp Arg Thr Leu Ser Gly Val Ile
   115 120 125
   Phe Gly Gly Gly Thr Gln Leu Thr Val Leu Gly Gly Gly Ser Ser Arg
   130 135 140
   Ser Ser Ser Ser Gly Gly Gly Gly Ser Gly Gly Gly Gly Gln Ile Thr
   145 150 155 160
   Leu Lys Glu Ser Gly Gly Gly Val Val Gln Pro Gly Ser Ser Arg Thr
   165 170 175
   Leu Ser Cys Glu Ala Ser Gly Phe Ser Phe Ser Thr Tyr Trp Met Thr
   180 185 190
   Trp Val Arg Gln Ala Pro Gly Lys Gly Leu Glu Trp Val Ala Asn Ile
   195 200 205
   Lys Gln Asp Gly Ser Glu Lys Tyr Tyr Val Asp Ser Val Lys Gly Arg
   210 215 220
   Phe Thr Ile Ser Arg Asp Asn Ala Lys Asn Ser Leu Tyr Leu Gln Met
   225 230 235 240
   Asn Ser Leu Arg Ala Glu Asp Thr Ala Val Tyr Tyr Cys Ser Arg Gly
   245 250 255
   Phe Phe Tyr Trp Gly Gln Gly Ala Leu Val Thr Val Ser Ser Ala Ser
   260 265 270
   Thr Lys Gly Pro Ser Val Thr Ser Gly Gln Ala Gly Arg Asn Ala Val
   275 280 285
   Gly Gln Asp Thr Gln Glu Val Ile Val Val Pro His Ser Leu Pro Phe
   290 295 300
   Lys Val Val Val Ile Ser Ala Ile Leu Ala Leu Val Val Leu Thr Ile
   305 310 315 320
   Ile Ser Leu Ile Ile Leu Ile Met Leu Trp Gln Lys Lys Pro Arg
   325 330 335
<210> 4
   <211> 349
   <212> PRT
   <213> artificial sequence
<220>
   <223> chemically synthesized
<400> 4
   Met Glu Thr Asp Thr Leu Leu Leu Trp Val Leu Leu Leu Trp Val Pro
   1               5                   10                  15
   Gly Ser Thr Gly Asp Tyr Glu Ala Gln Ala Ala Glu Leu Pro Ser Gln
   20 25 30
   Ser Pro Ser Val Ser Gly Ser Pro Gly Gln Ser Ile Thr Ile Ser Cys
   35 40 45
   Thr Gly Thr Ser Ser Asp Phe Gly Gly Tyr Lys Phe Val Ser Trp Tyr
   50 55 60
   Gln Gln His Pro Gly Lys Ala Pro Lys Leu Ile Ile Phe Asp Val Ser
   65 70 75 80
   Arg Arg Pro Ala Gly Val Ser Asn Arg Phe Ser Gly Ser Lys Ser Gly
   85 90 95
   Asn Thr Ala Ser Leu Thr Ile Ser Gly Leu Gln Ala Asp Asp Glu Ala
   100 105 110
   Glu Tyr Tyr Cys Ser Ser Tyr Lys Ser Gly Thr Thr Leu Tyr Val Phe
   115 120 125
   Gly Thr Gly Thr Glu Leu Thr Val Leu Gly Gly Gly Ser Ser Arg Ser
   130 135 140
   Ser Ser Ser Gly Gly Gly Gly Ser Gly Gly Gly Gly Glu Val Gln Leu
   145 150 155 160
   Val Gln Ser Gly Pro Gly Leu Leu Lys Pro Ser Glu Thr Leu Ser Leu
   165 170 175
   Thr Cys Ser Val Ser Gly Gly Ser Val Ala Ser Ser Ser Tyr Tyr Trp
   180 185 190
   Ser Trp Ile Arg Gln Ser Pro Arg Lys Gly Leu Glu Trp Ile Gly His
   195 200 205
   Ile Phe Tyr Ser Gly Ala Ala Lys Tyr Ser Pro Ser Leu Arg Ser Arg
   210 215 220
   Ala Thr Ile Ser Val Asp Thr Ser Arg Asn Gln Phe Asn Leu Lys Leu
   225 230 235 240
   Ser Ser Val Thr Ala Ala Asp Thr Ala Thr Tyr Tyr Cys Ala Arg Asp
   245 250 255
   Ala His Leu Ile Val Val Pro Ile Ala Gly Ala Leu Gly Ala Phe Asp
               260                 265                 270
   Val Trp Gly Gln Gly Thr Val Val Ala Val Ser Ser Ala Ser Thr Lys
           275                 280                 285
   Gly Pro Ser Val Thr Ser Gly Gln Ala Gly Arg Asn Ala Val Gly Gln
       290                 295                 300
   Asp Thr Gln Glu Val Ile Val Val Pro His Ser Leu Pro Phe Lys Val
   305                 310                 315                 320
   Val Val Ile Ser Ala Ile Leu Ala Leu Val Val Leu Thr Ile Ile Ser
                   325                 330                 335
   Leu Ile Ile Leu Ile Met Leu Trp Gln Lys Lys Pro Arg
               340                 345
<210> 5
   <211> 349
   <212> PRT
   <213> artificial sequence
<220>
   <223> chemically synthesized
<400> 5
   Met Glu Thr Asp Thr Leu Leu Leu Trp Val Leu Leu Leu Trp Val Pro
   1               5                   10                  15
   Gly Ser Thr Gly Asp Tyr Glu Ala Gln Ala Ala Glu Leu Leu Leu Thr
               20                  25                  30
   Gln Pro Pro Ser Val Ser Gly Ala Pro Gly Gln Arg Ala Thr Ile Ser
           35                  40                  45
   Cys Thr Gly Ser Ser Ser Asn Ile Gly Ala Gly Tyr Gly Val Gln Trp
       50                  55                  60
   Tyr Gln Gln Leu Pro Gly Thr Ala Pro Lys Leu Leu Ile Phe Gly Asn
   65                  70                  75                  80
   Asn Asn Arg Pro Ser Gly Val Pro Ala Arg Phe Ser Ala Ser Lys Ser
                   85                  90                  95
   Gly Thr Ser Ala Ser Leu Thr Ile Thr Gly Leu Gln Ala Glu Asp Glu
               100                 105                 110
   Ala Asp Tyr Tyr Cys Arg Ser Tyr Arg Ser Gly Val Ser Leu Ser Val
           115                 120                 125
   Phe Gly Thr Gly Thr Lys Leu Thr Val Leu Gly Gly Gly Ser Ser Arg
       130                 135                 140
   Ser Ser Ser Ser Gly Gly Gly Gly Ser Gly Gly Gly Gly Gln Val Gln
   145                 150                 155                 160
   Leu Val Gln Ser Gly Pro Gly Leu Val Lys Pro Ser Glu Thr Leu Ser
                   165                 170                 175
   Leu Thr Cys Ser Val Ser Gly Gly Ser Val Ser Asp Ala Ser Tyr Cys
               180                 185                 190
   Trp Thr Trp Ile Arg Gln Pro Pro Gly Lys Gly Leu Glu Trp Ile Gly
           195                 200                 205
   His Thr Ile Tyr Ser Gly Lys Thr Ser Tyr Asn Pro Ser Leu Lys Ser
       210                 215                 220
   Arg Val Ala Ile Ser Leu Asp Thr Ser Gln Asn His Phe Ser Leu Arg
   225                 230                 235                 240
   Leu Thr Ser Val Thr Ala Ala Asp Thr Ala Val Tyr Tyr Cys Ala Arg
                   245                 250                 255
   Gly Ala Cys Tyr Arg Ser Asn Trp Tyr Pro Leu Lys His Phe Phe Asp
               260                 265                 270
   Tyr Trp Gly Gln Gly Ala Leu Val Ala Val Ser Ser Ala Ser Thr Lys
           275                 280                 285
   Gly Pro Ser Val Thr Ser Gly Gln Ala Gly Arg Asn Ala Val Gly Gln
       290                 295                 300
   Asp Thr Gln Glu Val Ile Val Val Pro His Ser Leu Pro Phe Lys Val
   305                 310                 315                 320
   Val Val Ile Ser Ala Ile Leu Ala Leu Val Val Leu Thr Ile Ile Ser
                   325                 330                 335
   Leu Ile Ile Leu Ile Met Leu Trp Gln Lys Lys Pro Arg
               340                 345
<210> 6
   <211> 199
   <212> PRT
   <213> artificial sequence
<220>
   <223> chemically synthesized
<400> 6
   Met Glu Thr Asp Thr Leu Leu Leu Trp Val Leu Leu Leu Trp Val Pro
   1               5                   10                  15
   Gly Ser Thr Gly Asp Tyr Glu Ala Gln Ala Ala Glu Leu Thr Leu Thr
               20                  25                  30
   Gln Ser Pro Ala Thr Leu Ser Val Ser Pro Gly Glu Ser Ala Thr Leu
           35                  40                  45
   Ser Cys Arg Ala Ser Gln Ser Val Arg Arg Asn Leu Ala Trp Tyr Gln
       50                  55                  60
   Gln Arg Pro Gly Gln Ala Pro Arg Leu Leu Ile Tyr Gly Ala Thr Thr
   65                  70                  75                  80
   Arg Ala Thr Gly Val Pro Val Arg Ile Ser Gly Ser Gly Ser Gly Thr
                   85                  90                  95
   Glu Phe Thr Leu Thr Ile Ser Ser Leu Gln Ser Glu Asp Phe Val Val
               100                 105                 110
   Tyr Tyr Cys Gln Gln Tyr Asn Asp Trp Pro Gly Thr Phe Gly Gln Gly
           115                 120                 125
   Thr Lys Val Asp Ile Lys Gly Gly Ser Ser Arg Ser Ser Ser Ser Gly
       130                 135                 140
   Gly Gly Gly Ser Gly Gly Gly Gly Glu Val Gln Leu Val Glu Ser Gly
   145                 150                 155                 160
   Pro Gly Leu Val Lys Pro Ser Gly Thr Leu Ser Leu Thr Cys Ala Val
                   165                 170                 175
   Ser Gly Val Ser Ile Thr Ser Ser Asn Trp Trp Ser Trp Val Arg Gln
               180                 185                 190
   Pro Pro Gly Lys Gly Pro Glu
           195
<210> 7
   <211> 347
   <212> PRT
   <213> artificial sequence
<220>
   <223> chemically synthesized
<400> 7
   Met Glu Thr Asp Thr Leu Leu Leu Trp Val Leu Leu Leu Trp Val Pro
   1               5                   10                  15
   Gly Ser Thr Gly Asp Tyr Glu Ala Gln Ala Ala Glu Leu Gln Met Thr
               20                  25                  30
   Gln Ser Pro Ser Thr Leu Ser Ala Ser Val Gly Asp Arg Val Thr Ile
           35                  40                  45
   Thr Cys Arg Ala Ser Gln Gly Ile Ser Ser Tyr Leu Val Trp Tyr Gln
       50                  55                  60
   Gln Lys Pro Gly Lys Ala Pro Lys Leu Leu Ile Tyr Asp Ser Ser Thr
   65                  70                  75                  80
   Leu Gln Ser Gly Val Pro Ser Arg Phe Ser Gly Ser Gly Ser Gly Thr
                   85                  90                  95
   Asp Phe Thr Leu Thr Ile Ser Ser Leu Gln Pro Glu Asp Val Ala Ala
               100                 105                 110
   Tyr Phe Cys Gln Gln Val Tyr Ser Tyr Pro Arg Thr Phe Gly Gln Gly
           115                 120                 125
   Thr Lys Val Asp Ile Lys Gly Gly Ser Ser Arg Ser Ser Ser Ser Gly
       130                 135                 140
   Gly Gly Gly Ser Gly Gly Gly Gly Gln Ile Thr Leu Lys Glu Ser Gly
   145                 150                 155                 160
   Gly Gly Leu Val Lys Pro Gly Gly Ser Leu Arg Leu Ser Cys Val Ala
                   165                 170                 175
   Ser Gly Leu Ser Phe Lys Asp Ala Trp Met Ser Trp Val Arg Gln Ala
               180                 185                 190
   Pro Gly Lys Gly Leu Glu Trp Val Gly Arg Met Lys Ser Arg Ala Ser
           195                 200                 205
   Gly Gly Thr Thr Glu Tyr Gly Gly Leu Ala Asn Gly Arg Phe Thr Ile
       210                 215                 220
   Ser Arg Asp Asp Ser Lys Asn Thr Leu Phe Leu Gln Ile Asn Arg Leu
   225                 230                 235                 240
   Glu Thr Glu Asp Thr Ala Val Tyr Tyr Cys Thr Phe Ala Phe Cys Ser
                   245                 250                 255
   Gly Thr Ser Cys Tyr Gly Gln Tyr Thr Tyr Tyr Gly Leu Asp Val Trp
               260                 265                 270
   Gly Gln Gly Thr Thr Val Ile Val Ser Ser Ala Ser Thr Lys Gly Pro
           275                 280                 285
   Ser Val Thr Ser Gly Gln Ala Gly Arg Asn Ala Val Gly Gln Asp Thr
       290                 295                 300
   Gln Glu Val Ile Val Val Pro His Ser Leu Pro Phe Lys Val Val Val
   305                 310                 315                 320
   Ile Ser Ala Ile Leu Ala Leu Val Val Leu Thr Ile Ile Ser Leu Ile
                   325                 330                 335
   Ile Leu Ile Met Leu Trp Gln Lys Lys Pro Arg
               340                 345
<210> 8
   <211> 348
   <212> PRT
   <213> artificial sequence
<220>
   <223> chemically synthesized
<400> 8
   Met Glu Thr Asp Thr Leu Leu Leu Trp Val Leu Leu Leu Trp Val Pro
   1               5                   10                  15
   Gly Ser Thr Gly Asp Tyr Glu Ala Gln Ala Ala Glu Leu Gly Leu Thr
               20                  25                  30
   Gln Pro Pro Ser Val Ser Gly Ala Pro Gly Gln Arg Val Thr Ile Ser
           35                  40                  45
   Cys Thr Gly Ser Ser Ser Asn Ile Gly Arg Phe Asp Val His Trp Tyr
   50 55 60
   Gln Gln Leu Pro Gly Thr Ala Pro Lys Leu Leu Ile Tyr Gly Asn Thr
   65 70 75 80
   Asn Arg Pro Ser Gly Val Pro Asp Arg Phe Ser Gly Ser Lys Ser Gly
   85 90 95
   Ser Ser Ala Ser Leu Ala Ile Thr Gly Leu Gln Ala Glu Asp Glu Ala
   100 105 110
   Asp Tyr Tyr Cys Gln Ser Tyr Asp Arg Ser Leu Ser Gly Val Val Phe
   115 120 125
   Gly Gly Gly Thr Gln Leu Thr Val Leu Gly Gly Gly Ser Ser Arg Ser
   130 135 140
   Ser Ser Ser Gly Gly Gly Gly Ser Gly Gly Gly Gly Glu Val Gln Leu
   145 150 155 160
   Leu Glu Ser Gly Pro Gly Leu Val Lys Pro Ser Glu Thr Leu Ser Leu
   165 170 175
   Thr Cys Thr Val Ser Gly Gly Ser Ile Ser Ser Gly Asn Tyr Tyr Trp
   180 185 190
   Ser Trp Ile Arg Gln Thr Pro Glu Lys Gly Leu Glu Trp Leu Gly Tyr
   195 200 205
   Val His Tyr Thr Gly Ser Ser Lys Leu Asn Pro Ser Leu Lys Ser Arg
   210 215 220
   Val Thr Ile Ser Val Asp Thr Tyr Thr Asn Gln Phe Ser Leu Ser Leu
   225 230 235 240
   Ser Ser Met Thr Ala Ala Asp Thr Ala Val Tyr Tyr Cys Ala Arg Gly
   245 250 255
   Lys Asn Cys Ala Asn Asp Ile Cys Tyr Ile Gly Ser Trp Phe Asp Pro
   260 265 270
   Trp Gly Gln Gly Thr Leu Val Thr Val Ser Ser Ala Ser Thr Lys Gly
   275 280 285
   Pro Ser Val Thr Ser Gly Gln Ala Gly Arg Asn Ala Val Gly Gln Asp
   290 295 300
   Thr Gln Glu Val Ile Val Val Pro His Ser Leu Pro Phe Lys Val Val
   305                 310                 315                 320
   Val Ile Ser Ala Ile Leu Ala Leu Val Val Leu Thr Ile Ile Ser Leu
                   325                 330                 335
   Ile Ile Leu Ile Met Leu Trp Gln Lys Lys Pro Arg
               340                 345
<210> 9
   <211> 344
   <212> PRT
   <213> artificial sequence
<220>
   <223> chemically synthesized
<400> 9
   Met Glu Thr Asp Thr Leu Leu Leu Trp Val Leu Leu Leu Trp Val Pro
   1               5                   10                  15
   Gly Ser Thr Gly Asp Tyr Glu Ala Gln Ala Ala Glu Leu Val Met Thr
               20                  25                  30
   Gln Ser Pro Ser Ser Leu Ser Ala Ser Val Gly Asp Arg Val Thr Ile
           35                  40                  45
   Thr Cys Arg Ala Ser Gln Gly Val Ser Arg Ala Leu Ala Trp Tyr Gln
       50                  55                  60
   Gln Lys Pro Gly Asn Pro Pro Lys Leu Leu Ile Tyr Asp Ala Ser Asn
   65                  70                  75                  80
   Leu Gln Ser Gly Val Pro Ser Arg Phe Ser Gly Gly Gly Ser Gly Thr
                   85                  90                  95
   Glu Phe Ile Leu Thr Ile Ser Ser Leu Gln Pro Glu Asp Phe Ala Thr
               100                 105                 110
   Tyr Tyr Cys Gln Gln Tyr Asn Ala Tyr Pro Trp Thr Phe Gly Gln Gly
           115                 120                 125
   Thr Lys Leu Glu Ile Lys Gly Gly Ser Ser Arg Ser Ser Ser Ser Gly
       130                 135                 140
   Gly Gly Gly Ser Gly Gly Gly Gly Gln Val Gln Leu Gln Glu Ser Gly
   145 150 155 160
   Pro Gly Leu Val Lys Pro Ser Glu Thr Leu Ser Leu Thr Cys Thr Val
   165 170 175
   Ser Gly Gly Ser Ile Ser Ser Gly Asn Tyr Tyr Trp Ser Trp Ile Arg
   180 185 190
   Gln Thr Pro Glu Lys Gly Leu Glu Trp Leu Gly Tyr Val His Tyr Thr
   195 200 205
   Gly Ser Ser Lys Leu Asn Pro Ser Leu Lys Ser Arg Val Thr Ile Ser
   210 215 220
   Val Asp Thr Tyr Thr Asn Gln Phe Ser Leu Ser Leu Ser Ser Met Thr
   225 230 235 240
   Ala Ala Asp Thr Ala Val Tyr Tyr Cys Ala Arg Gly Lys Asn Cys Ala
   245 250 255
   Asn Asp Ile Cys Tyr Ile Gly Ser Trp Phe Asp Pro Trp Gly Gln Gly
   260 265 270
   Thr Leu Val Thr Val Ser Ser Ala Ser Thr Lys Gly Pro Ser Val Thr
   275 280 285
   Ser Gly Gln Ala Gly Arg Asn Ala Val Gly Gln Asp Thr Gln Glu Val
   290 295 300
   Ile Val Val Pro His Ser Leu Pro Phe Lys Val Val Val Ile Ser Ala
   305 310 315 320
   Ile Leu Ala Leu Val Val Leu Thr Ile Ile Ser Leu Ile Ile Leu Ile
   325 330 335
   Met Leu Trp Gln Lys Lys Pro Arg
   340
<210> 10
   <211> 349
   <212> PRT
   <213> artificial sequence
<220>
   <223> chemically synthesized
<400> 10
   Met Glu Thr Asp Thr Leu Leu Leu Trp Val Leu Leu Leu Trp Val Pro
   1 5 10 15
   Gly Ser Thr Gly Asp Tyr Glu Ala Gln Ala Ala Glu Leu Val Leu Thr
   20 25 30
   Gln Pro Pro Ser Val Ser Gly Ala Pro Gly Gln Arg Val Thr Ile Ser
   35 40 45
   Cys Thr Gly Thr Ser Ser Asn Ile Gly Ala Gly Tyr Ala Val His Trp
   50 55 60
   Tyr Gln Gln Val Pro Gly Thr Ala Pro Lys Leu Leu Ile Phe Gly Lys
   65 70 75 80
   Thr Asn Arg Pro Ser Gly Val Pro Gly Arg Phe Ser Gly Ser Lys Ala
   85 90 95
   Gly Thr Ser Ala Ser Leu Ala Ile Thr Gly Leu Gln Pro Glu Asp Glu
   100 105 110
   Ala His Tyr Tyr Cys Gln Ser Tyr Asp Ser Asn Leu Ser Glu Val Val
   115 120 125
   Phe Gly Gly Gly Thr Gln Leu Thr Val Leu Gly Gly Gly Ser Ser Arg
   130 135 140
   Ser Ser Ser Ser Gly Gly Gly Gly Ser Gly Gly Gly Gly Glu Val Gln
   145 150 155 160
   Leu Val Gln Ser Gly Pro Gly Leu Val Lys Pro Ser Glu Thr Leu Ser
   165 170 175
   Leu Thr Cys Thr Val Ser Gly Gly Ser Ile Ser Ser Gly Asn Tyr Tyr
   180 185 190
   Trp Ser Trp Ile Arg Gln Thr Pro Glu Lys Gly Leu Glu Trp Leu Gly
   195 200 205
   Tyr Val His Tyr Thr Gly Ser Ser Lys Leu Asn Pro Ser Leu Lys Ser
   210 215 220
   Arg Val Thr Ile Ser Val Asp Thr Tyr Thr Asn Gln Phe Ser Leu Ser
   225 230 235 240
   Leu Ser Ser Met Thr Ala Ala Asp Thr Ala Val Tyr Tyr Cys Ala Arg
   245 250 255
   Gly Lys Asn Cys Ala Asn Asp Ile Cys Tyr Ile Gly Ser Trp Phe Asp
   260 265 270
   Pro Trp Gly Gln Gly Thr Leu Val Thr Val Ser Ser Ala Ser Thr Lys
   275 280 285
   Gly Pro Ser Val Thr Ser Gly Gln Ala Gly Arg Asn Ala Val Gly Gln
   290 295 300
   Asp Thr Gln Glu Val Ile Val Val Pro His Ser Leu Pro Phe Lys Val
   305 310 315 320
   Val Val Ile Ser Ala Ile Leu Ala Leu Val Val Leu Thr Ile Ile Ser
   325 330 335
   Leu Ile Ile Leu Ile Met Leu Trp Gln Lys Lys Pro Arg
   340 345
<210> 11
   <211> 347
   <212> PRT
   <213> artificial sequence
<220>
   <223> chemically synthesized
<400> 11
<210> 12
   <211> 348
   <212> PRT
   <213> artificial sequence
<220>
   <223> chemically synthesized
<400> 12
   Met Glu Thr Asp Thr Leu Leu Leu Trp Val Leu Leu Leu Trp Val Pro
   1               5                   10                  15
   Gly Ser Thr Gly Asp Tyr Glu Ala Gln Ala Ala Glu Leu Val Leu Thr
               20                  25                  30
   Gln Pro Pro Ser Val Ser Gly Leu Pro Gly Gln Ser Val Thr Val Ser
           35                  40                  45
   Cys Thr Gly Thr Ser Ser Asp Val Ser His Ser Asn Tyr Val Ser Trp
       50                  55                  60
   Tyr Gln Gln Leu Pro Gly Lys Ala Pro Lys Leu Ile Ile Tyr Asp Val
   65                  70                  75                  80
   Thr Lys Arg Pro Ser Gly Val Pro Asn Arg Phe Ser Gly Ser Lys Ser
                   85                  90                  95
   Gly Asn Thr Ala Ser Leu Thr Ile Ser Gly Leu Gln Thr Glu Asp Glu
               100                 105                 110
   Ala Asp Tyr His Cys Cys Ser Tyr Ala Gly Gly Tyr Thr Trp Val Phe
           115                 120                 125
   Gly Gly Gly Thr Gln Leu Thr Val Leu Gly Gly Gly Ser Ser Arg Ser
       130                 135                 140
   Ser Ser Ser Gly Gly Gly Gly Ser Gly Gly Gly Gly Glu Val Gln Leu
   145                 150                 155                 160
   Val Glu Ser Gly Pro Gly Leu Val Lys Pro Ser Glu Thr Leu Ser Leu
                   165                 170                 175
   Thr Cys Thr Val Ser Gly Gly Ser Ile Ser Ser Gly Asn Tyr Tyr Trp
               180                 185                 190
   Ser Trp Ile Arg Gln Thr Pro Glu Lys Gly Leu Glu Trp Leu Gly Tyr
   195 200 205
   Val His Tyr Thr Gly Ser Ser Lys Leu Asn Pro Ser Leu Lys Ser Arg
   210 215 220
   Val Thr Ile Ser Val Asp Thr Tyr Thr Asn Gln Phe Ser Leu Ser Leu
   225 230 235 240
   Ser Ser Met Thr Ala Ala Asp Thr Ala Val Tyr Tyr Cys Ala Arg Gly
   245 250 255
   Lys Asn Cys Ala Asn Asp Ile Cys Tyr Ile Gly Ser Trp Phe Asp Pro
   260 265 270
   Trp Gly Gln Gly Thr Leu Val Thr Val Ser Ser Ala Ser Thr Lys Gly
   275 280 285
   Pro Ser Val Thr Ser Gly Gln Ala Gly Arg Asn Ala Val Gly Gln Asp
   290 295 300
   Thr Gln Glu Val Ile Val Val Pro His Ser Leu Pro Phe Lys Val Val
   305 310 315 320
   Val Ile Ser Ala Ile Leu Ala Leu Val Val Leu Thr Ile Ile Ser Leu
   325 330 335
   Ile Ile Leu Ile Met Leu Trp Gln Lys Lys Pro Arg
   340 345
<210> 13
   <211> 349
   <212> PRT
   <213> artificial sequence
<220>
   <223> chemically synthesized
<400> 13
   Met Glu Thr Asp Thr Leu Leu Leu Trp Val Leu Leu Leu Trp Val Pro
   1 5 10 15
   Gly Ser Thr Gly Asp Tyr Glu Ala Gln Ala Ala Glu Leu Val Leu Thr
   20 25 30
   Gln Pro Pro Ser Met Ser Gly Ala Pro Gly Gln Arg Val Ser Ile Ser
   35 40 45
   Cys Thr Gly Ser Ser Ser Asn Ile Gly Ala Arg Tyr Asp Val His Trp
       50                  55                  60
   Tyr Gln Gln Leu Pro Gly Thr Ala Pro Lys Leu Leu Ile Tyr Gly Asn
   65                  70                  75                  80
   Thr Asn Arg Pro Ser Gly Val Pro Asp Arg Phe Ser Gly Ser Lys Ser
                   85                  90                  95
   Gly Ser Ser Ala Ser Leu Ala Ile Thr Gly Leu Gln Ala Glu Asp Glu
               100                 105                 110
   Ala Asp Tyr Tyr Cys Gln Ser Tyr Asp Arg Ser Leu Ser Gly Val Val
           115                 120                 125
   Phe Gly Gly Gly Thr Lys Leu Thr Val Leu Gly Gly Gly Ser Ser Arg
       130                 135                 140
   Ser Ser Ser Ser Gly Gly Gly Gly Ser Gly Gly Gly Gly Gln Ile Thr
   145                 150                 155                 160
   Leu Lys Glu Ser Gly Pro Gly Leu Val Lys Pro Ser Glu Thr Leu Ser
                   165                 170                 175
   Leu Thr Cys Thr Val Ser Gly Gly Phe Ile Ser Ser Ser Ser Tyr Tyr
               180                 185                 190
   Trp Gly Trp Ile Arg Gln Pro Pro Gly Lys Gly Leu Glu Trp Ile Gly
           195                 200                 205
   Ser Ser Tyr Tyr Gly Gly Ser Thr Asn Tyr Asn Pro Ser Leu Lys Ser
       210                 215                 220
   Arg Val Thr Ile Leu Val Asp Arg Ser Lys Asn Gln Phe Ser Leu Lys
   225                 230                 235                 240
   Leu Ser Ser Val Thr Ala Ala Asp Thr Ala Val Tyr Tyr Cys Ala Arg
                   245                 250                 255
   Ser Thr Val Ala Val Val Ser Met Ala Gly Pro Ser Gly Trp Phe Asp
               260                 265                 270
   Pro Trp Gly Gln Gly Ile Met Val Thr Val Ser Ser Ala Ser Thr Lys
           275                 280                 285
   Gly Pro Ser Val Thr Ser Gly Gln Ala Gly Arg Asn Ala Val Gly Gln
       290                 295                 300
   Asp Thr Gln Glu Val Ile Val Val Pro His Ser Leu Pro Phe Lys Val
   305                 310                 315                 320
   Val Val Ile Ser Ala Ile Leu Ala Leu Val Val Leu Thr Ile Ile Ser
                   325                 330                 335
   Leu Ile Ile Leu Ile Met Leu Trp Gln Lys Lys Pro Arg
               340                 345
<210> 14
   <211> 346
   <212> PRT
   <213> artificial sequence
<220>
   <223> chemically synthesized
<210> 15
   <211> 342
   <212> PRT
   <213> artificial sequence
<220>
   <223> chemically synthesized
<210> 16
   <211> 347
   <212> PRT
   <213> artificial sequence
<220>
   <223> chemically synthesized
<400> 16
   Met Glu Thr Asp Thr Leu Leu Leu Trp Val Leu Leu Leu Trp Val Pro
   1 5 10 15
   Gly Ser Thr Gly Asp Tyr Glu Ala Gln Ala Ala Glu Leu Glu Leu Thr
   20 25 30
   Gln Pro Pro Ser Val Ser Gly Ala Pro Gly Gln Arg Val Ser Ile Ser
   35 40 45
   Cys Thr Gly Ser Ser Ser Asn Ile Gly Ala Arg Tyr Asp Val His Trp
   50 55 60
   Tyr Gln Gln Leu Pro Gly Thr Ala Pro Lys Leu Leu Ile Tyr Gly Asn
   65 70 75 80
   Thr Asn Arg Pro Ser Gly Val Pro Asp Arg Phe Ser Gly Ser Lys Ser
   85 90 95
   Gly Ser Ser Ala Ser Leu Ala Ile Thr Gly Leu Gln Ala Glu Asp Glu
   100 105 110
   Ala Asp Tyr Tyr Cys Gln Ser Tyr Asp Arg Ser Leu Ser Gly Val Val
   115 120 125
   Phe Gly Gly Gly Thr Lys Val Thr Val Leu Gly Gly Gly Ser Ser Arg
   130 135 140
   Ser Ser Ser Ser Gly Gly Gly Gly Ser Gly Gly Gly Gly Gln Ile Thr
   145 150 155 160
   Leu Lys Glu Ser Gly Pro Gly Leu Val Arg Pro Ser Glu Thr Leu Ser
   165 170 175
   Leu Thr Cys Ser Val Ser Gly Gly Ser Ile Asp Ser Thr Ser Tyr Ser
   180 185 190
   Trp Gly Trp Ile Arg Gln Pro Pro Gly Lys Gly Leu Glu Trp Ile Ala
   195 200 205
   Ser Ile His Tyr Lys Gly Arg Thr Gln Tyr Asn Pro Ser Leu Lys Ser
   210 215 220
   Arg Leu Thr Ile Ser Val Asp Pro Ser Arg Ser Gln Phe Ser Leu Arg
   225 230 235 240
   Leu Ser Ser Val Thr Ala Ala Asp Thr Ala Val Tyr Tyr Cys Ala Arg
   245 250 255
   Leu Tyr Tyr Ile Trp Gly Ser Tyr Gln Ser Gly Arg Phe Asp Tyr Trp
   260 265 270
   Gly Gln Gly Ser Leu Val Thr Val Ser Ser Ala Ser Thr Lys Gly Pro
   275 280 285
   Ser Val Thr Ser Gly Gln Ala Gly Arg Asn Ala Val Gly Gln Asp Thr
   290 295 300
   Gln Glu Val Ile Val Val Pro His Ser Leu Pro Phe Lys Val Val Val
   305 310 315 320
   Ile Ser Ala Ile Leu Ala Leu Val Val Leu Thr Ile Ile Ser Leu Ile
   325 330 335
   Ile Leu Ile Met Leu Trp Gln Lys Lys Pro Arg
   340 345
<210> 17
   <211> 350
   <212> PRT
   <213> artificial sequence
<220>
   <223> chemically synthesized
<400> 17
   Met Glu Thr Asp Thr Leu Leu Leu Trp Val Leu Leu Leu Trp Val Pro
   1               5                   10                  15
   Gly Ser Thr Gly Asp Tyr Glu Ala Gln Ala Ala Glu Leu Glu Leu Thr
               20                  25                  30
   Gln Pro Pro Ser Val Ser Gly Ala Pro Gly Gln Arg Val Thr Ile Ser
           35                  40                  45
   Cys Thr Gly Ser Asn Ser Asn Ile Gly Ala Gly Tyr Asp Val His Trp
       50                  55                  60
   Tyr Gln Gln Leu Pro Gly Thr Ala Pro Lys Leu Leu Ile Tyr Asn Asn
   65                  70                  75                  80
   Asn Asn Arg Pro Ser Gly Val Pro Asp Arg Phe Ser Gly Ser Gln Ser
                   85                  90                  95
   Gly Thr Ser Ala Ser Leu Ala Ile Thr Gly Val Gln Ala Glu Asp Glu
               100                 105                 110
   Ala Asp Tyr Tyr Cys Gln Ser Tyr Asp Ser Ser Leu Ser Gly Val Val
           115                 120                 125
   Phe Gly Gly Gly Thr Gln Leu Thr Val Leu Gly Gly Gly Ser Ser Arg
       130                 135                 140
   Ser Ser Ser Ser Gly Gly Gly Gly Ser Gly Gly Gly Gly Gly Val Gln
   145                 150                 155                 160
   Leu Val Glu Ser Gly Pro Arg Leu Val Lys Pro Ser Glu Thr Leu Ser
                   165                 170                 175
   Leu Thr Cys Phe Val Ser Gly Gly Ser Ile Ser Ser Ala Ser Tyr Gln
               180                 185                 190
   Trp Ser Trp Leu Arg Gln Arg Pro Gly Gln Gly Leu Glu Trp Ile Gly
   195 200 205
   Tyr Ile Tyr Tyr Ser Gly Ser Ser Asn Tyr Asn Pro Ser Leu Lys Arg
   210 215 220
   Arg Val Ser Phe Ser Ala Asp Ala Ser Lys Asn Gln Phe Ser Met Arg
   225 230 235 240
   Leu Val Ser Leu Thr Ala Ala Asp Thr Ala Val Tyr Tyr Cys Ala Arg
   245 250 255
   Gln Ser His Ile Ile Val Val Pro Thr Ala Gly Ala Leu Gly Thr Phe
   260 265 270
   Asp Ile Trp Gly His Gly Thr Met Val Thr Val Ser Ser Ala Ser Thr
   275 280 285
   Lys Gly Pro Ser Val Thr Ser Gly Gln Ala Gly Arg Asn Ala Val Gly
   290 295 300
   Gln Asp Thr Gln Glu Val Ile Val Val Pro His Ser Leu Pro Phe Lys
   305 310 315 320
   Val Val Val Ile Ser Ala Ile Leu Ala Leu Val Val Leu Thr Ile Ile
   325 330 335
   Ser Leu Ile Ile Leu Ile Met Leu Trp Gln Lys Lys Pro Arg
   340 345 350
<210> 18
   <211> 169
   <212> PRT
   <213> artificial sequence
<220>
   <223> chemically synthesized
<400> 18
   Met Glu Thr Asp Thr Leu Leu Leu Trp Val Leu Leu Leu Trp Val Pro
   1 5 10 15
   Gly Ser Thr Gly Asp Tyr Glu Ala Gln Ala Ala Glu Leu Val Met Thr
   20 25 30
   Gln Ser Pro Ala Thr Leu Ser Val Ser Pro Gly Glu Thr Ala Thr Leu
   35 40 45
   Ser Cys Arg Ala Ser Gln Ser Val Gly Ser Asn Leu Ala Trp Phe Gln
   50 55 60
   Gln Lys Pro Gly Gln Ala Pro Arg Leu Leu Ile Tyr Gly Ala Ser Thr
   65 70 75 80
   Arg Ala Thr Gly Ile Pro Ala Arg Phe Ser Gly Gly Gly Ser Gly Thr
   85 90 95
   Glu Phe Thr Leu Thr Ile Ser Ser Leu Gln Ser Glu Asp Phe Val Val
   100 105 110
   Tyr Tyr Cys His Gln Tyr Ala Asp Trp Pro Arg Thr Phe Gly Gln Gly
   115 120 125
   Thr Lys Val Glu Ile Lys Gly Gly Ser Ser Arg Ser Ser Ser Ser Gly
   130 135 140
   Gly Gly Gly Ser Gly Gly Gly Gly Gln Val Gln Leu Gln Gln Trp Gly
   145 150 155 160
   Glu Ala Trp Ser Ser Arg Gly Gly Pro
   165
<210> 19
   <211> 346
   <212> PRT
   <213> artificial sequence
<220>
   <223> chemically synthesized
<400> 19
   Met Glu Thr Asp Thr Leu Leu Leu Trp Val Leu Leu Leu Trp Val Pro
   1 5 10 15
   Gly Ser Thr Gly Asp Tyr Glu Ala Gln Ala Ala Glu Leu Val Leu Thr
   20 25 30
   Gln Pro Pro Ser Val Ser Gly Ala Pro Gly Gln Arg Val Thr Ile Ser
   35 40 45
   Cys Ser Gly Asn Ser Ser Asn Ile Gly Thr Arg Tyr Asp Val His Trp
   50 55 60
   Tyr Gln Gln Phe Pro Gly Thr Ala Pro Lys Leu Leu Ile Tyr Gly Asn
   65                  70                  75                  80
   Thr Asn Arg Pro Ser Gly Val Pro Asp Arg Phe Ser Gly Ser Thr Ser
                   85                  90                  95
   Gly Ala Ser Ala Ser Leu Ala Ile Thr Gly Leu Gln Ala Asp Asp Glu
               100                 105                 110
   Ala Asp Tyr Tyr Cys Gln Ser Tyr Asp Ser Ser Leu Arg Ala Thr Val
           115                 120                 125
   Phe Gly Gly Gly Thr Gln Leu Thr Val Leu Gly Gly Gly Ser Ser Arg
       130                 135                 140
   Ser Ser Ser Ser Gly Gly Gly Gly Ser Gly Gly Gly Gly Gln Val Gln
   145                 150                 155                 160
   Leu Val Gln Ser Gly Gly Gly Trp Val Gln Ser Gly Gly Ser Leu Arg
                   165                 170                 175
   Leu Ser Cys Ala Ala Ser Gly Phe Ser Phe Ser Ser Tyr Ala Met Ser
               180                 185                 190
   Trp Val Arg Gln Ala Pro Gly Lys Gly Leu Glu Trp Val Ser Ala Met
           195                 200                 205
   Ser Pro Ile Gly Gly Ser Thr Phe Tyr Ala Asp Ser Val Lys Gly Arg
       210                 215                 220
   Phe Thr Ile Ser Arg Asp Asn Ser Lys Asn Thr Leu Phe Leu Gln Met
   225                 230                 235                 240
   Asn Ser Leu Arg Ala Glu Asp Thr Ala Val Tyr Tyr Cys Ala Lys Asp
                   245                 250                 255
   Ala Val Val Thr Ala Val Gly Leu Gly Trp Tyr Phe Asp Leu Trp Gly
               260                 265                 270
   Arg Gly Thr Leu Val Ser Val Ser Ser Ala Ser Thr Lys Gly Pro Ser
           275                 280                 285
   Ala Thr Ser Gly Gln Ala Gly Arg Asn Ala Val Gly Gln Asp Thr Gln
       290                 295                 300
   Glu Val Ile Val Val Pro His Ser Leu Pro Phe Lys Val Val Val Ile
   305                 310                 315                 320
   Ser Ala Ile Leu Ala Leu Val Val Leu Thr Ile Ile Ser Leu Ile Ile
                   325                 330                 335
   Leu Ile Met Leu Trp Gln Lys Lys Pro Arg
               340                 345
<210> 20
   <211> 350
   <212> PRT
   <213> artificial sequence
<220>
   <223> chemically synthesized
<400> 20
   Met Glu Thr Asp Thr Leu Leu Leu Trp Val Leu Leu Leu Trp Val Pro
   1               5                   10                  15
   Gly Ser Thr Gly Asp Tyr Glu Ala Gln Ala Ala Glu Leu Gly Gln Thr
               20                  25                  30
   Gln Gln Leu Ser Val Ser Gly Ala Pro Gly Gln Ser Val Thr Ile Ser
           35                  40                  45
   Cys Thr Gly Gly Ser Ser Asn Ile Gly Ala Ser Tyr Asp Val His Trp
       50                  55                  60
   Tyr Lys Gln Leu Pro Gly Ala Ala Pro Ile Leu Leu Ile Tyr Ala Asn
   65                  70                  75                  80
   Tyr Ile Arg Pro Ser Gly Val Pro Asp Arg Phe Ser Ala Ser Lys Ser
                   85                  90                  95
   Gly Thr Ser Ala Ser Leu Ala Ile Thr Gly Leu Gln Ala Glu Asp Glu
               100                 105                 110
   Ala Asp Tyr Tyr Cys Gln Ser Tyr Asp Ser Ser Leu Ser Gly Val Val
           115                 120                 125
   Phe Gly Gly Gly Thr Lys Leu Thr Val Leu Gly Gly Gly Ser Ser Arg
       130                 135                 140
   Ser Ser Ser Ser Gly Gly Gly Gly Ser Gly Gly Gly Gly Glu Val Gln
   145                 150                 155                 160
   Leu Val Gln Ser Gly Pro Gly Leu Val Lys Pro Ser Gln Thr Leu Ser
   165 170 175
   Ile Thr Cys Thr Val Ser Gly Gly Ser Val Ser Asp Thr Ser Tyr Tyr
   180 185 190
   Trp Ala Trp Val Arg Gln Pro Pro Gly Lys Gly Leu Glu Trp Ile Ala
   195 200 205
   His Ala Phe Tyr Ser Gly Ser Ala Asn Tyr Asn Pro Ser Leu Lys Ser
   210 215 220
   Arg Ala Thr Ile Ser Val Asp Thr Ser Arg Asn Gln Phe Ser Leu Arg
   225 230 235 240
   Leu Asp Ser Val Thr Ala Ala Asp Thr Ala Val Tyr Tyr Cys Ala Arg
   245 250 255
   Glu Thr His Leu Val Val Val Pro Gly Ala Gly Ala Leu Gly Ala Phe
   260 265 270
   Asp Ile Trp Gly Gln Gly Thr Met Val Thr Val Ser Pro Ala Ser Thr
   275 280 285
   Lys Gly Pro Ser Val Thr Ser Gly Gln Ala Gly Arg Asn Ala Val Gly
   290 295 300
   Gln Asp Thr Gln Glu Val Ile Val Val Pro His Ser Leu Pro Phe Lys
   305 310 315 320
   Val Val Val Ile Ser Ala Ile Leu Ala Leu Val Val Leu Thr Ile Ile
   325 330 335
   Ser Leu Ile Ile Leu Ile Met Leu Trp Gln Lys Lys Pro Arg
   340 345 350
<210> 21
   <211> 343
   <212> PRT
   <213> artificial sequence
<220>
   <223> chemically synthesized
<400> 21
   Met Glu Thr Asp Thr Leu Leu Leu Trp Val Leu Leu Leu Trp Val Pro
   1               5                   10                  15
   Gly Ser Thr Gly Asp Tyr Glu Ala Gln Ala Ala Glu Leu Glu Leu Thr
               20                  25                  30
   Gln Pro Pro Ser Val Ser Gly Ser Pro Gly Gln Ser Val Thr Ile Ser
           35                  40                  45
   Cys Thr Gly Thr Ser Ser Asn Val Gly Gly Tyr Asn Tyr Val Ser Trp
       50                  55                  60
   Tyr Gln Gln Tyr Pro Gly Lys Ala Pro Lys Leu Met Ile Tyr Asp Val
   65                  70                  75                  80
   Thr Lys Arg Pro Ser Gly Val Pro Asp Arg Phe Ser Gly Ser Lys Ser
                   85                  90                  95
   Gly Ser Thr Ala Ser Leu Thr Ile Ser Gly Leu Gln Ser Asp Asp Asp
               100                 105                 110
   Ala Asp Tyr Tyr Cys Cys Ser Tyr Ala Gly Ser Tyr Ile Trp Val Phe
           115                 120                 125
   Gly Gly Gly Thr Lys Leu Thr Val Leu Gly Gly Gly Ser Ser Arg Ser
       130                 135                 140
   Ser Ser Ser Gly Gly Gly Gly Ser Gly Gly Gly Gly Glu Val Gln Leu
   145                 150                 155                 160
   Val Gln Ser Gly Pro Gly Leu Val Lys Pro Ser Glu Thr Leu Ser Leu
                   165                 170                 175
   Thr Cys Thr Val Ser Gly Val Ser Val Ser Ser Gly Ser Tyr His Trp
               180                 185                 190
   Ser Trp Ile Arg Gln Thr Pro Gly Lys Gly Leu Glu Trp Ile Gly Tyr
           195                 200                 205
   Ile Tyr Tyr Ile Gly Ser Thr Lys Tyr Asn Pro Ser Leu Lys Ser Arg
       210                 215                 220
   Ala Thr Ile Ser Ile Asn Thr Ser Thr Asn Gln Phe Ser Leu Lys Leu
   225                 230                 235                 240
   Ser Ser Val Thr Ala Ala Asp Thr Ala Val Tyr Tyr Cys Ala Arg Glu
                   245                 250                 255
   Ser Thr Ser Tyr Gly Glu Arg Arg Phe Asp Tyr Trp Gly Gln Gly Thr
   260 265 270
   Arg Val Thr Val Ser Ser Ala Ser Thr Lys Gly Pro Ser Val Thr Ser
   275 280 285
   Gly Gln Ala Gly Arg Asn Ala Val Gly Gln Asp Thr Gln Glu Val Ile
   290 295 300
   Val Val Pro His Ser Leu Pro Phe Lys Val Val Val Ile Ser Ala Ile
   305 310 315 320
   Leu Ala Leu Val Val Leu Thr Ile Ile Ser Leu Ile Ile Leu Ile Met
   325 330 335
   Leu Trp Gln Lys Lys Pro Arg
   340
<210> 22
   <211> 526
   <212> PRT
   <213> artificial sequence
<220>
   <223> chemically synthesized
<400> 22
<210> 23
   <211> 522
   <212> PRT
   <213> artificial sequence
<220>
   <223> chemically synthesized
<210> 24
   <211> 525
   <212> PRT
   <213> artificial sequence
<220>
   <223> chemically synthesized
<400> 24
<210> 25
   <211> 524
   <212> PRT
   <213> artificial sequence
<220>
   <223> chemically synthesized
<210> 26
   <211> 69
   <212> DNA
   <213> artificial sequence
<220>
   <223> chemically synthesized
<210> 27
   <211> 70
   <212> DNA
   <213> artificial sequence
<220>
   <223> chemically synthesized
<400> 27
   cctcctgcgt gtcctggccc acagcattgc ggccggcctg gccgctagcg gtaccggccg 60
   cctgggcctc 70
<210> 28
   <211> 69
   <212> DNA
   <213> artificial sequence
<220>
   <223> chemically synthesized
<400> 28
   ggccaggaca cgcaggaggt catcgtggtg ccacactcct tgccctttaa ggtggtggtg 60
   atctcagcc 69
<210> 29
   <211> 70
   <212> DNA
   <213> artificial sequence
<220>
   <223> chemically synthesized
<400> 29
   catgatgagg atgataaggg agatgatggt gagcaccacc agggccagga tggctgagat 60
   caccaccacc 70
<210> 30
   <211> 54
   <212> DNA
   <213> artificial sequence
<220>
   <223> chemically synthesized
<400> 30
   gagtctagag ccaccatgga gacagacaca ctcctgctat gggtactgct gctc 54
<210> 31
   <211> 55
   <212> DNA
   <213> artificial sequence
<220>
   <223> chemically synthesized
<400> 31
   ctcgggcccc taacgtggct tcttctgcca aagcatgatg aggatgataa gggag 55
<210> 32
   <211> 57
   <212> DNA
   <213> artificial sequence
<220>
   <223> chemically synthesized
<220>
   <221> misc_feature
   <222> (57)..(57)
   <223> n is a, c, g, or t
<400> 32
   aagcagtggt aacaacgcag agtacttttt tttttttttt tttttttttt tttttvn 57
<210> 33
   <211> 30
   <212> DNA
   <213> artificial sequence
<220>
   <223> chemically synthesized
<220>
   <221> misc_RNA
   <222> (28)..(30)
   <223> nucleotides 28 to 30 are ribonucleotides
<400> 33
   aagcagtggt aacaacgcag agtacgcggg 30
<210> 34
   <211> 23
   <212> DNA
   <213> artificial sequence
<220>
   <223> chemically synthesized
<400> 34
   aagcagtggt atcaacgcag agt 23
<210> 35
   <211> 22
   <212> DNA
   <213> artificial sequence
<220>
   <223> chemically synthesized
<400> 35
   acaaattgga ctaatcgatg gc 22
<210> 36
   <211> 20
   <212> DNA
   <213> artificial sequence
<220>
   <223> chemically synthesized
<400> 36
   gagcaaaaga gcattccaag 20
<210> 37
   <211> 10309
   <212> DNA
   <213> artificial sequence
<220>
   <223> chemically synthesized
<400> 37
   ggtaccatgg agacagacac actcctgcta tgggtactgc tgctctgggt tccaggttcc      60
   actggtgacg cggatccggc ccaggcggcc ttaattaaag gtttaaacgg ccaggccggc     120
   cgcaagctta ctcacacatg cccaccgtgc ccagcacctg aagccgaggg ggcaccgtca     180
   gtcttcctct tccccccaaa acccaaggac accctcatga tctcccggac ccctgaggtc      240
   acatgcgtgg tggtggacgt gagccacgaa gaccctgagg tcaagttcaa ctggtacgtg     300
   gacggcgtgg aggtgcataa tgccaagaca aagccgcggg aggagcagta caacagcacg     360
   taccgtgtgg tcagcgtcct caccgtcctg caccaggact ggctgaatgg caaggagtac     420
   aagtgcaagg tctccaacaa agccctccca gcctccatcg agaaaaccat ctccaaagcc     480
   aaagggcagc cccgagaacc acaggtgtac accctgcccc catcccggga tgagctgacc     540
   aagaaccagg tcagcctgac ctgcctggtc aaaggcttct atcccagcga catcgccgtg     600
   gagtgggaga gcaatgggca gccggagaac aactacaaga ccacgcctcc cgtgttggac     660
   tccgacggct ccttcttcct ctacagcaag ctcaccgtgg acaagagcag gtggcagcag     720
   gggaacgtct tctcatgctc cgtgatgcat gaggctctgc acaaccacta cacgcagaag     780
   agcctctccc tgtctccggg taaatgactc gaggcccgaa caaaaactca tctcagaaga     840
   ggatctgaat agcgccgtcg accatcatca tcatcatcat tgagtttaac gatccagaca     900
   tgataagata cattgatgag tttggacaaa ccacaactag aatgcagtga aaaaaatgct     960
   ttatttgtga aatttgtgat gctattgctt tatttgtaac cattataagc tgcaataaac    1020
   aagttaacaa caacaattgc attcatttta tgtttcaggt tcagggggag gtggggaggt  1080
   tttttaaagc aagtaaaacc tctacaaatg tggtatggct gattatgatc cggctgcctc    1140
   gcgcgtttcg gtgatgacgg tgaaaacctc tgacacatgc agctcccgga gacggtcaca    1200
   gcttgtctgt aagcggatgc cgggagcaga caagcccgtc agggcgcgtc agcgggtgtt 1260
   ggcgggtgtc ggggcgcagc catgaggtcg actctagagg atcgatcccc gccgccggac 1320
   gaactaaacc tgactacggc atctctgccc cttcttcgcg gggcagtgca tgtaatccct 1380
   tcagttggtt ggtacaactt gccaactggg ccctgttcca catgtgacac ggggggggac 1440
   caaacacaaa ggggttctct gactgtagtt gacatcctta taaatggatg tgcacatttg 1500
   ccaacactga gtggctttca tcctggagca gactttgcag tctgtggact gcaacacaac 1560
   attgccttta tgtgtaactc ttggctgaag ctcttacacc aatgctgggg gacatgtacc 1620
   tcccaggggc ccaggaagac tacgggaggc tacaccaacg tcaatcagag gggcctgtgt 1680
   agctaccgat aagcggaccc tcaagagggc attagcaata gtgtttataa ggcccccttg 1740
   ttaaccctaa acgggtagca tatgcttccc gggtagtagt atatactatc cagactaacc 1800
   ctaattcaat agcatatgtt acccaacggg aagcatatgc tatcgaatta gggttagtaa 1860
   aagggtccta aggaacagcg atatctccca ccccatgagc tgtcacggtt ttatttacat 1920
   ggggtcagga ttccacgagg gtagtgaacc attttagtca caagggcagt ggctgaagat 1980
   caaggagcgg gcagtgaact ctcctgaatc ttcgcctgct tcttcattct ccttcgttta 2040
   gctaatagaa taactgctga gttgtgaaca gtaaggtgta tgtgaggtgc tcgaaaacaa 2100
   ggtttcaggt gacgccccca gaataaaatt tggacggggg gttcagtggt ggcattgtgc 2160
   tatgacacca atataaccct cacaaacccc ttgggcaata aatactagtg taggaatgaa 2220
   acattctgaa tatctttaac aatagaaatc catggggtgg ggacaagccg taaagactgg 2280
   atgtccatct cacacgaatt tatggctatg ggcaacacat aatcctagtg caatatgata 2340
   ctggggttat taagatgtgt cccaggcagg gaccaagaca ggtgaaccat gttgttacac 2400
   tctatttgta acaaggggaa agagagtgga cgccgacagc agcggactcc actggttgtc 2460
   tctaacaccc ccgaaaatta aacggggctc cacgccaatg gggcccataa acaaagacaa 2520
   gtggccactc ttttttttga aattgtggag tgggggcacg cgtcagcccc cacacgccgc 2580
   cctgcggttt tggactgtaa aataagggtg taataacttg gctgattgta accccgctaa 2640
   ccactgcggt caaaccactt gcccacaaaa ccactaatgg caccccgggg aatacctgca 2700
   taagtaggtg ggcgggccaa gataggggcg cgattgctgc gatctggagg acaaattaca 2760
   cacacttgcg cctgagcgcc aagcacaggg ttgttggtcc tcatattcac gaggtcgctg 2820
   agagcacggt gggctaatgt tgccatgggt agcatatact acccaaatat ctggatagca 2880
   tatgctatcc taatctatat ctgggtagca taggctatcc taatctatat ctgggtagca 2940
   tatgctatcc taatctatat ctgggtagta tatgctatcc taatttatat ctgggtagca 3000
   taggctatcc taatctatat ctgggtagca tatgctatcc taatctatat ctgggtagta 3060
   tatgctatcc taatctgtat ccgggtagca tatgctatcc taatagagat tagggtagta 3120
   tatgctatcc taatttatat ctgggtagca tatactaccc aaatatctgg atagcatatg 3180
   ctatcctaat ctatatctgg gtagcatatg ctatcctaat ctatatctgg gtagcatagg 3240
   ctatcctaat ctatatctgg gtagcatatg ctatcctaat ctatatctgg gtagtatatg 3300
   ctatcctaat ttatatctgg gtagcatagg ctatcctaat ctatatctgg gtagcatatg 3360
   ctatcctaat ctatatctgg gtagtatatg ctatcctaat ctgtatccgg gtagcatatg 3420
   ctatcctcat gcatatacag tcagcatatg atacccagta gtagagtggg agtgctatcc 3480
   tttgcatatg ccgccacctc ccaagggggc gtgaattttc gctgcttgtc cttttcctgc 3540
   atgctggttg ctcccattct taggtgaatt taaggaggcc aggctaaagc cgtcgcatgt 3600
   ctgattgctc accaggtaaa tgtcgctaat gttttccaac gcgagaaggt gttgagcgcg 3660
   gagctgagtg acgtgacaac atgggtatgc ccaattgccc catgttggga ggacgaaaat 3720
   ggtgacaaga cagatggcca gaaatacacc aacagcacgc atgatgtcta ctggggattt 3780
   attctttagt gcgggggaat acacggcttt taatacgatt gagggcgtct cctaacaagt 3840
   tacatcactc ctgcccttcc tcaccctcat ctccatcacc tccttcatct ccgtcatctc 3900
   cgtcatcacc ctccgcggca gccccttcca ccataggtgg aaaccaggga ggcaaatcta 3960
   ctccatcgtc aaagctgcac acagtcaccc tgatattgca ggtaggagcg ggctttgtca 4020
   taacaaggtc cttaatcgca tccttcaaaa cctcagcaaa tatatgagtt tgtaaaaaga 4080
   ccatgaaata acagacaatg gactccctta gcgggccagg ttgtgggccg ggtccagggg 4140
   ccattccaaa ggggagacga ctcaatggtg taagacgaca ttgtggaata gcaagggcag 4200
   ttcctcgcct taggttgtaa agggaggtct tactacctcc atatacgaac acaccggcga 4260
   cccaagttcc ttcgtcggta gtcctttcta cgtgactcct agccaggaga gctcttaaac 4320
   cttctgcaat gttctcaaat ttcgggttgg aacctccttg accacgatgc tttccaaacc 4380
   accctccttt tttgcgcctg cctccatcac cctgaccccg gggtccagtg cttgggcctt 4440
   ctcctgggtc atctgcgggg ccctgctcta tcgctcccgg gggcacgtca ggctcaccat 4500
   ctgggccacc ttcttggtgg tattcaaaat aatcggcttc ccctacaggg tggaaaaatg 4560
   gccttctacc tggagggggc ctgcgcggtg gagacccgga tgatgatgac tgactactgg 4620
   gactcctggg cctcttttct ccacgtccac gacctctccc cctggctctt tcacgacttc 4680
   cccccctggc tctttcacgt cctctacccc ggcggcctcc actacctcct cgaccccggc 4740
   ctccactacc tcctcgaccc cggcctccac tgcctcctcg accccggcct ccacctcctg 4800
   ctcctgcccc tcctgctcct gcccctcctc ctgctcctgc cctcctgccc cctcctgctc 4860
   ctgcccctcc tgcccctcct gctcctgccc ctcctgcccc tcctgctcct gcccctcctg 4920
   cccctcctcc tgctcctgcc cctcctgccc ctcctcctgc tcctgcccct cctgcccctc 4980
   ctgctcctgc ccctcctgcc cctcctgctc ctgcccctcc tgcccctcct gctcctgccc 5040
   ctcctgctcc tgcccctcct gctcctgccc ctcctgctcc tgcccctcct gcccctcctg 5100
   cccctcctcc tgctcctgcc cctcctgctc ctgcccctcc tgcccctcct gcccctcctg 5160
   ctcctgcccc tcctcctgct cctgcccctc ctgcccctcc tgcccctcct cctgctcctg 5220
   cccctcctgc ccctcctcct gctcctgccc ctcctcctgc tcctgcccct cctgcccctc 5280
   ctgcccctcc tcctgctcct gcccctcctg cccctcctcc tgctcctgcc cctcctcctg 5340
   ctcctgcccc tcctgcccct cctgcccctc ctcctgctcc tgcccctcct cctgctcctg 5400
   cccctcctgc ccctcctgcc cctcctgccc ctcctcctgc tcctgcccct cctcctgctc 5460
   ctgcccctcc tgctcctgcc cctcccgctc ctgctcctgc tcctgttcca ccgtgggtcc 5520
   ctttgcagcc aatgcaactt ggacgttttt ggggtctccg gacaccatct ctatgtcttg 5580
   gccctgatcc tgagccgccc ggggctcctg gtcttccgcc tcctcgtcct cgtcctcttc 5640
   cccgtcctcg tccatggtta tcaccccctc ttctttgagg tccactgccg ccggagcctt 5700
   ctggtccaga tgtgtctccc ttctctccta ggccatttcc aggtcctgta cctggcccct 5760
   cgtcagacat gattcacact aaaagagatc aatagacatc tttattagac gacgctcagt 5820
   gaatacaggg agtgcagact cctgccccct ccaacagccc ccccaccctc atccccttca 5880
   tggtcgctgt cagacagatc caggtctgaa aattccccat cctccgaacc atcctcgtcc 5940
   tcatcaccaa ttactcgcag cccggaaaac tcccgctgaa catcctcaag atttgcgtcc 6000
   tgagcctcaa gccaggcctc aaattcctcg tccccctttt tgctggacgg tagggatggg 6060
   gattctcggg acccctcctc ttcctcttca aggtcaccag acagagatgc tactggggca 6120
   acggaagaaa agctgggtgc ggcctgtgag gatcagctta tcgatgataa gctgtcaaac 6180
   atgagaattc ttgaagacga aagggcctcg tgatacgcct atttttatag gttaatgtca 6240
   tgataataat ggtttcttag acgtcaggtg gcacttttcg gggaaatgtg cgcggaaccc 6300
   ctatttgttt atttttctaa atacattcaa atatgtatcc gctcatgaga caataaccct 6360
   gataaatgct tcaataatat tgaaaaagga agagtatgag tattcaacat ttccgtgtcg 6420
   cccttattcc cttttttgcg gcattttgcc ttcctgtttt tgctcaccca gaaacgctgg 6480
   tgaaagtaaa agatgctgaa gatcagttgg gtgcacgagt gggttacatc gaactggatc 6540
   tcaacagcgg taagatcctt gagagttttc gccccgaaga acgttttcca atgatgagca 6600
   cttttaaagt tctgctatgt ggcgcggtat tatcccgtgt tgacgccggg caagagcaac 6660
   tcggtcgccg catacactat tctcagaatg acttggttga gtactcacca gtcacagaaa 6720
   agcatcttac ggatggcatg acagtaagag aattatgcag tgctgccata accatgagtg 6780
   ataacactgc ggccaactta cttctgacaa cgatcggagg accgaaggag ctaaccgctt 6840
   ttttgcacaa catgggggat catgtaactc gccttgatcg ttgggaaccg gagctgaatg 6900
   aagccatacc aaacgacgag cgtgacacca cgatgcctgc agcaatggca acaacgttgc 6960
   gcaaactatt aactggcgaa ctacttactc tagcttcccg gcaacaatta atagactgga 7020
   tggaggcgga taaagttgca ggaccacttc tgcgctcggc ccttccggct ggctggttta 7080
   ttgctgataa atctggagcc ggtgagcgtg ggtctcgcgg tatcattgca gcactggggc 7140
   cagatggtaa gccctcccgt atcgtagtta tctacacgac ggggagtcag gcaactatgg 7200
   atgaacgaaa tagacagatc gctgagatag gtgcctcact gattaagcat tggtaactgt 7260
   cagaccaagt ttactcatat atactttaga ttgatttaaa acttcatttt taatttaaaa 7320
   ggatctaggt gaagatcctt tttgataatc tcatgaccaa aatcccttaa cgtgagtttt 7380
   cgttccactg agcgtcagac cccgtagaaa agatcaaagg atcttcttga gatccttttt 7440
   ttctgcgcgt aatctgctgc ttgcaaacaa aaaaaccacc gctaccagcg gtggtttgtt 7500
   tgccggatca agagctacca actctttttc cgaaggtaac tggcttcagc agagcgcaga 7560
   taccaaatac tgtccttcta gtgtagccgt agttaggcca ccacttcaag aactctgtag 7620
   caccgcctac atacctcgct ctgctaatcc tgttaccagt ggctgctgcc agtggcgata 7680
   agtcgtgtct taccgggttg gactcaagac gatagttacc ggataaggcg cagcggtcgg 7740
   gctgaacggg gggttcgtgc acacagccca gcttggagcg aacgacctac accgaactga 7800
   gatacctaca gcgtgagcta tgagaaagcg ccacgcttcc cgaagggaga aaggcggaca 7860
   ggtatccggt aagcggcagg gtcggaacag gagagcgcac gagggagctt ccagggggaa 7920
   acgcctggta tctttatagt cctgtcgggt ttcgccacct ctgacttgag cgtcgatttt 7980
   tgtgatgctc gtcagggggg cggagcctat ggaaaaacgc cagcaacgcg gcctttttac 8040
   ggttcctggc cttttgctgc gccgcgtgcg gctgctggag atggcggacg cgatggatat 8100
   gttctgccaa gggttggttt gcgcattcac agttctccgc aagaattgat tggctccaat 8160
   tcttggagtg gtgaatccgt tagcgaggcc atccagcctc gcgtcgaact agatgatccg 8220
   ctgtggaatg tgtgtcagtt agggtgtgga aagtccccag gctccccagc aggcagaagt 8280
   atgcaaagca tgcatctcaa ttagtcagca accaggtgtg gaaagtcccc aggctcccca 8340
   gcaggcagaa gtatgcaaag catgcatctc aattagtcag caaccatagt cccgccccta 8400
   actccgccca tcccgcccct aactccgccc agttccgccc attctccgcc ccatggctga 8460
   ctaatttttt ttatttatgc agaggccgag gccgcggcct ctgagctatt ccagaagtag 8520
   tgaggaggct tttttggagg gtgaccgcca cgaggtgccg ccaccatccc ctgacccacg 8580
   cccctgaccc ctcacaagga gacgaccttc catgaccgag tacaagccca cggtgcgcct 8640
   cgccacccgc gacgacgtcc cccgggccgt acgcaccctc gccgccgcgt tcgccgacta 8700
   ccccgccacg cgccacaccg tcgaccccga ccgccacatc gaacgcgtca ccgagctgca 8760
   agaactcttc ctcacgcgcg tcgggctcga catcggcaag gtgtgggtcg cggacgacgg 8820
   cgccgcggtg gcggtctgga ccacgccgga gagcgtcgaa gcgggggcgg tgttcgccga 8880
   gatcggcccg cgcatggccg agttgagcgg ttcccggctg gccgcgcagc aacagatgga 8940
   aggcctcctg gcgccgcacc ggcccaagga gcccgcgtgg ttcctggcca ccgtcggcgt 9000
   ctcgcccgac caccagggca agggtctggg cagcgccgtc gtgctccccg gagtggaggc 9060
   ggccgagcgc gccggggtgc ccgccttcct ggagacctcc gcgccccgca acctcccctt 9120
   ctacgagcgg ctcggcttca ccgtcaccgc cgacgtcgag tgcccgaagg accgcgcgac 9180
   ctggtgcatg acccgcaagc ccggtgcctg acgcccgccc cacgacccgc agcgcccgac 9240
   cgaaaggagc gcacgacccg gtccgacggc ggcccacggg tcccaggggg gtcgacctcg 9300
   aaacttgttt attgcagctt ataatggtta caaataaagc aatagcatca caaatttcac 9360
   aaataaagca tttttttcac tgcattctag ttgtggtttg tccaaactca tcaatgtatc 9420
   ttatcatgtc tggatcgatc cgaacccctt cctcgaccaa ttctcatgtt tgacagctta 9480
   tcatcgcaga tccgggcaac gttgttgcat tgctgcaggc gcagaactgg taggtatgga 9540
   agatctatac attgaatcaa tattggcaat tagccatatt agtcattggt tatatagcat 9600
   aaatcaatat tggctattgg ccattgcata cgttgtatct atatcataat atgtacattt 9660
   atattggctc atgtccaata tgaccgccat gttgacattg attattgact agttattaat 9720
   agtaatcaat tacggggtca ttagttcata gcccatatat ggagttccgc gttacataac 9780
   ttacggtaaa tggcccgcct ggctgaccgc ccaacgaccc ccgcccattg acgtcaataa 9840
   tgacgtatgt tcccatagta acgccaatag ggactttcca ttgacgtcaa tgggtggagt 9900
   atttacggta aactgcccac ttggcagtac atcaagtgta tcatatgcca agtccgcccc 9960
   ctattgacgt caatgacggt aaatggcccg cctggcatta tgcccagtac atgaccttac 10020
   gggactttcc tacttggcag tacatctacg tattagtcat cgctattacc atggtgatgc 10080
   ggttttggca gtacaccaat gggcgtggat agcggtttga ctcacgggga tttccaagtc 10140
   tccaccccat tgacgtcaat gggagtttgt tttggcacca aaatcaacgg gactttccaa 10200
   aatgtcgtaa taaccccgcc ccgttgacgc aaatgggcgg taggcgtgta cggtgggagg 10260
   tctatataag cagagctcgt ttagtgaacc gtcagatctc tagaagctg 10309
<210> 38
   <211> 10215
   <212> DNA
   <213> artificial sequence
<220>
   <223> chemically synthesized
<400> 38
   gagctcgtat ggacatattg tcgttagaac gcggctacaa ttaatacata accttatgta 60
   tcatacacat acgatttagg ggacactata gattgacggc gtagtacaca ctattgaatc 120
   aaacagccga ccaattgcac taccatcaca atggagaagc cagtagtaaa cgtagacgta 180
   gacccccaga gtccgtttgt cgtgcaactg caaaaaagct tcccgcaatt tgaggtagta 240
   gcacagcagg tcactccaaa tgaccatgct aatgccagag cattttcgca tctggccagt 300
   aaactaatcg agctggaggt tcctaccaca gcgacgatct tggacatagg cagcgcaccg 360
   gctcgtagaa tgttttccga gcaccagtat cattgtgtct gccccatgcg tagtccagaa 420
   gacccggacc gcatgatgaa atacgccagt aaactggcgg aaaaagcgtg caagattaca 480
   aacaagaact tgcatgagaa gattaaggat ctccggaccg tacttgatac gccggatgct 540
   gaaacaccat cgctctgctt tcacaacgat gttacctgca acatgcgtgc cgaatattcc 600
   gtcatgcagg acgtgtatat caacgctccc ggaactatct atcatcaggc tatgaaaggc 660
   gtgcggaccc tgtactggat tggcttcgac accacccagt tcatgttctc ggctatggca 720
   ggttcgtacc ctgcgtacaa caccaactgg gccgacgaga aagtccttga agcgcgtaac 780
   atcggacttt gcagcacaaa gctgagtgaa ggtaggacag gaaaattgtc gataatgagg 840
   aagaaggagt tgaagcccgg gtcgcgggtt tatttctccg taggatcgac actttatcca 900
   gaacacagag ccagcttgca gagctggcat cttccatcgg tgttccactt gaatggaaag 960
   cagtcgtaca cttgccgctg tgatacagtg gtgagttgcg aaggctacgt agtgaagaaa 1020
   atcaccatca gtcccgggat cacgggagaa accgtgggat acgcggttac acacaatagc 1080
   gagggcttct tgctatgcaa agttactgac acagtaaaag gagaacgggt atcgttccct 1140
   gtgtgcacgt acatcccggc caccatatgc gatcagatga ctggtataat ggccacggat 1200
   atatcacctg acgatgcaca aaaacttctg gttgggctca accagcgaat tgtcattaac 1260
   ggtaggacta acaggaacac caacaccatg caaaattacc ttctgccgat catagcacaa 1320
   gggttcagca aatgggctaa ggagcgcaag gatgatcttg ataacgagaa aatgctgggt 1380
   actagagaac gcaagcttac gtatggctgc ttgtgggcgt ttcgcactaa gaaagtacat 1440
   tcgttttatc gcccacctgg aacgcagacc tgcgtaaaag tcccagcctc ttttagcgct 1500
   tttcccatgt cgtccgtatg gacgacctct ttgcccatgt cgctgaggca gaaattgaaa 1560
   ctggcattgc aaccaaagaa ggaggaaaaa ctgctgcagg tctcggagga attagtcatg 1620
   gaggccaagg ctgcttttga ggatgctcag gaggaagcca gagcggagaa gctccgagaa 1680
   gcacttccac cattagtggc agacaaaggc atcgaggcag ccgcagaagt tgtctgcgaa 1740
   gtggaggggc tccaggcgga catcggagca gcattagttg aaaccccgcg cggtcacgta 1800
   aggataatac ctcaagcaaa tgaccgtatg atcggacagt atatcgttgt ctcgccaaac 1860
   tctgtgctga agaatgccaa actcgcacca gcgcacccgc tagcagatca ggttaagatc 1920
   ataacacact ccggaagatc aggaaggtac gcggtcgaac catacgacgc taaagtactg 1980
   atgccagcag gaggtgccgt accatggcca gaattcctag cactgagtga gagcgccacg 2040
   ttagtgtaca acgaaagaga gtttgtgaac cgcaaactat accacattgc catgcatggc 2100
   cccgccaaga atacagaaga ggagcagtac aaggttacaa aggcagagct tgcagaaaca 2160
   gagtacgtgt ttgacgtgga caagaagcgt tgcgttaaga aggaagaagc ctcaggtctg 2220
   gtcctctcgg gagaactgac caaccctccc tatcatgagc tagctctgga gggactgaag 2280
   acccgacctg cggtcccgta caaggtcgaa acaataggag tgataggcac accggggtcg 2340
   ggcaagtcag ctattatcaa gtcaactgtc acggcacgag atcttgttac cagcggaaag 2400
   aaagaaaatt gtcgcgaaat tgaggccgac gtgctaagac tgaggggtat gcagattacg 2460
   tcgaagacag tagattcggt tatgctcaac ggatgccaca aagccgtaga agtgctgtac 2520
   gttgacgaag cgttcgcgtg ccacgcagga gcactacttg ccttgattgc tatcgtcagg 2580
   ccccgcaaga aggtagtact atgcggagac cccatgcaat gcggattctt caacatgatg 2640
   caactaaagg tacatttcaa tcaccctgaa aaagacatat gcaccaagac attctacaag 2700
   tatatctccc ggcgttgcac acagccagtt acagctattg tatcgacact gcattacgat 2760
   ggaaagatga aaaccacgaa cccgtgcaag aagaacattg aaatcgatat tacaggggcc 2820
   acaaagccga agccagggga tatcatcctg acatgtttcc gcgggtgggt taagcaattg 2880
   caaatcgact atcccggaca tgaagtaatg acagccgcgg cctcacaagg gctaaccaga 2940
   aaaggagtgt atgccgtccg gcaaaaagtc aatgaaaacc cactgtacgc gatcacatca 3000
   gagcatgtga acgtgttgct cacccgcact gaggacaggc tagtgtggaa aaccttgcag 3060
   ggcgacccat ggattaagca gcccactaac atacctaaag gaaactttca ggctactata 3120
   gaggactggg aagctgaaca caagggaata attgctgcaa taaacagccc cactccccgt 3180
   gccaatccgt tcagctgcaa gaccaacgtt tgctgggcga aagcattgga accgatacta 3240
   gccacggccg gtatcgtact taccggttgc cagtggagcg aactgttccc acagtttgcg 3300
   gatgacaaac cacattcggc catttacgcc ttagacgtaa tttgcattaa gtttttcggc 3360
   atggacttga caagcggact gttttctaaa cagagcatcc cactaacgta ccatcccgcc 3420
   gattcagcga ggccggtagc tcattgggac aacagcccag gaacccgcaa gtatgggtac 3480
   gatcacgcca ttgccgccga actctcccgt agatttccgg tgttccagct agctgggaag 3540
   ggcacacaac ttgatttgca gacggggaga accagagtta tctctgcaca gcataacctg 3600
   gtcccggtga accgcaatct tcctcacgcc ttagtccccg agtacaagga gaagcaaccc 3660
   ggcccggtca aaaaattctt gaaccagttc aaacaccact cagtacttgt ggtatcagag 3720
   gaaaaaattg aagctccccg taagagaatc gaatggatcg ccccgattgg catagccggt 3780
   gcagataaga actacaacct ggctttcggg tttccgccgc aggcacggta cgacctggtg 3840
   ttcatcaaca ttggaactaa atacagaaac caccactttc agcagtgcga agaccatgcg 3900
   gcgaccttaa aaaccctttc gcgttcggcc ctgaattgcc ttaacccagg aggcaccctc 3960
   gtggtgaagt cctatggcta cgccgaccgc aacagtgagg acgtagtcac cgctcttgcc 4020
   agaaagtttg tcagggtgtc tgcagcgaga ccagattgtg tctcaagcaa tacagaaatg 4080
   tacctgattt tccgacaact agacaacagc cgtacacggc aattcacccc gcaccatctg 4140
   aattgcgtga tttcgtccgt gtatgagggt acaagagatg gagttggagc cgcgccgtca 4200
   taccgcacca aaagggagaa tattgctgac tgtcaagagg aagcagttgt caacgcagcc 4260
   aatccgctgg gtagaccagg cgaaggagtc tgccgtgcca tctataaacg ttggccgacc 4320
   agttttaccg attcagccac ggagacaggc accgcaagaa tgactgtgtg cctaggaaag 4380
   aaagtgatcc acgcggtcgg ccctgatttc cggaagcacc cagaagcaga agccttgaaa 4440
   ttgctacaaa acgcctacca tgcagtggca gacttagtaa atgaacataa catcaagtct 4500
   gtcgccattc cactgctatc tacaggcatt tacgcagccg gaaaagaccg ccttgaagta 4560
   tcacttaact gcttgacaac cgcgctagac agaactgacg cggacgtaac catctattgc 4620
   ctggataaga agtggaagga aagaatcgac gcggcactcc aacttaagga gtctgtaaca 4680
   gagctgaagg atgaagatat ggagatcgac gatgagttag tatggattca tccagacagt 4740
   tgcttgaagg gaagaaaggg attcagtact acaaaaggaa aattgtattc gtacttcgaa 4800
   ggcaccaaat tccatcaagc agcaaaagac atggcggaga taaaggtcct gttccctaat 4860
   gaccaggaaa gtaatgaaca actgtgtgcc tacatattgg gtgagaccat ggaagcaatc 4920
   cgcgaaaagt gcccggtcga ccataacccg tcgtctagcc cgcccaaaac gttgccgtgc 4980
   ctttgcatgt atgccatgac gccagaaagg gtccacagac ttagaagcaa taacgtcaaa 5040
   gaagttacag tatgctcctc cacccccctt cctaagcaca aaattaagaa tgttcagaag 5100
   gttcagtgca cgaaagtagt cctgtttaat ccgcacactc ccgcattcgt tcccgcccgt 5160
   aagtacatag aagtgccaga acagcctacc gctcctcctg cacaggctga ggaagccccc 5220
   gaagttgtag cgacaccgtc accatctaca gctgataaca cctcgcttga tgtcacagac 5280
   atctcactgg atatggatga cagtagcgaa ggctcacttt tttcgagctt tagcggatcg 5340
   gacaactcta ttactagtat ggacagttgg tcgtcaggac ctagttcact agagatagta 5400
   gaccgaaggc aggtggtggt ggctgacgtt catgccgtcc aagagcctgc ccctattcca 5460
   ccgccaaggc taaagaagat ggcccgcctg gcagcggcaa gaaaagagcc cactccaccg 5520
   gcaagcaata gctctgagtc cctccacctc tcttttggtg gggtatccat gtccctcgga 5580
   tcaattttcg acggagagac ggcccgccag gcagcggtac aacccctggc aacaggcccc 5640
   acggatgtgc ctatgtcttt cggatcgttt tccgacggag agattgatga gctgagccgc 5700
   agagtaactg agtccgaacc cgtcctgttt ggatcatttg aaccgggcga agtgaactca 5760
   attatatcgt cccgatcagc cgtatctttt ccactacgca agcagagacg tagacgcagg 5820
   agcaggagga ctgaatactg actaaccggg gtaggtgggt acatattttc gacggacaca 5880
   ggccctgggc acttgcaaaa gaagtccgtt ctgcagaacc agcttacaga accgaccttg 5940
   gagcgcaatg tcctggaaag aattcatgcc ccggtgctcg acacgtcgaa agaggaacaa 6000
   ctcaaactca ggtaccagat gatgcccacc gaagccaaca aaagtaggta ccagtctcgt 6060
   aaagtagaaa atcagaaagc cataaccact gagcgactac tgtcaggact acgactgtat 6120
   aactctgcca cagatcagcc agaatgctat aagatcacct atccgaaacc attgtactcc 6180
   agtagcgtac cggcgaacta ctccgatcca cagttcgctg tagctgtctg taacaactat 6240
   ctgcatgaga actatccgac agtagcatct tatcagatta ctgacgagta cgatgcttac 6300
   ttggatatgg tagacgggac agtcgcctgc ctggatactg caaccttctg ccccgctaag 6360
   cttagaagtt acccgaaaaa acatgagtat agagccccga atatccgcag tgcggttcca 6420
   tcagcgatgc agaacacgct acaaaatgtg ctcattgccg caactaaaag aaattgcaac 6480
   gtcacgcaga tgcgtgaact gccaacactg gactcagcga cattcaatgt cgaatgcttt 6540
   cgaaaatatg catgtaatga cgagtattgg gaggagttcg ctcggaagcc aattaggatt 6600
   accactgagt ttgtcaccgc atatgtagct agactgaaag gccctaaggc cgccgcacta 6660
   tttgcaaaga cgtataattt ggtcccattg caagaagtgc ctatggatag attcgtcatg 6720
   gacatgaaaa gagacgtgaa agttacacca ggcacgaaac acacagaaga aagaccgaaa 6780
   gtacaagtga tacaagccgc agaacccctg gcgactgctt acttatgcgg gattcaccgg 6840
   gaattagtgc gtaggcttac ggccgtcttg cttccaaaca ttcacacgct ttttgacatg 6900
   tcggcggagg attttgatgc aatcatagca gaacacttca agcaaggcga cccggtactg 6960
   gagacggata tcgcatcatt cgacaaaagc caagacgacg ctatggcgtt aaccggtctg 7020
   atgatcttgg aggacctggg tgtggatcaa ccactactcg acttgatcga gtgcgccttt 7080
   ggagaaatat catccaccca tctacctacg ggtactcgtt ttaaattcgg ggcgatgatg 7140
   aaatccggaa tgttcctcac actttttgtc aacacagttt tgaatgtcgt tatcgccagc 7200
   agagtactag aagagcggct taaaacgtcc agatgtgcag cgttcattgg cgacgacaac 7260
   atcatacatg gagtagtatc tgacaaagaa atggctgaga ggtgcgccac ctggctcaac 7320
   atggaggtta agatcatcga cgcagtcatc ggtgagagac caccttactt ctgcggcgga 7380
   tttatcttgc aagattcggt tacttccaca gcgtgccgcg tggcggatcc cctgaaaagg 7440
   ctgtttaagt tgggtaaacc gctcccagcc gacgacgagc aagacgaaga cagaagacgc 7500
   gctctgctag atgaaacaaa ggcgtggttt agagtaggta taacaggcac tttagcagtg 7560
   gccgtgacga cccggtatga ggtagacaat attacacctg tcctactggc attgagaact 7620
   tttgcccaga gcaaaagagc attccaagcc atcagagggg aaataaagca tctctacggt 7680
   ggtcctaaat agtcagcata gtacatttca tctgactaat actacaacac caccacctct 7740
   agagccacca tggagacaga cacactcctg ctatgggtac tgctgctctg ggttccaggt 7800
   tccactggtg actatgaggc ccaggcggcc ggtaccgcta gcggccaggc cggccgcaat 7860
   gctgtgggcc aggacacgca ggaggtcatc gtggtgccac actccttgcc ctttaaggtg 7920
   gtggtgatct cagccatcct ggccctggtg gtgctcacca tcatctccct tatcatcctc 7980
   atcatgcttt ggcagaagaa gccacgttag gggcccgcca tcgattagtc caatttgttg 8040
   gcccaatgat ccgaccagca aaactcgatg tacttccgag gaactgatgt gcataatgca 8100
   tcaggctggt acattagatc cccgcttacc gcgggcaata tagcaacact aaaaactcga 8160
   tgtacttccg aggaagcgca gtgcataatg ctgcgcagtg ttgccacata accactatat 8220
   taaccattta tctagcggac gccaaaaact caatgtattt ctgaggaagc gtggtgcata 8280
   atgccacgca gcgtctgcat aacttttatt atttctttta ttaatcaaca aaattttgtt 8340
   tttaacattt caaaaaaaaa aaaaaaaaaa aaaaaaaaaa aaaaaaaagg gaattcctcg 8400
   attaattaag cggccgctcg aggggaatta attcttgaag acgaaagggc caggtggcac 8460
   ttttcgggga aatgtgcgcg gaacccctat ttgtttattt ttctaaatac attcaaatat 8520
   gtatccgctc atgagacaat aaccctgata aatgcttcaa taatattgaa aaaggaagag 8580
   tatgagtatt caacatttcc gtgtcgccct tattcccttt tttgcggcat tttgccttcc 8640
   tgtttttgct cacccagaaa cgctggtgaa agtaaaagat gctgaagatc agttgggtgc 8700
   acgagtgggt tacatcgaac tggatctcaa cagcggtaag atccttgaga gttttcgccc 8760
   cgaagaacgt tttccaatga tgagcacttt taaagttctg ctatgtggcg cggtattatc 8820
   ccgtgttgac gccgggcaag agcaactcgg tcgccgcata cactattctc agaatgactt 8880
   ggttgagtac tcaccagtca cagaaaagca tcttacggat ggcatgacag taagagaatt 8940
   atgcagtgct gccataacca tgagtgataa cactgcggcc aacttacttc tgacaacgat 9000
   cggaggaccg aaggagctaa ccgctttttt gcacaacatg ggggatcatg taactcgcct 9060
   tgatcgttgg gaaccggagc tgaatgaagc cataccaaac gacgagcgtg acaccacgat 9120
   gcctgtagca atggcaacaa cgttgcgcaa actattaact ggcgaactac ttactctagc 9180
   ttcccggcaa caattaatag actggatgga ggcggataaa gttgcaggac cacttctgcg 9240
   ctcggccctt ccggctggct ggtttattgc tgataaatct ggagccggtg agcgtgggtc 9300
   tcgcggtatc attgcagcac tggggccaga tggtaagccc tcccgtatcg tagttatcta 9360
   cacgacgggg agtcaggcaa ctatggatga acgaaataga cagatcgctg agataggtgc 9420
   ctcactgatt aagcattggt aactgtcaga ccaagtttac tcatatatac tttagattga 9480
   tttaaaactt catttttaat ttaaaaggat ctaggtgaag atcctttttg ataatctcat 9540
   gaccaaaatc ccttaacgtg agttttcgtt ccactgagcg tcagaccccg tagaaaagat 9600
   caaaggatct tcttgagatc ctttttttct gcgcgtaatc tgctgcttgc aaacaaaaaa 9660
   accaccgcta ccagcggtgg tttgtttgcc ggatcaagag ctaccaactc tttttccgaa 9720
   ggtaactggc ttcagcagag cgcagatacc aaatactgtc cttctagtgt agccgtagtt 9780
   aggccaccac ttcaagaact ctgtagcacc gcctacatac ctcgctctgc taatcctgtt 9840
   accagtggct gctgccagtg gcgataagtc gtgtcttacc gggttggact caagacgata 9900
   gttaccggat aaggcgcagc ggtcgggctg aacggggggt tcgtgcacac agcccagctt 9960
   ggagcgaacg acctacaccg aactgagata cctacagcgt gagcattgag aaagcgccac 10020
   gcttcccgaa gggagaaagg cggacaggta tccggtaagc ggcagggtcg gaacaggaga 10080
   gcgcacgagg gagcttccag ggggaaacgc ctggtatctt tatagtcctg tcgggtttcg 10140
   ccacctctga cttgagcgtc gatttttgtg atgctcgtca ggggggcgga gcctatggaa 10200
   aaacgccagc aacgc 10215
<210> 39
   <211> 10245
   <212> DNA
   <213> artificial sequence
<220>
   <223> chemically synthesized
<400> 39
   gagctcgtat ggacatattg tcgttagaac gcggctacaa ttaatacata accttatgta 60
   tcatacacat acgatttagg ggacactata gattgacggc gtagtacaca ctattgaatc 120
   aaacagccga ccaattgcac taccatcaca atggagaagc cagtagtaaa cgtagacgta 180
   gacccccaga gtccgtttgt cgtgcaactg caaaaaagct tcccgcaatt tgaggtagta    240
   gcacagcagg tcactccaaa tgaccatgct aatgccagag cattttcgca tctggccagt    300
   aaactaatcg agctggaggt tcctaccaca gcgacgatct tggacatagg cagcgcaccg    360
   gctcgtagaa tgttttccga gcaccagtat cattgtgtct gccccatgcg tagtccagaa    420
   gacccggacc gcatgatgaa atacgccagt aaactggcgg aaaaagcgtg caagattaca    480
   aacaagaact tgcatgagaa gattaaggat ctccggaccg tacttgatac gccggatgct    540
   gaaacaccat cgctctgctt tcacaacgat gttacctgca acatgcgtgc cgaatattcc    600
   gtcatgcagg acgtgtatat caacgctccc ggaactatct atcatcaggc tatgaaaggc    660
   gtgcggaccc tgtactggat tggcttcgac accacccagt tcatgttctc ggctatggca    720
   ggttcgtacc ctgcgtacaa caccaactgg gccgacgaga aagtccttga agcgcgtaac    780
   atcggacttt gcagcacaaa gctgagtgaa ggtaggacag gaaaattgtc gataatgagg    840
   aagaaggagt tgaagcccgg gtcgcgggtt tatttctccg taggatcgac actttatcca    900
   gaacacagag ccagcttgca gagctggcat cttccatcgg tgttccactt gaatggaaag    960
   cagtcgtaca cttgccgctg tgatacagtg gtgagttgcg aaggctacgt agtgaagaaa   1020
   atcaccatca gtcccgggat cacgggagaa accgtgggat acgcggttac acacaatagc   1080
   gagggcttct tgctatgcaa agttactgac acagtaaaag gagaacgggt atcgttccct   1140
   gtgtgcacgt acatcccggc caccatatgc gatcagatga ctggtataat ggccacggat   1200
   atatcacctg acgatgcaca aaaacttctg gttgggctca accagcgaat tgtcattaac   1260
   ggtaggacta acaggaacac caacaccatg caaaattacc ttctgccgat catagcacaa   1320
   gggttcagca aatgggctaa ggagcgcaag gatgatcttg ataacgagaa aatgctgggt   1380
   actagagaac gcaagcttac gtatggctgc ttgtgggcgt ttcgcactaa gaaagtacat   1440
   tcgttttatc gcccacctgg aacgcagacc tgcgtaaaag tcccagcctc ttttagcgct   1500
   tttcccatgt cgtccgtatg gacgacctct ttgcccatgt cgctgaggca gaaattgaaa   1560
   ctggcattgc aaccaaagaa ggaggaaaaa ctgctgcagg tctcggagga attagtcatg   1620
   gaggccaagg ctgcttttga ggatgctcag gaggaagcca gagcggagaa gctccgagaa   1680
   gcacttccac cattagtggc agacaaaggc atcgaggcag ccgcagaagt tgtctgcgaa   1740
   gtggaggggc tccaggcgga catcggagca gcattagttg aaaccccgcg cggtcacgta   1800
   aggataatac ctcaagcaaa tgaccgtatg atcggacagt atatcgttgt ctcgccaaac   1860
   tctgtgctga agaatgccaa actcgcacca gcgcacccgc tagcagatca ggttaagatc   1920
   ataacacact ccggaagatc aggaaggtac gcggtcgaac catacgacgc taaagtactg   1980
   atgccagcag gaggtgccgt accatggcca gaattcctag cactgagtga gagcgccacg 2040
   ttagtgtaca acgaaagaga gtttgtgaac cgcaaactat accacattgc catgcatggc 2100
   cccgccaaga atacagaaga ggagcagtac aaggttacaa aggcagagct tgcagaaaca 2160
   gagtacgtgt ttgacgtgga caagaagcgt tgcgttaaga aggaagaagc ctcaggtctg 2220
   gtcctctcgg gagaactgac caaccctccc tatcatgagc tagctctgga gggactgaag 2280
   acccgacctg cggtcccgta caaggtcgaa acaataggag tgataggcac accggggtcg 2340
   ggcaagtcag ctattatcaa gtcaactgtc acggcacgag atcttgttac cagcggaaag 2400
   aaagaaaatt gtcgcgaaat tgaggccgac gtgctaagac tgaggggtat gcagattacg 2460
   tcgaagacag tagattcggt tatgctcaac ggatgccaca aagccgtaga agtgctgtac 2520
   gttgacgaag cgttcgcgtg ccacgcagga gcactacttg ccttgattgc tatcgtcagg 2580
   ccccgcaaga aggtagtact atgcggagac cccatgcaat gcggattctt caacatgatg 2640
   caactaaagg tacatttcaa tcaccctgaa aaagacatat gcaccaagac attctacaag 2700
   tatatctccc ggcgttgcac acagccagtt acagctattg tatcgacact gcattacgat 2760
   ggaaagatga aaaccacgaa cccgtgcaag aagaacattg aaatcgatat tacaggggcc 2820
   acaaagccga agccagggga tatcatcctg acatgtttcc gcgggtgggt taagcaattg 2880
   caaatcgact atcccggaca tgaagtaatg acagccgcgg cctcacaagg gctaaccaga 2940
   aaaggagtgt atgccgtccg gcaaaaagtc aatgaaaacc cactgtacgc gatcacatca 3000
   gagcatgtga acgtgttgct cacccgcact gaggacaggc tagtgtggaa aaccttgcag 3060
   ggcgacccat ggattaagca gcccactaac atacctaaag gaaactttca ggctactata 3120
   gaggactggg aagctgaaca caagggaata attgctgcaa taaacagccc cactccccgt 3180
   gccaatccgt tcagctgcaa gaccaacgtt tgctgggcga aagcattgga accgatacta 3240
   gccacggccg gtatcgtact taccggttgc cagtggagcg aactgttccc acagtttgcg 3300
   gatgacaaac cacattcggc catttacgcc ttagacgtaa tttgcattaa gtttttcggc 3360
   atggacttga caagcggact gttttctaaa cagagcatcc cactaacgta ccatcccgcc 3420
   gattcagcga ggccggtagc tcattgggac aacagcccag gaacccgcaa gtatgggtac 3480
   gatcacgcca ttgccgccga actctcccgt agatttccgg tgttccagct agctgggaag 3540
   ggcacacaac ttgatttgca gacggggaga accagagtta tctctgcaca gcataacctg 3600
   gtcccggtga accgcaatct tcctcacgcc ttagtccccg agtacaagga gaagcaaccc 3660
   ggcccggtca aaaaattctt gaaccagttc aaacaccact cagtacttgt ggtatcagag 3720
   gaaaaaattg aagctccccg taagagaatc gaatggatcg ccccgattgg catagccggt 3780
   gcagataaga actacaacct ggctttcggg tttccgccgc aggcacggta cgacctggtg 3840
   ttcatcaaca ttggaactaa atacagaaac caccactttc agcagtgcga agaccatgcg 3900
   gcgaccttaa aaaccctttc gcgttcggcc ctgaattgcc ttaacccagg aggcaccctc 3960
   gtggtgaagt cctatggcta cgccgaccgc aacagtgagg acgtagtcac cgctcttgcc 4020
   agaaagtttg tcagggtgtc tgcagcgaga ccagattgtg tctcaagcaa tacagaaatg 4080
   tacctgattt tccgacaact agacaacagc cgtacacggc aattcacccc gcaccatctg 4140
   aattgcgtga tttcgtccgt gtatgagggt acaagagatg gagttggagc cgcgccgtca 4200
   taccgcacca aaagggagaa tattgctgac tgtcaagagg aagcagttgt caacgcagcc 4260
   aatccgctgg gtagaccagg cgaaggagtc tgccgtgcca tctataaacg ttggccgacc 4320
   agttttaccg attcagccac ggagacaggc accgcaagaa tgactgtgtg cctaggaaag 4380
   aaagtgatcc acgcggtcgg ccctgatttc cggaagcacc cagaagcaga agccttgaaa 4440
   ttgctacaaa acgcctacca tgcagtggca gacttagtaa atgaacataa catcaagtct 4500
   gtcgccattc cactgctatc tacaggcatt tacgcagccg gaaaagaccg ccttgaagta 4560
   tcacttaact gcttgacaac cgcgctagac agaactgacg cggacgtaac catctattgc 4620
   ctggataaga agtggaagga aagaatcgac gcggcactcc aacttaagga gtctgtaaca 4680
   gagctgaagg atgaagatat ggagatcgac gatgagttag tatggattca tccagacagt 4740
   tgcttgaagg gaagaaaggg attcagtact acaaaaggaa aattgtattc gtacttcgaa 4800
   ggcaccaaat tccatcaagc agcaaaagac atggcggaga taaaggtcct gttccctaat 4860
   gaccaggaaa gtaatgaaca actgtgtgcc tacatattgg gtgagaccat ggaagcaatc 4920
   cgcgaaaagt gcccggtcga ccataacccg tcgtctagcc cgcccaaaac gttgccgtgc 4980
   ctttgcatgt atgccatgac gccagaaagg gtccacagac ttagaagcaa taacgtcaaa 5040
   gaagttacag tatgctcctc cacccccctt cctaagcaca aaattaagaa tgttcagaag 5100
   gttcagtgca cgaaagtagt cctgtttaat ccgcacactc ccgcattcgt tcccgcccgt 5160
   aagtacatag aagtgccaga acagcctacc gctcctcctg cacaggctga ggaagccccc 5220
   gaagttgtag cgacaccgtc accatctaca gctgataaca cctcgcttga tgtcacagac 5280
   atctcactgg atatggatga cagtagcgaa ggctcacttt tttcgagctt tagcggatcg 5340
   gacaactcta ttactagtat ggacagttgg tcgtcaggac ctagttcact agagatagta 5400
   gaccgaaggc aggtggtggt ggctgacgtt catgccgtcc aagagcctgc ccctattcca 5460
   ccgccaaggc taaagaagat ggcccgcctg gcagcggcaa gaaaagagcc cactccaccg 5520
   gcaagcaata gctctgagtc cctccacctc tcttttggtg gggtatccat gtccctcgga 5580
   tcaattttcg acggagagac ggcccgccag gcagcggtac aacccctggc aacaggcccc 5640
   acggatgtgc ctatgtcttt cggatcgttt tccgacggag agattgatga gctgagccgc 5700
   agagtaactg agtccgaacc cgtcctgttt ggatcatttg aaccgggcga agtgaactca 5760
   attatatcgt cccgatcagc cgtatctttt ccactacgca agcagagacg tagacgcagg 5820
   agcaggagga ctgaatactg actaaccggg gtaggtgggt acatattttc gacggacaca 5880
   ggccctgggc acttgcaaaa gaagtccgtt ctgcagaacc agcttacaga accgaccttg 5940
   gagcgcaatg tcctggaaag aattcatgcc ccggtgctcg acacgtcgaa agaggaacaa 6000
   ctcaaactca ggtaccagat gatgcccacc gaagccaaca aaagtaggta ccagtctcgt 6060
   aaagtagaaa atcagaaagc cataaccact gagcgactac tgtcaggact acgactgtat 6120
   aactctgcca cagatcagcc agaatgctat aagatcacct atccgaaacc attgtactcc 6180
   agtagcgtac cggcgaacta ctccgatcca cagttcgctg tagctgtctg taacaactat 6240
   ctgcatgaga actatccgac agtagcatct tatcagatta ctgacgagta cgatgcttac 6300
   ttggatatgg tagacgggac agtcgcctgc ctggatactg caaccttctg ccccgctaag 6360
   cttagaagtt acccgaaaaa acatgagtat agagccccga atatccgcag tgcggttcca 6420
   tcagcgatgc agaacacgct acaaaatgtg ctcattgccg caactaaaag aaattgcaac 6480
   gtcacgcaga tgcgtgaact gccaacactg gactcagcga cattcaatgt cgaatgcttt 6540
   cgaaaatatg catgtaatga cgagtattgg gaggagttcg ctcggaagcc aattaggatt 6600
   accactgagt ttgtcaccgc atatgtagct agactgaaag gccctaaggc cgccgcacta 6660
   tttgcaaaga cgtataattt ggtcccattg caagaagtgc ctatggatag attcgtcatg 6720
   gacatgaaaa gagacgtgaa agttacacca ggcacgaaac acacagaaga aagaccgaaa 6780
   gtacaagtga tacaagccgc agaacccctg gcgactgctt acttatgcgg gattcaccgg 6840
   gaattagtgc gtaggcttac ggccgtcttg cttccaaaca ttcacacgct ttttgacatg 6900
   tcggcggagg attttgatgc aatcatagca gaacacttca agcaaggcga cccggtactg 6960
   gagacggata tcgcatcatt cgacaaaagc caagacgacg ctatggcgtt aaccggtctg 7020
   atgatcttgg aggacctggg tgtggatcaa ccactactcg acttgatcga gtgcgccttt 7080
   ggagaaatat catccaccca tctacctacg ggtactcgtt ttaaattcgg ggcgatgatg 7140
   aaatccggaa tgttcctcac actttttgtc aacacagttt tgaatgtcgt tatcgccagc 7200
   agagtactag aagagcggct taaaacgtcc agatgtgcag cgttcattgg cgacgacaac 7260
   atcatacatg gagtagtatc tgacaaagaa atggctgaga ggtgcgccac ctggctcaac 7320
   atggaggtta agatcatcga cgcagtcatc ggtgagagac caccttactt ctgcggcgga 7380
   tttatcttgc aagattcggt tacttccaca gcgtgccgcg tggcggatcc cctgaaaagg 7440
   ctgtttaagt tgggtaaacc gctcccagcc gacgacgagc aagacgaaga cagaagacgc 7500
   gctctgctag atgaaacaaa ggcgtggttt agagtaggta taacaggcac tttagcagtg 7560
   gccgtgacga cccggtatga ggtagacaat attacacctg tcctactggc attgagaact 7620
   tttgcccaga gcaaaagagc attccaagcc atcagagggg aaataaagca tctctacggt 7680
   ggtcctaaat agtcagcata gtacatttca tctgactaat actacaacac caccacctct 7740
   agagccacca tggagacaga cacactcctg ctatgggtac tgctgctctg ggttccaggt 7800
   tccactggtg actatgaggc ccaggcggcc ggtaccgcta gcggccaggc cggccgctat 7860
   ccttacgacg tgccagatta tgcctctaat gctgtgggcc aggacacgca ggaggtcatc 7920
   gtggtgccac actccttgcc ctttaaggtg gtggtgatct cagccatcct ggccctggtg 7980
   gtgctcacca tcatctccct tatcatcctc atcatgcttt ggcagaagaa gccacgttag 8040
   gggcccgcca tcgattagtc caatttgttg gcccaatgat ccgaccagca aaactcgatg 8100
   tacttccgag gaactgatgt gcataatgca tcaggctggt acattagatc cccgcttacc 8160
   gcgggcaata tagcaacact aaaaactcga tgtacttccg aggaagcgca gtgcataatg 8220
   ctgcgcagtg ttgccacata accactatat taaccattta tctagcggac gccaaaaact 8280
   caatgtattt ctgaggaagc gtggtgcata atgccacgca gcgtctgcat aacttttatt 8340
   atttctttta ttaatcaaca aaattttgtt tttaacattt caaaaaaaaa aaaaaaaaaa 8400
   aaaaaaaaaa aaaaaaaagg gaattcctcg attaattaag cggccgctcg aggggaatta 8460
   attcttgaag acgaaagggc caggtggcac ttttcgggga aatgtgcgcg gaacccctat 8520
   ttgtttattt ttctaaatac attcaaatat gtatccgctc atgagacaat aaccctgata 8580
   aatgcttcaa taatattgaa aaaggaagag tatgagtatt caacatttcc gtgtcgccct 8640
   tattcccttt tttgcggcat tttgccttcc tgtttttgct cacccagaaa cgctggtgaa 8700
   agtaaaagat gctgaagatc agttgggtgc acgagtgggt tacatcgaac tggatctcaa 8760
   cagcggtaag atccttgaga gttttcgccc cgaagaacgt tttccaatga tgagcacttt 8820
   taaagttctg ctatgtggcg cggtattatc ccgtgttgac gccgggcaag agcaactcgg 8880
   tcgccgcata cactattctc agaatgactt ggttgagtac tcaccagtca cagaaaagca 8940
   tcttacggat ggcatgacag taagagaatt atgcagtgct gccataacca tgagtgataa 9000
   cactgcggcc aacttacttc tgacaacgat cggaggaccg aaggagctaa ccgctttttt 9060
   gcacaacatg ggggatcatg taactcgcct tgatcgttgg gaaccggagc tgaatgaagc 9120
   cataccaaac gacgagcgtg acaccacgat gcctgtagca atggcaacaa cgttgcgcaa 9180
   actattaact ggcgaactac ttactctagc ttcccggcaa caattaatag actggatgga 9240
   ggcggataaa gttgcaggac cacttctgcg ctcggccctt ccggctggct ggtttattgc 9300
   tgataaatct ggagccggtg agcgtgggtc tcgcggtatc attgcagcac tggggccaga 9360
   tggtaagccc tcccgtatcg tagttatcta cacgacgggg agtcaggcaa ctatggatga 9420
   acgaaataga cagatcgctg agataggtgc ctcactgatt aagcattggt aactgtcaga 9480
   ccaagtttac tcatatatac tttagattga tttaaaactt catttttaat ttaaaaggat 9540
   ctaggtgaag atcctttttg ataatctcat gaccaaaatc ccttaacgtg agttttcgtt 9600
   ccactgagcg tcagaccccg tagaaaagat caaaggatct tcttgagatc ctttttttct 9660
   gcgcgtaatc tgctgcttgc aaacaaaaaa accaccgcta ccagcggtgg tttgtttgcc 9720
   ggatcaagag ctaccaactc tttttccgaa ggtaactggc ttcagcagag cgcagatacc 9780
   aaatactgtc cttctagtgt agccgtagtt aggccaccac ttcaagaact ctgtagcacc 9840
   gcctacatac ctcgctctgc taatcctgtt accagtggct gctgccagtg gcgataagtc 9900
   gtgtcttacc gggttggact caagacgata gttaccggat aaggcgcagc ggtcgggctg 9960
   aacggggggt tcgtgcacac agcccagctt ggagcgaacg acctacaccg aactgagata 10020
   cctacagcgt gagcattgag aaagcgccac gcttcccgaa gggagaaagg cggacaggta 10080
   tccggtaagc ggcagggtcg gaacaggaga gcgcacgagg gagcttccag ggggaaacgc 10140
   ctggtatctt tatagtcctg tcgggtttcg ccacctctga cttgagcgtc gatttttgtg 10200
   atgctcgtca ggggggcgga gcctatggaa aaacgccagc aacgc 10245
<210> 40
   <211> 315
   <212> DNA
   <213> artificial sequence
<220>
   <223> chemically synthesized
<400> 40
   gagtctagag ccaccatgga gacagacaca ctcctgctat gggtactgct gctctgggtt 60
   ccaggttcca ctggtgacta tgaggcccag gcggccggta ccgctagcgg ccaggccggc 120
   cgctatcctt acgacgtgcc agattatgcc tctaatgctg tgggccagga cacgcaggag 180
   gtcatcgtgg tgccacactc cttgcccttt aaggtggtgg tgatctcagc catcctggcc 240
   ctggtggtgc tcaccatcat ctcccttatc atcctcatca tgctttggca gaagaagcca 300
   cgttaggggc ccgag 315
<210> 41
   <211> 100
   <212> DNA
   <213> artificial sequence
<220>
   <223> chemically synthesized
<400> 41
   cctcctgcgt gtcctggccc acagcattag aggcataatc tggcacgtcg taaggatagc 60
   ggccggcctg gccgctagcg gtaccggccg cctgggcctc 100
<210> 42
   <211> 77
   <212> DNA
   <213> artificial sequence
<220>
   <223> chemically synthesized
<400> 42
   ggtggttcct ctagatcttc ctcctctggt ggcggtggct cgggcggtgg tgggcaggtg 60
   cagctggtgc agtctgg 77
<210> 43
   <211> 77
   <212> DNA
   <213> artificial sequence
<220>
   <223> chemically synthesized
<400> 43
   ggtggttcct ctagatcttc ctcctctggt ggcggtggct cgggcggtgg tgggcagatc 60
   accttgaagg agtctgg 77
<210> 44
   <211> 76
   <212> DNA
   <213> artificial sequence
<220>
   <223> chemically synthesized
<400> 44
   ggtggttcct ctagatcttc ctcctctggt ggcggtggct cgggcggtgg tggggaggtg 60
   cagctgktgg agtctg 76
<210> 45
   <211> 77
   <212> DNA
   <213> artificial sequence
<220>
   <223> chemically synthesized
<400> 45
   ggtggttcct ctagatcttc ctcctctggt ggcggtggct cgggcggtgg tgggcaggtg 60
   cagctacagc agtgggg 77
<210> 46
   <211> 77
   <212> DNA
   <213> artificial sequence
<220>
   <223> chemically synthesized
<400> 46
   ggtggttcct ctagatcttc ctcctctggt ggcggtggct cgggcggtgg tgggcaggtg 60
   cagctgcagg agtcggg 77
<210> 47
   <211> 77
   <212> DNA
   <213> artificial sequence
<220>
   <223> chemically synthesized
<400> 47
   ggtggttcct ctagatcttc ctcctctggt ggcggtggct cgggcggtgg tggggaggtg 60
   cagctggtgs agtctgg 77
<210> 48
   <211> 46
   <212> DNA
   <213> artificial sequence
<220>
   <223> chemically synthesized
<400> 48
   cctggccggc ctggccacta gtgaccgatg ggcccttggt ggargc 46
<210> 49
   <211> 37
   <212> DNA
   <213> artificial sequence
<220>
   <223> chemically synthesized
<400> 49
   gggcccaggc ggccgagctc cagatgaccc agtctcc 37
<210> 50
   <211> 37
   <212> DNA
   <213> artificial sequence
<220>
   <223> chemically synthesized
<400> 50
   gggcccaggc ggccgagctc gtgatgacyc agtctcc 37
<210> 51
   <211> 37
   <212> DNA
   <213> artificial sequence
<220>
   <223> chemically synthesized
<400> 51
   gggcccaggc ggccgagctc gtgwtgacrc agtctcc 37
<210> 52
   <211> 37
   <212> DNA
   <213> artificial sequence
<220>
   <223> chemically synthesized
<400> 52
   gggcccaggc ggccgagctc acactcacgc agtctcc 37
<210> 53
   <211> 42
   <212> DNA
   <213> artificial sequence
<220>
   <223> chemically synthesized
<400> 53
   ggaagatcta gaggaaccac ctttgatytc caccttggtc cc 42
<210> 54
   <211> 42
   <212> DNA
   <213> artificial sequence
<220>
   <223> chemically synthesized
<400> 54
   ggaagatcta gaggaaccac ctttgatctc cagcttggtc cc 42
<210> 55
   <211> 42
   <212> DNA
   <213> artificial sequence
<220>
   <223> chemically synthesized
<400> 55
   ggaagatcta gaggaaccac ctttgatatc cactttggtc cc 42
<210> 56
   <211> 42
   <212> DNA
   <213> artificial sequence
<220>
   <223> chemically synthesized
<400> 56
   ggaagatcta gaggaaccac ctttaatctc cagtcgtgtc cc 42
<210> 57
   <211> 40
   <212> DNA
   <213> artificial sequence
<220>
   <223> chemically synthesized
<400> 57
   gggcccaggc ggccgagctc gtgbtgacgc agccgccctc 40
<210> 58
   <211> 40
   <212> DNA
   <213> artificial sequence
<220>
   <223> chemically synthesized
<400> 58
   gggcccaggc ggccgagctc gtgctgactc agccaccctc 40
<210> 59
   <211> 43
   <212> DNA
   <213> artificial sequence
<220>
   <223> chemically synthesized
<400> 59
   gggcccaggc ggccgagctc gccctgactc agcctccctc cgt 43
<210> 60
   <211> 46
   <212> DNA
   <213> artificial sequence
<220>
   <223> chemically synthesized
<400> 60
   gggcccaggc ggccgagctc gagctgactc agccaccctc agtgtc 46
<210> 61
   <211> 40
   <212> DNA
   <213> artificial sequence
<220>
   <223> chemically synthesized
<400> 61
   gggcccaggc ggccgagctc gtgctgactc aatcgccctc 40
<210> 62
   <211> 40
   <212> DNA
   <213> artificial sequence
<220>
   <223> chemically synthesized
<400> 62
   gggcccaggc ggccgagctc atgctgactc agccccactc 40
<210> 63
   <211> 40
   <212> DNA
   <213> artificial sequence
<220>
   <223> chemically synthesized
<400> 63
   gggcccaggc ggccgagctc gtggtgacyc aggagccmtc 40
<210> 64
   <211> 40
   <212> DNA
   <213> artificial sequence
<220>
   <223> chemically synthesized
<400> 64
   gggcccaggc ggccgagctc gtgctgactc agccaccttc 40
<210> 65
   <211> 40
   <212> DNA
   <213> artificial sequence
<220>
   <223> chemically synthesized
<400> 65
   gggcccaggc ggccgagctc gggcagactc agcagctctc 40
<210> 66
   <211> 45
   <212> DNA
   <213> artificial sequence
<220>
   <223> chemically synthesized
<400> 66
   ggaagatcta gaggaaccac cgcctaggac ggtcascttg gtscc 45
<210> 67
   <211> 65
   <212> DNA
   <213> artificial synthesized
<400> 67
   ggaagatcta gaggaaccac cgcctaaaat gatcagctgg gttcc 45
<210> 68
   <211> 45
   <212> DNA
   <213> artificial sequence
<220>
   <223> chemically synthesized
<400> 68
   ggaagatcta gaggaaccac cgccgaggac ggtcagctag gtscc 45
<210> 69
   <211> 41
   <212> DNA
   <213> artificial sequence
<220>
   <223> chemically synthesized
<400> 69
   gaggaggagg aggaggaggc ggggcccagg cggccgagct c 41
<210> 70
   <211> 41
   <212> DNA
   <213> artificial sequence
<220>
   <223> chemically synthesized
<400> 70
   gaggaggagg aggaggagcc tggccggcct ggccactagt g 41
<210> 71
   <211> 4625
   <212> DNA
   <213> artificial sequence
<220>
   <223> chemically synthesized
<400> 71
   atgcattagt tattaatagt aatcaattac ggggtcatta gttcatagcc catatatgga     60
   gttccgcgtt acataactta cggtaaatgg cccgcctggc tgaccgccca acgacccccg    120
   cccattgacg tcaataatga cgtatgttcc catagtaacg ccaataggga ctttccattg    180
   acgtcaatgg gtggagtatt tacggtaaac tgcccacttg gcagtacatc aagtgtatca    240
   tatgccaagt acgcccccta ttgacgtcaa tgacggtaaa tggcccgcct ggcattatgc    300
   ccagtacatg accttatggg actttcctac ttggcagtac atctacgtat tagtcatcgc    360
   tattaccatg gtgatgcggt tttggcagta catcaatggg cgtggatagc ggtttgactc    420
   acggggattt ccaagtctcc accccattga cgtcaatggg agtttgtttt ggcaccaaaa    480
   tcaacgggac tttccaaaat gtcgtaacaa ctccgcccca ttgacgcaaa tgggcggtag    540
   gcgtgtacgg tgggaggtct atataagcag agctggttta gtgaaccgtc agatccgcta    600
   gcgattacgc caagctcgaa attaaccctc actaaaggga acaaaagctg gagctggcta    660
   gcgccaccat ggacatgagg gtccccgctc agctcctggg gctcctgcta ctctggctcc    720
   gaggtgccag atgtgacatc gagctcctgc aggaattcga tatcaaacga actgtggctg    780
   caccatctgt cttcatcttc ccgccatctg atgagcagtt gaaatctgga actgcctctg    840
   ttgtgtgcct gctgaataac ttctatccca gagaggccaa agtacagtgg aaggtggata    900
   acgccctcca atcgggtaac tcccaggaga gtgtcacaga gcaggacagc aaggacagca    960
   cctacagcct cagcagcacc ctgacgctga gcaaagcaga ctacgagaaa cacaaagtct   1020
   acgcctgcga agtcacccat cagggcctga gttcgcccgt cacaaagagc ttcaacaggg   1080
   gagagtgtta ggtttaaacg gtaccaggta agtgtaccca attcgcccta tagtgagtcg   1140
   tattacaatt cactcgatcg cccttcccaa cagttgcgca gcctgaatgg cgaatggaga   1200
   tccaattttt aagtgtataa tgtgttaaac tactgattct aattgtttgt gtattttaga   1260
   ttcacagtcc caaggctcat ttcaggcccc tcagtcctca cagtctgttc atgatcataa   1320
   tcagccatac cacatttgta gaggttttac ttgctttaaa aaacctccca cacctccccc   1380
   tgaacctgaa acataaaatg aatgcaattg ttgttgttaa cttgtttatt gcagcttata   1440
   atggttacaa ataaagcaat agcatcacaa atttcacaaa taaagcattt ttttcactgc   1500
   attctagttg tggtttgtcc aaactcatca atgtatctta acgcgtaaat tgtaagcgtt 1560
   aatattttgt taaaattcgc gttaaatttt tgttaaatca gctcattttt taaccaatag 1620
   gccgaaatcg gcaaaatccc ttataaatca aaagaataga ccgagatagg gttgagtgtt 1680
   gttccagttt ggaacaagag tccactatta aagaacgtgg actccaacgt caaagggcga 1740
   aaaaccgtct atcagggcga tggcccacta cgtgaaccat caccctaatc aagttttttg 1800
   gggtcgaggt gccgtaaagc actaaatcgg aaccctaaag ggagcccccg atttagagct 1860
   tgacggggaa agccggcgaa cgtggcgaga aaggaaggga agaaagcgaa aggagcgggc 1920
   gctagggcgc tggcaagtgt agcggtcacg ctgcgcgtaa ccaccacacc cgccgcgctt 1980
   aatgcgccgc tacagggcgc gtcaggtggc acttttcggg gaaatgtgcg cggaacccct 2040
   atttgtttat ttttctaaat acattcaaat atgtatccgc tcatgagaca ataaccctga 2100
   taaatgcttc aataatattg aaaaaggaag aatcctgagg cggaaagaac cagctgtgga 2160
   atgtgtgtca gttagggtgt ggaaagtccc caggctcccc agcaggcaga agtatgcaaa 2220
   gcatgcatct caattagtca gcaaccaggt gtggaaagtc cccaggctcc ccagcaggca 2280
   gaagtatgca aagcatgcat ctcaattagt cagcaaccat agtcccgccc ctaactccgc 2340
   ccatcccgcc cctaactccg cccagttccg cccattctcc gccccatggc tgactaattt 2400
   tttttattta tgcagaggcc gaggccgcct cggcctctga gctattccag aagtagtgag 2460
   gaggcttttt tggaggccta ggcttttgca aagatcgatc aagagacagg atgaggatcg 2520
   tttcgcatga ttgaacaaga tggattgcac gcaggttctc cggccgcttg ggtggagagg 2580
   ctattcggct atgactgggc acaacagaca atcggctgct ctgatgccgc cgtgttccgg 2640
   ctgtcagcgc aggggcgccc ggttcttttt gtcaagaccg acctgtccgg tgccctgaat 2700
   gaactgcaag acgaggcagc gcggctatcg tggctggcca cgacgggcgt tccttgcgca 2760
   gctgtgctcg acgttgtcac tgaagcggga agggactggc tgctattggg cgaagtgccg 2820
   gggcaggatc tcctgtcatc tcaccttgct cctgccgaga aagtatccat catggctgat 2880
   gcaatgcggc ggctgcatac gcttgatccg gctacctgcc cattcgacca ccaagcgaaa 2940
   catcgcatcg agcgagcacg tactcggatg gaagccggtc ttgtcgatca ggatgatctg 3000
   gacgaagaac atcaggggct cgcgccagcc gaactgttcg ccaggctcaa ggcgagcatg 3060
   cccgacggcg aggatctcgt cgtgacccat ggcgatgcct gcttgccgaa tatcatggtg 3120
   gaaaatggcc gcttttctgg attcatcgac tgtggccggc tgggtgtggc ggaccgctat 3180
   caggacatag cgttggctac ccgtgatatt gctgaagaac ttggcggcga atgggctgac 3240
   cgcttcctcg tgctttacgg tatcgccgct cccgattcgc agcgcatcgc cttctatcgc 3300
   cttcttgacg agttcttctg agcgggactc tggggttcga aatgaccgac caagcgacgc 3360
   ccaacctgcc atcacgagat ttcgattcca ccgccgcctt ctatgaaagg ttgggcttcg 3420
   gaatcgtttt ccgggacgcc ggctggatga tcctccagcg cggggatctc atgctggagt 3480
   tcttcgccca ccctaggggg aggctaactg aaacacggaa ggagacaata ccggaaggaa 3540
   cccgcgctat gacggcaata aaaagacaga ataaaacgca cggtgttggg tcgtttgttc 3600
   ataaacgcgg ggttcggtcc cagggctggc actctgtcga taccccaccg agaccccatt 3660
   ggggccaata cgcccgcgtt tcttcctttt ccccacccca ccccccaagt tcgggtgaag 3720
   gcccagggct cgcagccaac gtcggggcgg caggccctgc catagcctca ggttactcat 3780
   atatacttta gattgattta aaacttcatt tttaatttaa aaggatctag gtgaagatcc 3840
   tttttgataa tctcatgacc aaaatccctt aacgtgagtt ttcgttccac tgagcgtcag 3900
   accccgtaga aaagatcaaa ggatcttctt gagatccttt ttttctgcgc gtaatctgct 3960
   gcttgcaaac aaaaaaacca ccgctaccag cggtggtttg tttgccggat caagagctac 4020
   caactctttt tccgaaggta actggcttca gcagagcgca gataccaaat actgtccttc 4080
   tagtgtagcc gtagttaggc caccacttca agaactctgt agcaccgcct acatacctcg 4140
   ctctgctaat cctgttacca gtggctgctg ccagtggcga taagtcgtgt cttaccgggt 4200
   tggactcaag acgatagtta ccggataagg cgcagcggtc gggctgaacg gggggttcgt 4260
   gcacacagcc cagcttggag cgaacgacct acaccgaact gagataccta cagcgtgagc 4320
   tatgagaaag cgccacgctt cccgaaggga gaaaggcgga caggtatccg gtaagcggca 4380
   gggtcggaac aggagagcgc acgagggagc ttccaggggg aaacgcctgg tatctttata 4440
   gtcctgtcgg gtttcgccac ctctgacttg agcgtcgatt tttgtgatgc tcgtcagggg 4500
   ggcggagcct atggaaaaac gccagcaacg cggccttttt acggttcctg gccttttgct 4560
   ggccttttgc tcacatgttc tttcctgcgt tatcccctga ttctgtggat aaccgtatta 4620
   ccgcc 4625
<210> 72
   <211> 49
   <212> DNA
   <213> artificial sequence
<220>
   <223> chemically synthesized
<400> 72
   ggctagcgcc accatggaca tgagggtccc cgctcagctc ctggggctc 49
<210> 73
   <211> 47
   <212> DNA
   <213> artificial sequence
<220>
   <223> chemically synthesized
<400> 73
   caggagctga gcggggaccc tcatgtccat ggtggcgcta gccagct 47
<210> 74
   <211> 47
   <212> DNA
   <213> artificial sequence
<220>
   <223> chemically synthesized
<400> 74
   ctgctactct ggctccgagg tgccagatgt gacatcgagc tcctgca 47
<210> 75
   <211> 49
   <212> DNA
   <213> artificial sequence
<220>
   <223> chemically synthesized
<400> 75
   ggagctcgat gtcacatctg gcacctcgga gccagagtag caggagccc 49
<210> 76
   <211> 35
   <212> DNA
   <213> artificial sequence
<220>
   <223> chemically synthesized
<400> 76
   gaggaggata tcaaacgaac tgtggctgca ccatc 35
<210> 77
   <211> 68
   <212> DNA
   <213> artificial sequence
<220>
   <223> chemically synthesized
<400> 77
   gaggagggta ccgtttaaac ctaacactct cccctgttga agctctttgt gacgggcgaa 60
   ctcaggcc 68
<210> 78
   <211> 5257
   <212> DNA
   <213> artificial sequence
<220>
   <223> chemically synthesized
<400> 78
   atgcattagt tattaatagt aatcaattac ggggtcatta gttcatagcc catatatgga      60
   gttccgcgtt acataactta cggtaaatgg cccgcctggc tgaccgccca acgacccccg     120
   cccattgacg tcaataatga cgtatgttcc catagtaacg ccaataggga ctttccattg     180
   acgtcaatgg gtggagtatt tacggtaaac tgcccacttg gcagtacatc aagtgtatca     240
   tatgccaagt acgcccccta ttgacgtcaa tgacggtaaa tggcccgcct ggcattatgc     300
   ccagtacatg accttatggg actttcctac ttggcagtac atctacgtat tagtcatcgc     360
   tattaccatg gtgatgcggt tttggcagta catcaatggg cgtggatagc ggtttgactc     420
   acggggattt ccaagtctcc accccattga cgtcaatggg agtttgtttt ggcaccaaaa     480
   tcaacgggac tttccaaaat gtcgtaacaa ctccgcccca ttgacgcaaa tgggcggtag     540
   gcgtgtacgg tgggaggtct atataagcag agctggttta gtgaaccgtc agatccgcta     600
   gcgattacgc caagctcgaa attaaccctc actaaaggga acaaaagctg gagctcggcg     660
   cgccaccatg gactggacct ggaggatcct cttcttggtg gcagcagcca caggagccca     720
   ctcccagatg caactgctcg aggcctccac caagggccca tcggtcttcc ccctggcgcc     780
   ctgctccagg agcacctccg agagcacagc ggccctgggc tgcctggtca aggactactt     840
   ccccgaaccg gtgacggtgt cgtggaactc aggcgctctg accagcggcg tgcacacctt     900
   cccagctgtc ctacagtcct caggactcta ctccctcagc agcgtggtga ccgtgccctc     960
   cagcaacttc ggcacccaga cctacacctg caacgtagat cacaagccca gcaacaccaa    1020
   ggtggacaag acagttgagc gcaaatgttg tgtcgagtgc ccaccgtgcc cagcaccacc    1080
   tgtggcagga ccgtcagtct tcctcttccc cccaaaaccc aaggacaccc tcatgatctc    1140
   ccggacccct gaggtcacgt gcgtggtggt ggacgtgagc cacgaagacc ccgaggtcca    1200
   gttcaactgg tacgtggacg gcgtggaggt gcataatgcc aagacaaagc cacgggagga    1260
   gcagttcaac agcacgttcc gtgtggtcag cgtcctcacc gttgtgcacc aggactggct    1320
   gaacggcaag gagtacaagt gcaaggtctc caacaaaggc ctcccagccc ccatcgagaa    1380
   aaccatctcc aaaaccaaag ggcagccccg agaaccacag gtgtacaccc tgcccccatc    1440
   ccgggaggag atgaccaaga accaggtcag cctgacctgc ctggtcaaag gcttctaccc    1500
   cagcgacatc gccgtggagt gggagagcaa tgggcagccg gagaacaact acaagaccac    1560
   acctcccatg ctggactccg acggctcctt cttcctctac agcaagctca ccgtggacaa    1620
   gagcaggtgg cagcagggga acgtcttctc atgctccgtg atgcatgagg ctctgcacaa 1680
   ccactacacg cagaagagcc tgtccctgtc tccgggtaaa tgattaatta aggtaccagg 1740
   taagtgtacc caattcgccc tatagtgagt cgtattacaa ttcactcgat cgcccttccc 1800
   aacagttgcg cagcctgaat ggcgaatgga gatccaattt ttaagtgtat aatgtgttaa 1860
   actactgatt ctaattgttt gtgtatttta gattcacagt cccaaggctc atttcaggcc 1920
   cctcagtcct cacagtctgt tcatgatcat aatcagccat accacatttg tagaggtttt 1980
   acttgcttta aaaaacctcc cacacctccc cctgaacctg aaacataaaa tgaatgcaat 2040
   tgttgttgtt aacttgttta ttgcagctta taatggttac aaataaagca atagcatcac 2100
   aaatttcaca aataaagcat ttttttcact gcattctagt tgtggtttgt ccaaactcat 2160
   caatgtatct taacgcgtaa attgtaagcg ttaatatttt gttaaaattc gcgttaaatt 2220
   tttgttaaat cagctcattt tttaaccaat aggccgaaat cggcaaaatc ccttataaat 2280
   caaaagaata gaccgagata gggttgagtg ttgttccagt ttggaacaag agtccactat 2340
   taaagaacgt ggactccaac gtcaaagggc gaaaaaccgt ctatcagggc gatggcccac 2400
   tacgtgaacc atcaccctaa tcaagttttt tggggtcgag gtgccgtaaa gcactaaatc 2460
   ggaaccctaa agggagcccc cgatttagag cttgacgggg aaagccggcg aacgtggcga 2520
   gaaaggaagg gaagaaagcg aaaggagcgg gcgctagggc gctggcaagt gtagcggtca 2580
   cgctgcgcgt aaccaccaca cccgccgcgc ttaatgcgcc gctacagggc gcgtcaggtg 2640
   gcacttttcg gggaaatgtg cgcggaaccc ctatttgttt atttttctaa atacattcaa 2700
   atatgtatcc gctcatgaga caataaccct gataaatgct tcaataatat tgaaaaagga 2760
   agaatcctga ggcggaaaga accagctgtg gaatgtgtgt cagttagggt gtggaaagtc 2820
   cccaggctcc ccagcaggca gaagtatgca aagcatgcat ctcaattagt cagcaaccag 2880
   gtgtggaaag tccccaggct ccccagcagg cagaagtatg caaagcatgc atctcaatta 2940
   gtcagcaacc atagtcccgc ccctaactcc gcccatcccg cccctaactc cgcccagttc 3000
   cgcccattct ccgccccatg gctgactaat tttttttatt tatgcagagg ccgaggccgc 3060
   ctcggcctct gagctattcc agaagtagtg aggaggcttt tttggaggcc taggcttttg 3120
   caaagatcga tcaagagaca ggatgaggat cgtttcgcat gattgaacaa gatggattgc 3180
   acgcaggttc tccggccgct tgggtggaga ggctattcgg ctatgactgg gcacaacaga 3240
   caatcggctg ctctgatgcc gccgtgttcc ggctgtcagc gcaggggcgc ccggttcttt 3300
   ttgtcaagac cgacctgtcc ggtgccctga atgaactgca agacgaggca gcgcggctat 3360
   cgtggctggc cacgacgggc gttccttgcg cagctgtgct cgacgttgtc actgaagcgg 3420
   gaagggactg gctgctattg ggcgaagtgc cggggcagga tctcctgtca tctcaccttg 3480
   ctcctgccga gaaagtatcc atcatggctg atgcaatgcg gcggctgcat acgcttgatc 3540
   cggctacctg cccattcgac caccaagcga aacatcgcat cgagcgagca cgtactcgga 3600
   tggaagccgg tcttgtcgat caggatgatc tggacgaaga acatcagggg ctcgcgccag 3660
   ccgaactgtt cgccaggctc aaggcgagca tgcccgacgg cgaggatctc gtcgtgaccc 3720
   atggcgatgc ctgcttgccg aatatcatgg tggaaaatgg ccgcttttct ggattcatcg 3780
   actgtggccg gctgggtgtg gcggaccgct atcaggacat agcgttggct acccgtgata 3840
   ttgctgaaga acttggcggc gaatgggctg accgcttcct cgtgctttac ggtatcgccg 3900
   ctcccgattc gcagcgcatc gccttctatc gccttcttga cgagttcttc tgagcgggac 3960
   tctggggttc gaaatgaccg accaagcgac gcccaacctg ccatcacgag atttcgattc 4020
   caccgccgcc ttctatgaaa ggttgggctt cggaatcgtt ttccgggacg ccggctggat 4080
   gatcctccag cgcggggatc tcatgctgga gttcttcgcc caccctaggg ggaggctaac 4140
   tgaaacacgg aaggagacaa taccggaagg aacccgcgct atgacggcaa taaaaagaca 4200
   gaataaaacg cacggtgttg ggtcgtttgt tcataaacgc ggggttcggt cccagggctg 4260
   gcactctgtc gataccccac cgagacccca ttggggccaa tacgcccgcg tttcttcctt 4320
   ttccccaccc caccccccaa gttcgggtga aggcccaggg ctcgcagcca acgtcggggc 4380
   ggcaggccct gccatagcct caggttactc atatatactt tagattgatt taaaacttca 4440
   tttttaattt aaaaggatct aggtgaagat cctttttgat aatctcatga ccaaaatccc 4500
   ttaacgtgag ttttcgttcc actgagcgtc agaccccgta gaaaagatca aaggatcttc 4560
   ttgagatcct ttttttctgc gcgtaatctg ctgcttgcaa acaaaaaaac caccgctacc 4620
   agcggtggtt tgtttgccgg atcaagagct accaactctt tttccgaagg taactggctt 4680
   cagcagagcg cagataccaa atactgtcct tctagtgtag ccgtagttag gccaccactt 4740
   caagaactct gtagcaccgc ctacatacct cgctctgcta atcctgttac cagtggctgc 4800
   tgccagtggc gataagtcgt gtcttaccgg gttggactca agacgatagt taccggataa 4860
   ggcgcagcgg tcgggctgaa cggggggttc gtgcacacag cccagcttgg agcgaacgac 4920
   ctacaccgaa ctgagatacc tacagcgtga gctatgagaa agcgccacgc ttcccgaagg 4980
   gagaaaggcg gacaggtatc cggtaagcgg cagggtcgga acaggagagc gcacgaggga 5040
   gcttccaggg ggaaacgcct ggtatcttta tagtcctgtc gggtttcgcc acctctgact 5100
   tgagcgtcga tttttgtgat gctcgtcagg ggggcggagc ctatggaaaa acgccagcaa 5160
   cgcggccttt ttacggttcc tggccttttg ctggcctttt gctcacatgt tctttcctgc 5220
   gttatcccct gattctgtgg ataaccgtat taccgcc 5257
<210> 79
   <211> 38
   <212> DNA
   <213> artificial sequence
<220>
   <223> chemically synthesized
<400> 79
   cggcgcgcca ccatggactg gacctggagg atcctctt 38
<210> 80
   <211> 48
   <212> DNA
   <213> artificial sequence
<220>
   <223> chemically synthesized
<400> 80
   accaagaaga ggatcctcca ggtccagtcc atggtggcgc gccgagct 48
<210> 81
   <211> 44
   <212> DNA
   <213> artificial sequence
<220>
   <223> chemically synthesized
<400> 81
   cttggtggca gcagccacag gagcccactc ccagatgcaa ctgc 44
<210> 82
   <211> 42
   <212> DNA
   <213> artificial sequence
<220>
   <223> chemically synthesized
<400> 82
   tcgagcagtt gcatctggga gtgggctcct gtggctgctg cc 42
<210> 83
   <211> 69
   <212> DNA
   <213> artificial sequence
<220>
   <223> chemically synthesized
<400> 83
   gaggagctcg aggcctccac caagggccca tcggtcttcc ccctggcgcc ctgctccagg 60
   agcacctcc 69
<210> 84
   <211> 42
   <212> DNA
   <213> artificial sequence
<220>
   <223> chemically synthesized
<400> 84
   gaggagggta ccttaattaa tcatttaccc ggagacaggg ag 42
<210> 85
   <211> 4582
   <212> DNA
   <213> artificial sequence
<220>
   <223> chemically synthesized
<400> 85
   atgcattagt tattaatagt aatcaattac ggggtcatta gttcatagcc catatatgga      60
   gttccgcgtt acataactta cggtaaatgg cccgcctggc tgaccgccca acgacccccg     120
   cccattgacg tcaataatga cgtatgttcc catagtaacg ccaataggga ctttccattg     180
   acgtcaatgg gtggagtatt tacggtaaac tgcccacttg gcagtacatc aagtgtatca     240
   tatgccaagt acgcccccta ttgacgtcaa tgacggtaaa tggcccgcct ggcattatgc     300
   ccagtacatg accttatggg actttcctac ttggcagtac atctacgtat tagtcatcgc     360
   tattaccatg gtgatgcggt tttggcagta catcaatggg cgtggatagc ggtttgactc     420
   acggggattt ccaagtctcc accccattga cgtcaatggg agtttgtttt ggcaccaaaa     480
   tcaacgggac tttccaaaat gtcgtaacaa ctccgcccca ttgacgcaaa tgggcggtag     540
   gcgtgtacgg tgggaggtct atataagcag agctggttta gtgaaccgtc agatccgcta     600
   gcgattacgc caagctcgaa attaaccctc actaaaggga acaaaagctg gagctcggcg     660
   cgccaccatg gactggacct ggaggatcct cttcttggtg gcagcagcca caggagccca     720
   ctcccagatg caactgctcg aggcctccac caagggccca tcggtcttcc ccctggcgcc     780
   ctgctccagg agcacctccg agagcacagc ggccctgggc tgcctggtca aggactactt     840
   ccccgaaccg gtgacggtgt cgtggaactc aggcgctctg accagcggcg tgcacacctt     900
   cccagctgtc ctacagtcct caggactcta ctccctcagc agcgtggtga ccgtgccctc     960
   cagcaacttc ggcacccaga cctacacctg caacgtagat cacaagccca gcaacaccaa    1020
   ggtggacaag acagttgagc gcaaatgatt aattaaggta ccaggtaagt gtacccaatt    1080
   cgccctatag tgagtcgtat tacaattcac tcgatcgccc ttcccaacag ttgcgcagcc    1140
   tgaatggcga atggagatcc aatttttaag tgtataatgt gttaaactac tgattctaat 1200
   tgtttgtgta ttttagattc acagtcccaa ggctcatttc aggcccctca gtcctcacag 1260
   tctgttcatg atcataatca gccataccac atttgtagag gttttacttg ctttaaaaaa 1320
   cctcccacac ctccccctga acctgaaaca taaaatgaat gcaattgttg ttgttaactt 1380
   gtttattgca gcttataatg gttacaaata aagcaatagc atcacaaatt tcacaaataa 1440
   agcatttttt tcactgcatt ctagttgtgg tttgtccaaa ctcatcaatg tatcttaacg 1500
   cgtaaattgt aagcgttaat attttgttaa aattcgcgtt aaatttttgt taaatcagct 1560
   cattttttaa ccaataggcc gaaatcggca aaatccctta taaatcaaaa gaatagaccg 1620
   agatagggtt gagtgttgtt ccagtttgga acaagagtcc actattaaag aacgtggact 1680
   ccaacgtcaa agggcgaaaa accgtctatc agggcgatgg cccactacgt gaaccatcac 1740
   cctaatcaag ttttttgggg tcgaggtgcc gtaaagcact aaatcggaac cctaaaggga 1800
   gcccccgatt tagagcttga cggggaaagc cggcgaacgt ggcgagaaag gaagggaaga 1860
   aagcgaaagg agcgggcgct agggcgctgg caagtgtagc ggtcacgctg cgcgtaacca 1920
   ccacacccgc cgcgcttaat gcgccgctac agggcgcgtc aggtggcact tttcggggaa 1980
   atgtgcgcgg aacccctatt tgtttatttt tctaaataca ttcaaatatg tatccgctca 2040
   tgagacaata accctgataa atgcttcaat aatattgaaa aaggaagaat cctgaggcgg 2100
   aaagaaccag ctgtggaatg tgtgtcagtt agggtgtgga aagtccccag gctccccagc 2160
   aggcagaagt atgcaaagca tgcatctcaa ttagtcagca accaggtgtg gaaagtcccc 2220
   aggctcccca gcaggcagaa gtatgcaaag catgcatctc aattagtcag caaccatagt 2280
   cccgccccta actccgccca tcccgcccct aactccgccc agttccgccc attctccgcc 2340
   ccatggctga ctaatttttt ttatttatgc agaggccgag gccgcctcgg cctctgagct 2400
   attccagaag tagtgaggag gcttttttgg aggcctaggc ttttgcaaag atcgatcaag 2460
   agacaggatg aggatcgttt cgcatgattg aacaagatgg attgcacgca ggttctccgg 2520
   ccgcttgggt ggagaggcta ttcggctatg actgggcaca acagacaatc ggctgctctg 2580
   atgccgccgt gttccggctg tcagcgcagg ggcgcccggt tctttttgtc aagaccgacc 2640
   tgtccggtgc cctgaatgaa ctgcaagacg aggcagcgcg gctatcgtgg ctggccacga 2700
   cgggcgttcc ttgcgcagct gtgctcgacg ttgtcactga agcgggaagg gactggctgc 2760
   tattgggcga agtgccgggg caggatctcc tgtcatctca ccttgctcct gccgagaaag 2820
   tatccatcat ggctgatgca atgcggcggc tgcatacgct tgatccggct acctgcccat 2880
   tcgaccacca agcgaaacat cgcatcgagc gagcacgtac tcggatggaa gccggtcttg 2940
   tcgatcagga tgatctggac gaagaacatc aggggctcgc gccagccgaa ctgttcgcca 3000
   ggctcaaggc gagcatgccc gacggcgagg atctcgtcgt gacccatggc gatgcctgct   3060
   tgccgaatat catggtggaa aatggccgct tttctggatt catcgactgt ggccggctgg   3120
   gtgtggcgga ccgctatcag gacatagcgt tggctacccg tgatattgct gaagaacttg   3180
   gcggcgaatg ggctgaccgc ttcctcgtgc tttacggtat cgccgctccc gattcgcagc   3240
   gcatcgcctt ctatcgcctt cttgacgagt tcttctgagc gggactctgg ggttcgaaat   3300
   gaccgaccaa gcgacgccca acctgccatc acgagatttc gattccaccg ccgccttcta   3360
   tgaaaggttg ggcttcggaa tcgttttccg ggacgccggc tggatgatcc tccagcgcgg   3420
   ggatctcatg ctggagttct tcgcccaccc tagggggagg ctaactgaaa cacggaagga   3480
   gacaataccg gaaggaaccc gcgctatgac ggcaataaaa agacagaata aaacgcacgg   3540
   tgttgggtcg tttgttcata aacgcggggt tcggtcccag ggctggcact ctgtcgatac   3600
   cccaccgaga ccccattggg gccaatacgc ccgcgtttct tccttttccc caccccaccc   3660
   cccaagttcg ggtgaaggcc cagggctcgc agccaacgtc ggggcggcag gccctgccat   3720
   agcctcaggt tactcatata tactttagat tgatttaaaa cttcattttt aatttaaaag   3780
   gatctaggtg aagatccttt ttgataatct catgaccaaa atcccttaac gtgagttttc   3840
   gttccactga gcgtcagacc ccgtagaaaa gatcaaagga tcttcttgag atcctttttt   3900
   tctgcgcgta atctgctgct tgcaaacaaa aaaaccaccg ctaccagcgg tggtttgttt   3960
   gccggatcaa gagctaccaa ctctttttcc gaaggtaact ggcttcagca gagcgcagat   4020
   accaaatact gtccttctag tgtagccgta gttaggccac cacttcaaga actctgtagc   4080
   accgcctaca tacctcgctc tgctaatcct gttaccagtg gctgctgcca gtggcgataa   4140
   gtcgtgtctt accgggttgg actcaagacg atagttaccg gataaggcgc agcggtcggg   4200
   ctgaacgggg ggttcgtgca cacagcccag cttggagcga acgacctaca ccgaactgag   4260
   atacctacag cgtgagctat gagaaagcgc cacgcttccc gaagggagaa aggcggacag   4320
   gtatccggta agcggcaggg tcggaacagg agagcgcacg agggagcttc cagggggaaa   4380
   cgcctggtat ctttatagtc ctgtcgggtt tcgccacctc tgacttgagc gtcgattttt   4440
   gtgatgctcg tcaggggggc ggagcctatg gaaaaacgcc agcaacgcgg cctttttacg   4500
   gttcctggcc ttttgctggc cttttgctca catgttcttt cctgcgttat cccctgattc   4560
   tgtggataac cgtattaccg cc                                               4582
<210> 86
   <211> 33
   <212> DNA
   <213> artificial sequence
<220>
   <223> chemically synthesized
<400> 86
   gaggagctcg aggcctccac caagggccca tcg 33
<210> 87
   <211> 44
   <212> DNA
   <213> artificial sequence
<220>
   <223> chemically synthesized
<400> 87
   gaggagggta ccttaattaa tcatttgcgc tcaactgtct tgtc 44
<210> 88
   <211> 5269
   <212> DNA
   <213> artificial sequence
<220>
   <223> chemically synthesized
<400> 88
   atgcattagt tattaatagt aatcaattac ggggtcatta gttcatagcc catatatgga      60
   gttccgcgtt acataactta cggtaaatgg cccgcctggc tgaccgccca acgacccccg     120
   cccattgacg tcaataatga cgtatgttcc catagtaacg ccaataggga ctttccattg     180
   acgtcaatgg gtggagtatt tacggtaaac tgcccacttg gcagtacatc aagtgtatca     240
   tatgccaagt acgcccccta ttgacgtcaa tgacggtaaa tggcccgcct ggcattatgc     300
   ccagtacatg accttatggg actttcctac ttggcagtac atctacgtat tagtcatcgc     360
   tattaccatg gtgatgcggt tttggcagta catcaatggg cgtggatagc ggtttgactc     420
   acggggattt ccaagtctcc accccattga cgtcaatggg agtttgtttt ggcaccaaaa     480
   tcaacgggac tttccaaaat gtcgtaacaa ctccgcccca ttgacgcaaa tgggcggtag     540
   gcgtgtacgg tgggaggtct atataagcag agctggttta gtgaaccgtc agatccgcta     600
   gcgattacgc caagctcgaa attaaccctc actaaaggga acaaaagctg gagctcggcg     660
   cgccaccatg gactggacct ggaggatcct cttcttggtg gcagcagcca caggagccca     720
   ctcccagatg caactgctcg aggcctccac caagggccca tcggtcttcc ccctggcacc     780
   ctcctccaag agcacctctg ggggcacagc ggccctgggc tgcctggtca aggactactt     840
   ccccgaaccg gtgacggtgt cgtggaactc aggcgccctg accagcggcg tgcacacctt     900
   cccggctgtc ctacagtcct caggactcta ctccctcagc agcgtggtga ccgtgccctc     960
   cagcagcttg ggcacccaga cctacatctg caacgtgaat cacaagccca gcaacaccaa    1020
   ggtggacaag aaagttgagc ccaaatcttg tgacaaaact cacacatgcc caccgtgccc 1080
   agcacctgaa ctcctggggg gaccgtcagt cttcctcttc cccccaaaac ccaaggacac 1140
   cctcatgatc tcccggaccc ctgaggtcac atgcgtggtg gtggacgtga gccacgaaga 1200
   ccctgaggtc aagttcaact ggtacgtgga cggcgtggag gtgcataatg ccaagacaaa 1260
   gccgcgggag gagcagtaca acagcacgta ccgtgtggtc agcgtcctca ccgtcctgca 1320
   ccaggactgg ctgaatggca aggagtacaa gtgcaaggtc tccaacaaag ccctcccagc 1380
   ccccatcgag aaaaccatct ccaaagccaa agggcagccc cgagaaccac aggtgtacac 1440
   cctgccccca tcccgggatg agctgaccaa gaaccaggtc agcctgacct gcctggtcaa 1500
   aggcttctat cccagcgaca tcgccgtgga gtgggagagc aatgggcagc cggagaacaa 1560
   ctacaagacc acgcctcccg tgctggactc cgacggctcc ttcttcctct acagcaagct 1620
   caccgtggac aagagcaggt ggcagcaggg gaacgtcttc tcatgctccg tgatgcatga 1680
   ggctctgcac aaccactaca cgcagaagag cctctccctg tctccgggta aatgattaat 1740
   taaggtacca ggtaagtgta cccaattcgc cctatagtga gtcgtattac aattcactcg 1800
   atcgcccttc ccaacagttg cgcagcctga atggcgaatg gagatccaat ttttaagtgt 1860
   ataatgtgtt aaactactga ttctaattgt ttgtgtattt tagattcaca gtcccaaggc 1920
   tcatttcagg cccctcagtc ctcacagtct gttcatgatc ataatcagcc ataccacatt 1980
   tgtagaggtt ttacttgctt taaaaaacct cccacacctc cccctgaacc tgaaacataa 2040
   aatgaatgca attgttgttg ttaacttgtt tattgcagct tataatggtt acaaataaag 2100
   caatagcatc acaaatttca caaataaagc atttttttca ctgcattcta gttgtggttt 2160
   gtccaaactc atcaatgtat cttaacgcgt aaattgtaag cgttaatatt ttgttaaaat 2220
   tcgcgttaaa tttttgttaa atcagctcat tttttaacca ataggccgaa atcggcaaaa 2280
   tcccttataa atcaaaagaa tagaccgaga tagggttgag tgttgttcca gtttggaaca 2340
   agagtccact attaaagaac gtggactcca acgtcaaagg gcgaaaaacc gtctatcagg 2400
   gcgatggccc actacgtgaa ccatcaccct aatcaagttt tttggggtcg aggtgccgta 2460
   aagcactaaa tcggaaccct aaagggagcc cccgatttag agcttgacgg ggaaagccgg 2520
   cgaacgtggc gagaaaggaa gggaagaaag cgaaaggagc gggcgctagg gcgctggcaa 2580
   gtgtagcggt cacgctgcgc gtaaccacca cacccgccgc gcttaatgcg ccgctacagg 2640
   gcgcgtcagg tggcactttt cggggaaatg tgcgcggaac ccctatttgt ttatttttct 2700
   aaatacattc aaatatgtat ccgctcatga gacaataacc ctgataaatg cttcaataat 2760
   attgaaaaag gaagaatcct gaggcggaaa gaaccagctg tggaatgtgt gtcagttagg 2820
   gtgtggaaag tccccaggct ccccagcagg cagaagtatg caaagcatgc atctcaatta 2880
   gtcagcaacc aggtgtggaa agtccccagg ctccccagca ggcagaagta tgcaaagcat 2940
   gcatctcaat tagtcagcaa ccatagtccc gcccctaact ccgcccatcc cgcccctaac 3000
   tccgcccagt tccgcccatt ctccgcccca tggctgacta atttttttta tttatgcaga 3060
   ggccgaggcc gcctcggcct ctgagctatt ccagaagtag tgaggaggct tttttggagg 3120
   cctaggcttt tgcaaagatc gatcaagaga caggatgagg atcgtttcgc atgattgaac 3180
   aagatggatt gcacgcaggt tctccggccg cttgggtgga gaggctattc ggctatgact 3240
   gggcacaaca gacaatcggc tgctctgatg ccgccgtgtt ccggctgtca gcgcaggggc 3300
   gcccggttct ttttgtcaag accgacctgt ccggtgccct gaatgaactg caagacgagg 3360
   cagcgcggct atcgtggctg gccacgacgg gcgttccttg cgcagctgtg ctcgacgttg 3420
   tcactgaagc gggaagggac tggctgctat tgggcgaagt gccggggcag gatctcctgt 3480
   catctcacct tgctcctgcc gagaaagtat ccatcatggc tgatgcaatg cggcggctgc 3540
   atacgcttga tccggctacc tgcccattcg accaccaagc gaaacatcgc atcgagcgag 3600
   cacgtactcg gatggaagcc ggtcttgtcg atcaggatga tctggacgaa gaacatcagg 3660
   ggctcgcgcc agccgaactg ttcgccaggc tcaaggcgag catgcccgac ggcgaggatc 3720
   tcgtcgtgac ccatggcgat gcctgcttgc cgaatatcat ggtggaaaat ggccgctttt 3780
   ctggattcat cgactgtggc cggctgggtg tggcggaccg ctatcaggac atagcgttgg 3840
   ctacccgtga tattgctgaa gaacttggcg gcgaatgggc tgaccgcttc ctcgtgcttt 3900
   acggtatcgc cgctcccgat tcgcagcgca tcgccttcta tcgccttctt gacgagttct 3960
   tctgagcggg actctggggt tcgaaatgac cgaccaagcg acgcccaacc tgccatcacg 4020
   agatttcgat tccaccgccg ccttctatga aaggttgggc ttcggaatcg ttttccggga 4080
   cgccggctgg atgatcctcc agcgcgggga tctcatgctg gagttcttcg cccaccctag 4140
   ggggaggcta actgaaacac ggaaggagac aataccggaa ggaacccgcg ctatgacggc 4200
   aataaaaaga cagaataaaa cgcacggtgt tgggtcgttt gttcataaac gcggggttcg 4260
   gtcccagggc tggcactctg tcgatacccc accgagaccc cattggggcc aatacgcccg 4320
   cgtttcttcc ttttccccac cccacccccc aagttcgggt gaaggcccag ggctcgcagc 4380
   caacgtcggg gcggcaggcc ctgccatagc ctcaggttac tcatatatac tttagattga 4440
   tttaaaactt catttttaat ttaaaaggat ctaggtgaag atcctttttg ataatctcat 4500
   gaccaaaatc ccttaacgtg agttttcgtt ccactgagcg tcagaccccg tagaaaagat 4560
   caaaggatct tcttgagatc ctttttttct gcgcgtaatc tgctgcttgc aaacaaaaaa 4620
   accaccgcta ccagcggtgg tttgtttgcc ggatcaagag ctaccaactc tttttccgaa 4680
   ggtaactggc ttcagcagag cgcagatacc aaatactgtc cttctagtgt agccgtagtt 4740
   aggccaccac ttcaagaact ctgtagcacc gcctacatac ctcgctctgc taatcctgtt 4800
   accagtggct gctgccagtg gcgataagtc gtgtcttacc gggttggact caagacgata 4860
   gttaccggat aaggcgcagc ggtcgggctg aacggggggt tcgtgcacac agcccagctt 4920
   ggagcgaacg acctacaccg aactgagata cctacagcgt gagctatgag aaagcgccac 4980
   gcttcccgaa gggagaaagg cggacaggta tccggtaagc ggcagggtcg gaacaggaga 5040
   gcgcacgagg gagcttccag ggggaaacgc ctggtatctt tatagtcctg tcgggtttcg 5100
   ccacctctga cttgagcgtc gatttttgtg atgctcgtca ggggggcgga gcctatggaa 5160
   aaacgccagc aacgcggcct ttttacggtt cctggccttt tgctggcctt ttgctcacat 5220
   gttctttcct gcgttatccc ctgattctgt ggataaccgt attaccgcc 5269
<210> 89
   <211> 33
   <212> DNA
   <213> artificial sequence
<220>
   <223> chemically synthesized
<400> 89
   caagggccca tcggtcttcc ccctggcacc ctc 33
<210> 90
   <211> 5273
   <212> DNA
   <213> artificial sequence
<220>
   <223> chemically synthesized
<400> 90
   atgcattagt tattaatagt aatcaattac ggggtcatta gttcatagcc catatatgga 60
   gttccgcgtt acataactta cggtaaatgg cccgcctggc tgaccgccca acgacccccg 120
   cccattgacg tcaataatga cgtatgttcc catagtaacg ccaataggga ctttccattg 180
   acgtcaatgg gtggagtatt tacggtaaac tgcccacttg gcagtacatc aagtgtatca 240
   tatgccaagt acgcccccta ttgacgtcaa tgacggtaaa tggcccgcct ggcattatgc 300
   ccagtacatg accttatggg actttcctac ttggcagtac atctacgtat tagtcatcgc 360
   tattaccatg gtgatgcggt tttggcagta catcaatggg cgtggatagc ggtttgactc 420
   acggggattt ccaagtctcc accccattga cgtcaatggg agtttgtttt ggcaccaaaa 480
   tcaacgggac tttccaaaat gtcgtaacaa ctccgcccca ttgacgcaaa tgggcggtag 540
   gcgtgtacgg tgggaggtct atataagcag agctggttta gtgaaccgtc agatccgcta 600
   gcgattacgc caagctcgaa attaaccctc actaaaggga acaaaagctg gagctcggcg 660
   cgccaccatg gactggacct ggaggatcct cttcttggtg gcagcagcca caggagccca 720
   ctcccagatg caactgctcg aggcctccac caagggccca tcggtcttcc ccctggcgcc 780
   ctgctccagg agcacctccg agagcacagc ggccctgggc tgcctggtca aggactactt 840
   ccccgaaccg gtgacggtgt cgtggaactc aggcgccctg accagcggcg tgcacacctt 900
   cccggctgtc ctacagtcct caggactcta ctccctcagc agcgtggtga ccgtgccctc 960
   cagcagcttg ggcacgaaga cctacacctg caatgtagat cacaagccca gcaacaccaa 1020
   ggtggacaag agagttgagt ccaaatatgg tcccccatgc ccatcatgcc cagcacctga 1080
   gttcctgggg ggaccatcag tcttcctgtt ccccccaaaa cccaaggaca ctctcatgat 1140
   ctcccggacc cctgaggtca cgtgcgtggt ggtggacgtg agccaggaag accccgaggt 1200
   ccagttcaac tggtacgtgg atggcgtgga ggtgcataat gccaagacaa agccgcggga 1260
   ggagcagttc aacagcacgt accgtgtggt cagcgtcctc accgtcgtgc accaggactg 1320
   gctgaacggc aaggagtaca agtgcaaggt ctccaacaaa ggcctcccgt cctccatcga 1380
   gaaaaccatc tccaaagcca aagggcagcc ccgagagcca caggtgtaca ccctgccccc 1440
   atcccaggag gagatgacca agaaccaggt cagcctgacc tgcctggtca aaggcttcta 1500
   ccccagcgac atcgccgtgg agtgggagag caatgggcag ccggagaaca actacaagac 1560
   cacgcctccc gtgctggact ccgacggctc cttcttcctc tacagcaggc taaccgtgga 1620
   caagagcagg tggcaggagg ggaatgtctt ctcatgctcc gtgatgcatg aggctctgca 1680
   caaccactac acgcagaaga gcctctccct gtctctgggt aaatgagtgc cagggccggt 1740
   taattaaggt accaggtaag tgtacccaat tcgccctata gtgagtcgta ttacaattca 1800
   ctcgatcgcc cttcccaaca gttgcgcagc ctgaatggcg aatggagatc caatttttaa 1860
   gtgtataatg tgttaaacta ctgattctaa ttgtttgtgt attttagatt cacagtccca 1920
   aggctcattt caggcccctc agtcctcaca gtctgttcat gatcataatc agccatacca 1980
   catttgtaga ggttttactt gctttaaaaa acctcccaca cctccccctg aacctgaaac 2040
   ataaaatgaa tgcaattgtt gttgttaact tgtttattgc agcttataat ggttacaaat 2100
   aaagcaatag catcacaaat ttcacaaata aagcattttt ttcactgcat tctagttgtg 2160
   gtttgtccaa actcatcaat gtatcttaac gcgtaaattg taagcgttaa tattttgtta 2220
   aaattcgcgt taaatttttg ttaaatcagc tcatttttta accaataggc cgaaatcggc 2280
   aaaatccctt ataaatcaaa agaatagacc gagatagggt tgagtgttgt tccagtttgg 2340
   aacaagagtc cactattaaa gaacgtggac tccaacgtca aagggcgaaa aaccgtctat 2400
   cagggcgatg gcccactacg tgaaccatca ccctaatcaa gttttttggg gtcgaggtgc 2460
   cgtaaagcac taaatcggaa ccctaaaggg agcccccgat ttagagcttg acggggaaag 2520
   ccggcgaacg tggcgagaaa ggaagggaag aaagcgaaag gagcgggcgc tagggcgctg 2580
   gcaagtgtag cggtcacgct gcgcgtaacc accacacccg ccgcgcttaa tgcgccgcta 2640
   cagggcgcgt caggtggcac ttttcgggga aatgtgcgcg gaacccctat ttgtttattt 2700
   ttctaaatac attcaaatat gtatccgctc atgagacaat aaccctgata aatgcttcaa 2760
   taatattgaa aaaggaagaa tcctgaggcg gaaagaacca gctgtggaat gtgtgtcagt 2820
   tagggtgtgg aaagtcccca ggctccccag caggcagaag tatgcaaagc atgcatctca 2880
   attagtcagc aaccaggtgt ggaaagtccc caggctcccc agcaggcaga agtatgcaaa 2940
   gcatgcatct caattagtca gcaaccatag tcccgcccct aactccgccc atcccgcccc 3000
   taactccgcc cagttccgcc cattctccgc cccatggctg actaattttt tttatttatg 3060
   cagaggccga ggccgcctcg gcctctgagc tattccagaa gtagtgagga ggcttttttg 3120
   gaggcctagg cttttgcaaa gatcgatcaa gagacaggat gaggatcgtt tcgcatgatt 3180
   gaacaagatg gattgcacgc aggttctccg gccgcttggg tggagaggct attcggctat 3240
   gactgggcac aacagacaat cggctgctct gatgccgccg tgttccggct gtcagcgcag 3300
   gggcgcccgg ttctttttgt caagaccgac ctgtccggtg ccctgaatga actgcaagac 3360
   gaggcagcgc ggctatcgtg gctggccacg acgggcgttc cttgcgcagc tgtgctcgac 3420
   gttgtcactg aagcgggaag ggactggctg ctattgggcg aagtgccggg gcaggatctc 3480
   ctgtcatctc accttgctcc tgccgagaaa gtatccatca tggctgatgc aatgcggcgg 3540
   ctgcatacgc ttgatccggc tacctgccca ttcgaccacc aagcgaaaca tcgcatcgag 3600
   cgagcacgta ctcggatgga agccggtctt gtcgatcagg atgatctgga cgaagaacat 3660
   caggggctcg cgccagccga actgttcgcc aggctcaagg cgagcatgcc cgacggcgag 3720
   gatctcgtcg tgacccatgg cgatgcctgc ttgccgaata tcatggtgga aaatggccgc 3780
   ttttctggat tcatcgactg tggccggctg ggtgtggcgg accgctatca ggacatagcg 3840
   ttggctaccc gtgatattgc tgaagaactt ggcggcgaat gggctgaccg cttcctcgtg 3900
   ctttacggta tcgccgctcc cgattcgcag cgcatcgcct tctatcgcct tcttgacgag 3960
   ttcttctgag cgggactctg gggttcgaaa tgaccgacca agcgacgccc aacctgccat 4020
   cacgagattt cgattccacc gccgccttct atgaaaggtt gggcttcgga atcgttttcc 4080
   gggacgccgg ctggatgatc ctccagcgcg gggatctcat gctggagttc ttcgcccacc 4140
   ctagggggag gctaactgaa acacggaagg agacaatacc ggaaggaacc cgcgctatga 4200
   cggcaataaa aagacagaat aaaacgcacg gtgttgggtc gtttgttcat aaacgcgggg   4260
   ttcggtccca gggctggcac tctgtcgata ccccaccgag accccattgg ggccaatacg   4320
   cccgcgtttc ttccttttcc ccaccccacc ccccaagttc gggtgaaggc ccagggctcg   4380
   cagccaacgt cggggcggca ggccctgcca tagcctcagg ttactcatat atactttaga   4440
   ttgatttaaa acttcatttt taatttaaaa ggatctaggt gaagatcctt tttgataatc   4500
   tcatgaccaa aatcccttaa cgtgagtttt cgttccactg agcgtcagac cccgtagaaa   4560
   agatcaaagg atcttcttga gatccttttt ttctgcgcgt aatctgctgc ttgcaaacaa   4620
   aaaaaccacc gctaccagcg gtggtttgtt tgccggatca agagctacca actctttttc   4680
   cgaaggtaac tggcttcagc agagcgcaga taccaaatac tgtccttcta gtgtagccgt   4740
   agttaggcca ccacttcaag aactctgtag caccgcctac atacctcgct ctgctaatcc   4800
   tgttaccagt ggctgctgcc agtggcgata agtcgtgtct taccgggttg gactcaagac   4860
   gatagttacc ggataaggcg cagcggtcgg gctgaacggg gggttcgtgc acacagccca   4920
   gcttggagcg aacgacctac accgaactga gatacctaca gcgtgagcta tgagaaagcg   4980
   ccacgcttcc cgaagggaga aaggcggaca ggtatccggt aagcggcagg gtcggaacag   5040
   gagagcgcac gagggagctt ccagggggaa acgcctggta tctttatagt cctgtcgggt   5100
   ttcgccacct ctgacttgag cgtcgatttt tgtgatgctc gtcagggggg cggagcctat   5160
   ggaaaaacgc cagcaacgcg gcctttttac ggttcctggc cttttgctgg ccttttgctc   5220
   acatgttctt tcctgcgtta tcccctgatt ctgtggataa ccgtattacc gcc          5273
<210> 91
   <211> 44
   <212> DNA
   <213> artificial sequence
<220>
   <223> chemically synthesized
<400> 91
   gaggagggta ccttaattaa ccggccctgg cactcattta ccca 44
<210> 92
   <211> 28
   <212> DNA
   <213> artificial sequence
<220>
   <223> chemically synthesized
<220>
   <221> misc_feature
   <222> (21)..(21)
   <223> n is a, c, g, or t
<400> 92
   gcggccgaga tcgagctcac ncagwctc 28
<210> 93
   <211> 23
   <212> DNA
   <213> artificial sequence
<220>
   <223> chemically synthesized
<400> 93
   acctttgata tccagtcgtg tcc 23
<210> 94
   <211> 23
   <212> DNA
   <213> artificial sequence
<220>
   <223> chemically synthesized
<400> 94
   acctttgata tccasyttgg tcc 23
<210> 95
   <211> 27
   <212> DNA
   <213> artificial sequence
<220>
   <223> chemically synthesized
<220>
   <221> misc_feature
   <222> (24)..(24)
   <223> n is a, c, g, or t
<400> 95
   caggcggccg agatcgagct cabncar 27
<210> 96
   <211> 27
   <212> DNA
   <213> artificial sequence
<220>
   <223> chemically synthesized
<400> 96
   caggcggccg agatcgagct cabdcag 27
<210> 97
   <211> 29
   <212> DNA
   <213> artificial sequence
<220>
   <223> chemically synthesized
<400> 97
   caggcggccg agatcgagct caykcagcc 29
<210> 98
   <211> 27
   <212> DNA
   <213> artificial sequence
<220>
   <223> chemically synthesized
<400> 98
   caggcggccg agatcgagct cacycar 27
<210> 99
   <211> 28
   <212> DNA
   <213> artificial sequence
<220>
   <223> chemically synthesized
<400> 99
   caggcggccg agatcgagct cactcagc 28
<210> 100
   <211> 24
   <212> DNA
   <213> artificial sequence
<220>
   <223> chemically synthesized
<400> 100
   accgccgagg atatccagct gggt 24
<210> 101
   <211> 24
   <212> DNA
   <213> artificial sequence
<220>
   <223> chemically synthesized
<400> 101
   accgcctagg atatcsasct tggt 24
<210> 102
   <211> 23
   <212> DNA
   <213> artificial sequence
<220>
   <223> chemically synthesized
<400> 102
   saggtgcagc tgctcgagtc kgg 23
<210> 103
   <211> 21
   <212> DNA
   <213> artificial sequence
<220>
   <223> chemically synthesized
<400> 103
   gccactagtg accgatgggc c 21
<210> 104
   <211> 15920
   <212> DNA
   <213> artificial sequence
<220>
   <223> chemically synthesized
<400> 104
   cgcgttttga gatttctgtc gccgactaaa ttcatgtcgc gcgatagtgg tgtttatcgc 60
   cgatagagat ggcgatattg gaaaaatcga tatttgaaaa tatggcatat tgaaaatgtc 120
   gccgatgtga gtttctgtgt aactgatatc gccatttttc caaaagtgat ttttgggcat 180
   acgcgatatc tggcgatagc gcttatatcg tttacggggg atggcgatag acgactttgg 240
   tgacttgggc gattctgtgt gtcgcaaata tcgcagtttc gatataggtg acagacgata 300
   tgaggctata tcgccgatag aggcgacatc aagctggcac atggccaatg catatcgatc 360
   tatacattga atcaatattg gccattagcc atattattca ttggttatat agcataaatc 420
   aatattggct attggccatt gcatacgttg tatccatatc ataatatgta catttatatt 480
   ggctcatgtc caacattacc gccatgttga cattgattat tgactagtta ttaatagtaa 540
   tcaattacgg ggtcattagt tcatagccca tatatggagt tccgcgttac ataacttacg 600
   gtaaatggcc cgcctggctg accgcccaac gacccccgcc cattgacgtc aataatgacg 660
   tatgttccca tagtaacgcc aatagggact ttccattgac gtcaatgggt ggagtattta 720
   cggtaaactg cccacttggc agtacatcaa gtgtatcata tgccaagtac gccccctatt 780
   gacgtcaatg acggtaaatg gcccgcctgg cattatgccc agtacatgac cttatgggac 840
   tttcctactt ggcagtacat ctacgtatta gtcatcgcta ttaccatggt gatgcggttt 900
   tggcagtaca tcaatgggcg tggatagcgg tttgactcac ggggatttcc aagtctccac     960
   cccattgacg tcaatgggag tttgttttgg caccaaaatc aacgggactt tccaaaatgt    1020
   cgtaacaact ccgccccatt gacgcaaatg ggcggtaggc gtgtacggtg ggaggtctat    1080
   ataagcagag ctcgtttagt gaaccgtcag atcgcctgga gacgccatcc acgctgtttt    1140
   gacctccata gaagacaccg ggaccgatcc agcctccgcg gccgggaacg gtgcattgga    1200
   acgcggattc cccgtgccaa gagtgacgta agtaccgcct atagagtcta taggcccacc    1260
   cccttggctt cttatgcatg ctatactgtt tttggcttgg ggtctataca cccccgcttc    1320
   ctcatgttat aggtgatggt atagcttagc ctataggtgt gggttattga ccattattga    1380
   ccactcccct attggtgacg atactttcca ttactaatcc ataacatggc tctttgccac    1440
   aactctcttt attggctata tgccaataca ctgtccttca gagactgaca cggactctgt    1500
   atttttacag gatggggtct catttattat ttacaaattc acatatacaa caccaccgtc    1560
   cccagtgccc gcagttttta ttaaacataa cgtgggatct ccacgcgaat ctcgggtacg    1620
   tgttccggac atgggctctt ctccggtagc ggcggagctt ctacatccga gccctgctcc    1680
   catgcctcca gcgactcatg gtcgctcggc agctccttgc tcctaacagt ggaggccaga    1740
   cttaggcaca gcacgatgcc caccaccacc agtgtgccgc acaaggccgt ggcggtaggg    1800
   tatgtgtctg aaaatgagct cggggagcgg gcttgcaccg ctgacgcatt tggaagactt    1860
   aaggcagcgg cagaagaaga tgcaggcagc tgagttgttg tgttctgata agagtcagag    1920
   gtaactcccg ttgcggtgct gttaacggtg gagggcagtg tagtctgagc agtactcgtt    1980
   gctgccgcgc gcgccaccag acataatagc tgacagacta acagactgtt cctttccatg    2040
   ggtcttttct gcagtcaccg tccttgacac gaagctgtcg cgagtcgcta gcaaggttta    2100
   aacgaattca ttgatcataa tcagccatac cacatttgta gaggttttac ttgctttaaa    2160
   aaacctccca cacctccccc tgaacctgaa acataaaatg aatgcaattg ttgttgttaa    2220
   cttgtttatt gcagcttata atggttacaa ataaagcaat agcatcacaa atttcacaaa    2280
   taaagcattt ttttcactgc attctagttg tggtttgtcc aaactcatca atgtatctta    2340
   tcatgtctgg cggccgccga tatttgaaaa tatggcatat tgaaaatgtc gccgatgtga    2400
   gtttctgtgt aactgatatc gccatttttc caaaagtgat ttttgggcat acgcgatatc    2460
   tggcgatagc gcttatatcg tttacggggg atggcgatag acgactttgg tgacttgggc    2520
   gattctgtgt gtcgcaaata tcgcagtttc gatataggtg acagacgata tgaggctata    2580
   tcgccgatag aggcgacatc aagctggcac atggccaatg catatcgatc tatacattga    2640
   atcaatattg gccattagcc atattattca ttggttatat agcataaatc aatattggct    2700
   attggccatt gcatacgttg tatccatatc ataatatgta catttatatt ggctcatgtc    2760
   caacattacc gccatgttga cattgattat tgactagtta ttaatagtaa tcaattacgg   2820
   ggtcattagt tcatagccca tatatggagt tccgcgttac ataacttacg gtaaatggcc   2880
   cgcctggctg accgcccaac gacccccgcc cattgacgtc aataatgacg tatgttccca   2940
   tagtaacgcc aatagggact ttccattgac gtcaatgggt ggagtattta cggtaaactg   3000
   cccacttggc agtacatcaa gtgtatcata tgccaagtac gccccctatt gacgtcaatg   3060
   acggtaaatg gcccgcctgg cattatgccc agtacatgac cttatgggac tttcctactt   3120
   ggcagtacat ctacgtatta gtcatcgcta ttaccatggt gatgcggttt tggcagtaca   3180
   tcaatgggcg tggatagcgg tttgactcac ggggatttcc aagtctccac cccattgacg   3240
   tcaatgggag tttgttttgg caccaaaatc aacgggactt tccaaaatgt cgtaacaact   3300
   ccgccccatt gacgcaaatg ggcggtaggc gtgtacggtg ggaggtctat ataagcagag   3360
   ctcgtttagt gaaccgtcag atcgcctgga gacgccatcc acgctgtttt gacctccata   3420
   gaagacaccg ggaccgatcc agcctccgcg gccgggaacg gtgcattgga acgcggattc   3480
   cccgtgccaa gagtgacgta agtaccgcct atagagtcta taggcccacc cccttggctt   3540
   cttatgcatg ctatactgtt tttggcttgg ggtctataca cccccgcttc ctcatgttat   3600
   aggtgatggt atagcttagc ctataggtgt gggttattga ccattattga ccactcccct   3660
   attggtgacg atactttcca ttactaatcc ataacatggc tctttgccac aactctcttt   3720
   attggctata tgccaataca ctgtccttca gagactgaca cggactctgt atttttacag   3780
   gatggggtct catttattat ttacaaattc acatatacaa caccaccgtc cccagtgccc   3840
   gcagttttta ttaaacataa cgtgggatct ccacgcgaat ctcgggtacg tgttccggac   3900
   atgggctctt ctccggtagc ggcggagctt ctacatccga gccctgctcc catgcctcca   3960
   gcgactcatg gtcgctcggc agctccttgc tcctaacagt ggaggccaga cttaggcaca   4020
   gcacgatgcc caccaccacc agtgtgccgc acaaggccgt ggcggtaggg tatgtgtctg   4080
   aaaatgagct cggggagcgg gcttgcaccg ctgacgcatt tggaagactt aaggcagcgg   4140
   cagaagaaga tgcaggcagc tgagttgttg tgttctgata agagtcagag gtaactcccg   4200
   ttgcggtgct gttaacggtg gagggcagtg tagtctgagc agtactcgtt gctgccgcgc   4260
   gcgccaccag acataatagc tgacagacta acagactgtt cctttccatg ggtcttttct   4320
   gcagtcaccg tccttgacac gaagcttggc gcgcccttta attaagactc gagcaattca   4380
   ttgatcataa tcagccatac cacatttgta gaggttttac ttgctttaaa aaacctccca   4440
   cacctccccc tgaacctgaa acataaaatg aatgcaattg ttgttgttaa cttgtttatt   4500
   gcagcttata atggttacaa ataaagcaat agcatcacaa atttcacaaa taaagcattt   4560
   ttttcactgc attctagttg tggtttgtcc aaactcatca atgtatctta tcatgtctgg 4620
   atcctctaga attcagcaag gtcgccacgc acaagatcaa tattaacaat cagtcatctc 4680
   tctttagcaa taaaaaggtg aaaaattaca ttttaaaaat gacaccatag acgatgtatg 4740
   aaaataatct acttggaaat aaatctaggc aaagaagtgc aagactgtta cccagaaaac 4800
   ttacaaattg taaatgagag gttagtgaag atttaaatga atgaagatct aaataaactt 4860
   ataaattgtg agagaaatta atgaatgtct aagttaatgc agaaacggag agacatacta 4920
   tattcatgaa ctaaaagact taatattgtg aaggtatact ttcttttcac ataaatttgt 4980
   agtcaatatg ttcaccccaa aaaagctgtt tgttaacttg tcaacctcat ttcaaaatgt 5040
   atatagaaag cccaaagaca ataacaaaaa tattcttgta gaacaaaatg ggaaagaatg 5100
   ttccactaaa tatcaagatt tagagcaaag catgagatgt gtggggatag acagtgaggc 5160
   tgataaaata gagtagagct cagaaacaga cccattgata tatgtaagtg acctatgaaa 5220
   aaaatatggc attttacaat gggaaaatga tgatcttttt cttttttaga aaaacaggga 5280
   aatatattta tatgtaaaaa ataaaaggga acccatatgt cataccatac acacaaaaaa 5340
   attccagtga attataagtc taaatggaga aggcaaaact ttaaatcttt tagaaaataa 5400
   tatagaagca tgccatcatg acttcagtgt agagaaaaat ttcttatgac tcaaagtcct 5460
   aaccacaaag aaaagattgt taattagatt gcatgaatat taagacttat ttttaaaatt 5520
   aaaaaaccat taagaaaagt caggccatag aatgacagaa aatatttgca acaccccagt 5580
   aaagagaatt gtaatatgca gattataaaa agaagtctta caaatcagta aaaaataaaa 5640
   ctagacaaaa atttgaacag atgaaagaga aactctaaat aatcattaca catgagaaac 5700
   tcaatctcag aaatcagaga actatcattg catatacact aaattagaga aatattaaaa 5760
   ggctaagtaa catctgtggc aatattgatg gtatataacc ttgatatgat gtgatgagaa 5820
   cagtacttta ccccatgggc ttcctcccca aacccttacc ccagtataaa tcatgacaaa 5880
   tatactttaa aaaccattac cctatatcta accagtactc ctcaaaactg tcaaggtcat 5940
   caaaaataag aaaagtctga ggaactgtca aaactaagag gaacccaagg agacatgaga 6000
   attatatgta atgtggcatt ctgaatgaga tcccagaaca gaaaaagaac agtagctaaa 6060
   aaactaatga aatataaata aagtttgaac tttagttttt tttaaaaaag agtagcatta 6120
   acacggcaaa gtcattttca tatttttctt gaacattaag tacaagtcta taattaaaaa 6180
   ttttttaaat gtagtctgga acattgccag aaacagaagt acagcagcta tctgtgctgt 6240
   cgcctaacta tccatagctg attggtctaa aatgagatac atcaacgctc ctccatgttt 6300
   tttgttttct ttttaaatga aaaactttat tttttaagag gagtttcagg ttcatagcaa 6360
   aattgagagg aaggtacatt caagctgagg aagttttcct ctattcctag tttactgaga 6420
   gattgcatca tgaatgggtg ttaaattttg tcaaatgctt tttctgtgtc tatcaatatg 6480
   accatgtgat tttcttcttt aacctgttga tgggacaaat tacgttaatt gattttcaaa 6540
   cgttgaacca cccttacata tctggaataa attctacttg gttgtggtgt atattttttg 6600
   atacattctt ggattctttt tgctaatatt ttgttgaaaa tgtttgtatc tttgttcatg 6660
   agagatattg gtctgttgtt ttcttttctt gtaatgtcat tttctagttc cggtattaag 6720
   gtaatgctgg cctagttgaa tgatttagga agtattccct ctgcttctgt cttctgaaag 6780
   agattgtaga aagttgatac aatttttttt tctttaaata tcttgataga attctagagg 6840
   atcgatcccc gccgccggac gaactaaacc tgactacggc atctctgccc cttcttcgcg 6900
   gggcagtgca tgtaatccct tcagttggtt ggtacaactt gccaactggg ccctgttcca 6960
   catgtgacac ggggggggac caaacacaaa ggggttctct gactgtagtt gacatcctta 7020
   taaatggatg tgcacatttg ccaacactga gtggctttca tcctggagca gactttgcag 7080
   tctgtggact gcaacacaac attgccttta tgtgtaactc ttggctgaag ctcttacacc 7140
   aatgctgggg gacatgtacc tcccaggggc ccaggaagac tacgggaggc tacaccaacg 7200
   tcaatcagag gggcctgtgt agctaccgat aagcggaccc tcaagagggc attagcaata 7260
   gtgtttataa ggcccccttg ttaaccctaa acgggtagca tatgcttccc gggtagtagt 7320
   atatactatc cagactaacc ctaattcaat agcatatgtt acccaacggg aagcatatgc 7380
   tatcgaatta gggttagtaa aagggtccta aggaacagcg atatctccca ccccatgagc 7440
   tgtcacggtt ttatttacat ggggtcagga ttccacgagg gtagtgaacc attttagtca 7500
   caagggcagt ggctgaagat caaggagcgg gcagtgaact ctcctgaatc ttcgcctgct 7560
   tcttcattct ccttcgttta gctaatagaa taactgctga gttgtgaaca gtaaggtgta 7620
   tgtgaggtgc tcgaaaacaa ggtttcaggt gacgccccca gaataaaatt tggacggggg 7680
   gttcagtggt ggcattgtgc tatgacacca atataaccct cacaaacccc ttgggcaata 7740
   aatactagtg taggaatgaa acattctgaa tatctttaac aatagaaatc catggggtgg 7800
   ggacaagccg taaagactgg atgtccatct cacacgaatt tatggctatg ggcaacacat 7860
   aatcctagtg caatatgata ctggggttat taagatgtgt cccaggcagg gaccaagaca 7920
   ggtgaaccat gttgttacac tctatttgta acaaggggaa agagagtgga cgccgacagc 7980
   agcggactcc actggttgtc tctaacaccc ccgaaaatta aacggggctc cacgccaatg 8040
   gggcccataa acaaagacaa gtggccactc ttttttttga aattgtggag tgggggcacg 8100
   cgtcagcccc cacacgccgc cctgcggttt tggactgtaa aataagggtg taataacttg 8160
   gctgattgta accccgctaa ccactgcggt caaaccactt gcccacaaaa ccactaatgg 8220
   caccccgggg aatacctgca taagtaggtg ggcgggccaa gataggggcg cgattgctgc 8280
   gatctggagg acaaattaca cacacttgcg cctgagcgcc aagcacaggg ttgttggtcc 8340
   tcatattcac gaggtcgctg agagcacggt gggctaatgt tgccatgggt agcatatact 8400
   acccaaatat ctggatagca tatgctatcc taatctatat ctgggtagca taggctatcc 8460
   taatctatat ctgggtagca tatgctatcc taatctatat ctgggtagta tatgctatcc 8520
   taatttatat ctgggtagca taggctatcc taatctatat ctgggtagca tatgctatcc 8580
   taatctatat ctgggtagta tatgctatcc taatctgtat ccgggtagca tatgctatcc 8640
   taatagagat tagggtagta tatgctatcc taatttatat ctgggtagca tatactaccc 8700
   aaatatctgg atagcatatg ctatcctaat ctatatctgg gtagcatatg ctatcctaat 8760
   ctatatctgg gtagcatagg ctatcctaat ctatatctgg gtagcatatg ctatcctaat 8820
   ctatatctgg gtagtatatg ctatcctaat ttatatctgg gtagcatagg ctatcctaat 8880
   ctatatctgg gtagcatatg ctatcctaat ctatatctgg gtagtatatg ctatcctaat 8940
   ctgtatccgg gtagcatatg ctatcctcat gcatatacag tcagcatatg atacccagta 9000
   gtagagtggg agtgctatcc tttgcatatg ccgccacctc ccaagggggc gtgaattttc 9060
   gctgcttgtc cttttcctgc atgctggttg ctcccattct taggtgaatt taaggaggcc 9120
   aggctaaagc cgtcgcatgt ctgattgctc accaggtaaa tgtcgctaat gttttccaac 9180
   gcgagaaggt gttgagcgcg gagctgagtg acgtgacaac atgggtatgc ccaattgccc 9240
   catgttggga ggacgaaaat ggtgacaaga cagatggcca gaaatacacc aacagcacgc 9300
   atgatgtcta ctggggattt attctttagt gcgggggaat acacggcttt taatacgatt 9360
   gagggcgtct cctaacaagt tacatcactc ctgcccttcc tcaccctcat ctccatcacc 9420
   tccttcatct ccgtcatctc cgtcatcacc ctccgcggca gccccttcca ccataggtgg 9480
   aaaccaggga ggcaaatcta ctccatcgtc aaagctgcac acagtcaccc tgatattgca 9540
   ggtaggagcg ggctttgtca taacaaggtc cttaatcgca tccttcaaaa cctcagcaaa 9600
   tatatgagtt tgtaaaaaga ccatgaaata acagacaatg gactccctta gcgggccagg 9660
   ttgtgggccg ggtccagggg ccattccaaa ggggagacga ctcaatggtg taagacgaca 9720
   ttgtggaata gcaagggcag ttcctcgcct taggttgtaa agggaggtct tactacctcc 9780
   atatacgaac acaccggcga cccaagttcc ttcgtcggta gtcctttcta cgtgactcct 9840
   agccaggaga gctcttaaac cttctgcaat gttctcaaat ttcgggttgg aacctccttg 9900
   accacgatgc tttccaaacc accctccttt tttgcgcctg cctccatcac cctgaccccg 9960
   gggtccagtg cttgggcctt ctcctgggtc atctgcgggg ccctgctcta tcgctcccgg 10020
   gggcacgtca ggctcaccat ctgggccacc ttcttggtgg tattcaaaat aatcggcttc 10080
   ccctacaggg tggaaaaatg gccttctacc tggagggggc ctgcgcggtg gagacccgga 10140
   tgatgatgac tgactactgg gactcctggg cctcttttct ccacgtccac gacctctccc 10200
   cctggctctt tcacgacttc cccccctggc tctttcacgt cctctacccc ggcggcctcc 10260
   actacctcct cgaccccggc ctccactacc tcctcgaccc cggcctccac tgcctcctcg 10320
   accccggcct ccacctcctg ctcctgcccc tcctgctcct gcccctcctc ctgctcctgc 10380
   ccctcctgcc cctcctgctc ctgcccctcc tgcccctcct gctcctgccc ctcctgcccc 10440
   tcctgctcct gcccctcctg cccctcctcc tgctcctgcc cctcctgccc ctcctcctgc 10500
   tcctgcccct cctgcccctc ctgctcctgc ccctcctgcc cctcctgctc ctgcccctcc 10560
   tgcccctcct gctcctgccc ctcctgctcc tgcccctcct gctcctgccc ctcctgctcc 10620
   tgcccctcct gcccctcctg cccctcctcc tgctcctgcc cctcctgctc ctgcccctcc 10680
   tgcccctcct gcccctcctg ctcctgcccc tcctcctgct cctgcccctc ctgcccctcc 10740
   tgcccctcct cctgctcctg cccctcctgc ccctcctcct gctcctgccc ctcctcctgc 10800
   tcctgcccct cctgcccctc ctgcccctcc tcctgctcct gcccctcctg cccctcctcc 10860
   tgctcctgcc cctcctcctg ctcctgcccc tcctgcccct cctgcccctc ctcctgctcc 10920
   tgcccctcct cctgctcctg cccctcctgc ccctcctgcc cctcctgccc ctcctcctgc 10980
   tcctgcccct cctcctgctc ctgcccctcc tgctcctgcc cctcccgctc ctgctcctgc 11040
   tcctgttcca ccgtgggtcc ctttgcagcc aatgcaactt ggacgttttt ggggtctccg 11100
   gacaccatct ctatgtcttg gccctgatcc tgagccgccc ggggctcctg gtcttccgcc 11160
   tcctcgtcct cgtcctcttc cccgtcctcg tccatggtta tcaccccctc ttctttgagg 11220
   tccactgccg ccggagcctt ctggtccaga tgtgtctccc ttctctccta ggccatttcc 11280
   aggtcctgta cctggcccct cgtcagacat ggtaaatcga tggccatggt ggccacgtgt 11340
   tcacgacacc tgaaatggaa gaaaaaaact ttgaaccact gtctgaggct tgagaatgaa 11400
   ccaagatcca aactcaaaaa ggccaaattc caaggagaat tacatcaagt gccaagctgg 11460
   cctaacttca gtctccaccc actcagtgtg gggaaactcc atcgcataaa acccctcccc 11520
   ccaacctaaa gacgacgtac tccaaaagct ccagaactaa tcgaggtgcc tggacggcgc 11580
   ccggtactcc gtggagtcac atgaagcgac ggctgaggac ggaaaggccc ttttcctttg 11640
   tgtgggtgac tcacccgccc gctctcccga gcgccgcgtc ctccattttg agccccctgg 11700
   agcagggccg ggaagcggcc atctttccgc tcacgcaact ggtgccgacc gggccagcct 11760
   tgccgcccag ggcggggcga tacacggcgg cgcgaggcca ggcaccagag caggccggcc 11820
   agcttgagac tacccccgtc cgattctcgg tggccgcgct cgcaggcccc gcctcgccga 11880
   acatgtgcgc tgggacgcac gggccccgtc gccggccgcg ggcccaaaaa ccgaaatacc 11940
   agtgtgcaga tcctggcccg catttacaag actatcttgc cagaaaaaaa gcgtcgcagc 12000
   aggtcatcaa aaattttaaa tggctagaga cttatcgaaa gcagcgagac aggcgcgaag 12060
   gtgccaccag attcgcacgc ggcggcccca gcgcccaggc caggcctcaa ctcaagcacg 12120
   aggcgaaggg gctcctaaag cgcaaggccc gcccctggct ccagctcggg atcaagaatc 12180
   acgtactgga gccaggtgga agtaattcaa ggcacgcaag ggccataacc cgtaaagagg 12240
   ccaggcccgc gggaaccaca cacggcactt acctgtgttc tggcggcaaa cccgttgcga 12300
   aaaagaacgt tcacggcgac tactgcactt atatacggtt ctcccccacc ctcgggaaaa 12360
   aggcggagcc agtacacgac atcactttcc cagtttaccc cgcgccacct tctctaggca 12420
   ccggttcaat tgccgacccc tccccccaac ttctcgggga ctgtgggcga tgtgcgctct 12480
   gcccactgac gggcaccgga gcctcacgaa gcttgaattc ggtaccatcg atgataagct 12540
   gtcaaacatg agaattcttg aagacgaaag ggcctcgtga tacgcctatt tttataggtt 12600
   aatgtcatga taataatggt ttcttagacg tcaggtggca cttttcgggg aaatgtgcgc 12660
   ggaaccccta tttgtttatt tttctaaata cattcaaata tgtatccgct catgagacaa 12720
   taaccctgat aaatgcttca ataatattga aaaaggaaga gtatgagtat tcaacatttc 12780
   cgtgtcgccc ttattccctt ttttgcggca ttttgccttc ctgtttttgc tcacccagaa 12840
   acgctggtga aagtaaaaga tgctgaagat cagttgggtg cacgagtggg ttacatcgaa 12900
   ctggatctca acagcggtaa gatccttgag agttttcgcc ccgaagaacg ttttccaatg 12960
   atgagcactt ttaaagttct gctatgtggc gcggtattat cccgtgttga cgccgggcaa 13020
   gagcaactcg gtcgccgcat acactattct cagaatgact tggttgagta ctcaccagtc 13080
   acagaaaagc atcttacgga tggcatgaca gtaagagaat tatgcagtgc tgccataacc 13140
   atgagtgata acactgcggc caacttactt ctgacaacga tcggaggacc gaaggagcta 13200
   accgcttttt tgcacaacat gggggatcat gtaactcgcc ttgatcgttg ggaaccggag 13260
   ctgaatgaag ccataccaaa cgacgagcgt gacaccacga tgcctgcagc aatggcaaca 13320
   acgttgcgca aactattaac tggcgaacta cttactctag cttcccggca acaattaata 13380
   gactggatgg aggcggataa agttgcagga ccacttctgc gctcggccct tccggctggc 13440
   tggtttattg ctgataaatc tggagccggt gagcgtgggt ctcgcggtat cattgcagca 13500
   ctggggccag atggtaagcc ctcccgtatc gtagttatct acacgacggg gagtcaggca 13560
   actatggatg aacgaaatag acagatcgct gagataggtg cctcactgat taagcattgg 13620
   taactgtcag accaagttta ctcatatata ctttagattg atttaaaact tcatttttaa 13680
   tttaaaagga tctaggtgaa gatccttttt gataatctca tgaccaaaat cccttaacgt 13740
   gagttttcgt tccactgagc gtcagacccc gtagaaaaga tcaaaggatc ttcttgagat 13800
   cctttttttc tgcgcgtaat ctgctgcttg caaacaaaaa aaccaccgct accagcggtg 13860
   gtttgtttgc cggatcaaga gctaccaact ctttttccga aggtaactgg cttcagcaga 13920
   gcgcagatac caaatactgt ccttctagtg tagccgtagt taggccacca cttcaagaac 13980
   tctgtagcac cgcctacata cctcgctctg ctaatcctgt taccagtggc tgctgccagt 14040
   ggcgataagt cgtgtcttac cgggttggac tcaagacgat agttaccgga taaggcgcag 14100
   cggtcgggct gaacgggggg ttcgtgcaca cagcccagct tggagcgaac gacctacacc 14160
   gaactgagat acctacagcg tgagctatga gaaagcgcca cgcttcccga agggagaaag 14220
   gcggacaggt atccggtaag cggcagggtc ggaacaggag agcgcacgag ggagcttcca 14280
   gggggaaacg cctggtatct ttatagtcct gtcgggtttc gccacctctg acttgagcgt 14340
   cgatttttgt gatgctcgtc aggggggcgg agcctatgga aaaacgccag caacgcggcc 14400
   tttttacggt tcctggcctt ttgctgcgcc gcgtgcggct gctggagatg gcggacgcga 14460
   tggatatgtt ctgccaaggg ttggtttgcg cattcacagt tctccgcaag aattgattgg 14520
   ctccaattct tggagtggtg aatccgttag cgaggccatc cagcctcgcg tcgaactaga 14580
   tgatccgctg tggaatgtgt gtcagttagg gtgtggaaag tccccaggct ccccagcagg 14640
   cagaagtatg caaagcatgc atctcaatta gtcagcaacc aggtgtggaa agtccccagg 14700
   ctccccagca ggcagaagta tgcaaagcat gcatctcaat tagtcagcaa ccatagtccc 14760
   gcccctaact ccgcccatcc cgcccctaac tccgcccagt tccgcccatt ctccgcccca 14820
   tggctgacta atttttttta tttatgcaga ggccgaggcc gcctcggcct ctgagctatt 14880
   ccagaagtag tgaggaggct tttttggagg gtgaccgcca cgaggtgccg ccaccatccc 14940
   ctgacccacg cccctgaccc ctcacaagga gacgaccttc catgaccgag tacaagccca 15000
   cggtgcgcct cgccacccgc gacgacgtcc cccgggccgt acgcaccctc gccgccgcgt 15060
   tcgccgacta ccccgccacg cgccacaccg tcgaccccga ccgccacatc gaacgcgtca 15120
   ccgagctgca agaactcttc ctcacgcgcg tcgggctcga catcggcaag gtgtgggtcg 15180
   cggacgacgg cgccgcggtg gcggtctgga ccacgccgga gagcgtcgaa gcgggggcgg 15240
   tgttcgccga gatcggcccg cgcatggccg agttgagcgg ttcccggctg gccgcgcagc 15300
   aacagatgga aggcctcctg gcgccgcacc ggcccaagga gcccgcgtgg ttcctggcca 15360
   ccgtcggcgt ctcgcccgac caccagggca agggtctggg cagcgccgtc gtgctccccg 15420
   gagtggaggc ggccgagcgc gccggggtgc ccgccttcct ggagacctcc gcgccccgca 15480
   acctcccctt ctacgagcgg ctcggcttca ccgtcaccgc cgacgtcgag tgcccgaagg 15540
   accgcgcgac ctggtgcatg acccgcaagc ccggtgcctg acgcccgccc cacgacccgc  15600
   agcgcccgac cgaaaggagc gcacgacccg gtccgacggc ggcccacggg tcccaggggg  15660
   gtcgacctcg aaacttgttt attgcagctt ataatggtta caaataaagc aatagcatca  15720
   caaatttcac aaataaagca tttttttcac tgcattctag ttgtggtttg tccaaactca  15780
   tcaatgtatc ttatcatgtc tggatcgatc cgaacccctt cctcgaccaa ttctcatgtt  15840
   tgacagctta tcatcgcaga tccgggcaac gttgttgcat tgctgcaggc gcagaactgg  15900
   taggtatgga agatctgggg                                              15920
<210> 105
   <211> 21
   <212> PRT
   <213> Mus musculus
<400> 105
   Met Glu Thr Asp Thr Leu Leu Leu Trp Val Leu Leu Leu Trp Val Pro
   1               5                   10                  15
   Gly Ser Thr Gly Asp
               20
<210> 106
   <211> 51
   <212> PRT
   <213> Homo sapiens
<400> 106
   Arg Asn Ala Val Gly Gln Asp Thr Gln Glu Val Ile Val Val Pro His
   1               5                   10                  15
   Ser Leu Pro Phe Lys Val Val Val Ile Ser Ala Ile Leu Ala Leu Val
               20                  25                  30
   Val Leu Thr Ile Ile Ser Leu Ile Ile Leu Ile Met Leu Trp Gln Lys
           35                  40                  45
   Lys Pro Arg
       50
<210> 107
   <211> 18
   <212> PRT
   <213> artificial sequence
<220>
   <223> chemically synthesized
<400> 107
   Gly Gly Ser Ser Arg Ser Ser Ser Ser Gly Gly Gly Gly Ser Gly Gly
   1               5                   10                  15
   Gly Gly
<210> 108
   <211> 10
   <212> PRT
   <213> artificial sequence
<220>
   <223> chemically synthesized
<400> 108
   Tyr Pro Tyr Asp Val Pro Asp Tyr Ala Ser
   1               5                   10
<210> 109
   <211> 225
   <212> PRT
   <213> artificial sequence
<220>
   <223> chemically synthesized
<400> 109
   Thr His Thr Cys Pro Pro Cys Pro Ala Pro Glu Ala Glu Gly Ala Pro
   1               5                   10                  15
   Ser Val Phe Leu Phe Pro Pro Lys Pro Lys Asp Thr Leu Met Ile Ser
               20                  25                  30
   Arg Thr Pro Glu Val Thr Cys Val Val Val Asp Val Ser His Glu Asp
           35                  40                  45
   Pro Glu Val Lys Phe Asn Trp Tyr Val Asp Gly Val Glu Val His Asn
       50                  55                  60
   Ala Lys Thr Lys Pro Arg Glu Glu Gln Tyr Asn Ser Thr Tyr Arg Val
   65                  70                  75                  80
   Val Ser Val Leu Thr Val Leu His Gln Asp Trp Leu Asn Gly Lys Glu
                   85                  90                  95
   Tyr Lys Cys Lys Val Ser Asn Lys Ala Leu Pro Ala Ser Ile Glu Lys
               100                 105                 110
   Thr Ile Ser Lys Ala Lys Gly Gln Pro Arg Glu Pro Gln Val Tyr Thr
           115                 120                 125
   Leu Pro Pro Ser Arg Asp Glu Leu Thr Lys Asn Gln Val Ser Leu Thr
       130                 135                 140
   Cys Leu Val Lys Gly Phe Tyr Pro Ser Asp Ile Ala Val Glu Trp Glu
   145                 150                 155                 160
   Ser Asn Gly Gln Pro Glu Asn Asn Tyr Lys Thr Thr Pro Pro Val Leu
                   165                 170                 175
   Asp Ser Asp Gly Ser Phe Phe Leu Tyr Ser Lys Leu Thr Val Asp Lys
               180                 185                 190
   Ser Arg Trp Gln Gln Gly Asn Val Phe Ser Cys Ser Val Met His Glu
           195                 200                 205
   Ala Leu His Asn His Tyr Thr Gln Lys Ser Leu Ser Leu Ser Pro Gly
       210                 215                 220
   Lys
   225
<210> 110
   <211> 4613
   <212> DNA
   <213> artificial sequence
<220>
   <223> chemically synthesized
<400> 110
   gagctggcta gcgccaccat ggcctgggct ctgctcctcc tcaccctcct cactcagggc      60
   acagggtcct gggcccagtc tgagctcctg caggaattcg atatcctagg tcagcccaag     120
   gctgccccct cggtcactct gttcccgccc tcctctgagg agcttcaagc caacaaggcc    180
   acactggtgt gtctcataag tgacttctac ccgggagccg tgacagtggc ctggaaggca     240
   gatagcagcc ccgtcaaggc gggagtggag accaccacac cctccaaaca aagcaacaac     300
   aagtacgcgg ccagcagcta tctgagcctg acgcctgagc agtggaagtc ccacagaagc     360
   tacagctgcc aggtcacgca tgaagggagc accgtggaga agacagtggc ccctacagaa     420
   tgttcatagg tttaaacggt accaggtaag tgtacccaat tcgccctata gtgagtcgta     480
   ttacaattca ctcgatcgcc cttcccaaca gttgcgcagc ctgaatggcg aatggagatc     540
   caatttttaa gtgtataatg tgttaaacta ctgattctaa ttgtttgtgt attttagatt     600
   cacagtccca aggctcattt caggcccctc agtcctcaca gtctgttcat gatcataatc 660
   agccatacca catttgtaga ggttttactt gctttaaaaa acctcccaca cctccccctg 720
   aacctgaaac ataaaatgaa tgcaattgtt gttgttaact tgtttattgc agcttataat 780
   ggttacaaat aaagcaatag catcacaaat ttcacaaata aagcattttt ttcactgcat 840
   tctagttgtg gtttgtccaa actcatcaat gtatcttaac gcgtaaattg taagcgttaa 900
   tattttgtta aaattcgcgt taaatttttg ttaaatcagc tcatttttta accaataggc 960
   cgaaatcggc aaaatccctt ataaatcaaa agaatagacc gagatagggt tgagtgttgt 1020
   tccagtttgg aacaagagtc cactattaaa gaacgtggac tccaacgtca aagggcgaaa 1080
   aaccgtctat cagggcgatg gcccactacg tgaaccatca ccctaatcaa gttttttggg 1140
   gtcgaggtgc cgtaaagcac taaatcggaa ccctaaaggg agcccccgat ttagagcttg 1200
   acggggaaag ccggcgaacg tggcgagaaa ggaagggaag aaagcgaaag gagcgggcgc 1260
   tagggcgctg gcaagtgtag cggtcacgct gcgcgtaacc accacacccg ccgcgcttaa 1320
   tgcgccgcta cagggcgcgt caggtggcac ttttcgggga aatgtgcgcg gaacccctat 1380
   ttgtttattt ttctaaatac attcaaatat gtatccgctc atgagacaat aaccctgata 1440
   aatgcttcaa taatattgaa aaaggaagaa tcctgaggcg gaaagaacca gctgtggaat 1500
   gtgtgtcagt tagggtgtgg aaagtcccca ggctccccag caggcagaag tatgcaaagc 1560
   atgcatctca attagtcagc aaccaggtgt ggaaagtccc caggctcccc agcaggcaga 1620
   agtatgcaaa gcatgcatct caattagtca gcaaccatag tcccgcccct aactccgccc 1680
   atcccgcccc taactccgcc cagttccgcc cattctccgc cccatggctg actaattttt 1740
   tttatttatg cagaggccga ggccgcctcg gcctctgagc tattccagaa gtagtgagga 1800
   ggcttttttg gaggcctagg cttttgcaaa gatcgatcaa gagacaggat gaggatcgtt 1860
   tcgcatgatt gaacaagatg gattgcacgc aggttctccg gccgcttggg tggagaggct 1920
   attcggctat gactgggcac aacagacaat cggctgctct gatgccgccg tgttccggct 1980
   gtcagcgcag gggcgcccgg ttctttttgt caagaccgac ctgtccggtg ccctgaatga 2040
   actgcaagac gaggcagcgc ggctatcgtg gctggccacg acgggcgttc cttgcgcagc 2100
   tgtgctcgac gttgtcactg aagcgggaag ggactggctg ctattgggcg aagtgccggg 2160
   gcaggatctc ctgtcatctc accttgctcc tgccgagaaa gtatccatca tggctgatgc 2220
   aatgcggcgg ctgcatacgc ttgatccggc tacctgccca ttcgaccacc aagcgaaaca 2280
   tcgcatcgag cgagcacgta ctcggatgga agccggtctt gtcgatcagg atgatctgga 2340
   cgaagaacat caggggctcg cgccagccga actgttcgcc aggctcaagg cgagcatgcc 2400
   cgacggcgag gatctcgtcg tgacccatgg cgatgcctgc ttgccgaata tcatggtgga 2460
   aaatggccgc ttttctggat tcatcgactg tggccggctg ggtgtggcgg accgctatca 2520
   ggacatagcg ttggctaccc gtgatattgc tgaagaactt ggcggcgaat gggctgaccg 2580
   cttcctcgtg ctttacggta tcgccgctcc cgattcgcag cgcatcgcct tctatcgcct 2640
   tcttgacgag ttcttctgag cgggactctg gggttcgaaa tgaccgacca agcgacgccc 2700
   aacctgccat cacgagattt cgattccacc gccgccttct atgaaaggtt gggcttcgga 2760
   atcgttttcc gggacgccgg ctggatgatc ctccagcgcg gggatctcat gctggagttc 2820
   ttcgcccacc ctagggggag gctaactgaa acacggaagg agacaatacc ggaaggaacc 2880
   cgcgctatga cggcaataaa aagacagaat aaaacgcacg gtgttgggtc gtttgttcat 2940
   aaacgcgggg ttcggtccca gggctggcac tctgtcgata ccccaccgag accccattgg 3000
   ggccaatacg cccgcgtttc ttccttttcc ccaccccacc ccccaagttc gggtgaaggc 3060
   ccagggctcg cagccaacgt cggggcggca ggccctgcca tagcctcagg ttactcatat 3120
   atactttaga ttgatttaaa acttcatttt taatttaaaa ggatctaggt gaagatcctt 3180
   tttgataatc tcatgaccaa aatcccttaa cgtgagtttt cgttccactg agcgtcagac 3240
   cccgtagaaa agatcaaagg atcttcttga gatccttttt ttctgcgcgt aatctgctgc 3300
   ttgcaaacaa aaaaaccacc gctaccagcg gtggtttgtt tgccggatca agagctacca 3360
   actctttttc cgaaggtaac tggcttcagc agagcgcaga taccaaatac tgtccttcta 3420
   gtgtagccgt agttaggcca ccacttcaag aactctgtag caccgcctac atacctcgct 3480
   ctgctaatcc tgttaccagt ggctgctgcc agtggcgata agtcgtgtct taccgggttg 3540
   gactcaagac gatagttacc ggataaggcg cagcggtcgg gctgaacggg gggttcgtgc 3600
   acacagccca gcttggagcg aacgacctac accgaactga gatacctaca gcgtgagcta 3660
   tgagaaagcg ccacgcttcc cgaagggaga aaggcggaca ggtatccggt aagcggcagg 3720
   gtcggaacag gagagcgcac gagggagctt ccagggggaa acgcctggta tctttatagt 3780
   cctgtcgggt ttcgccacct ctgacttgag cgtcgatttt tgtgatgctc gtcagggggg 3840
   cggagcctat ggaaaaacgc cagcaacgcg gcctttttac ggttcctggc cttttgctgg 3900
   ccttttgctc acatgttctt tcctgcgtta tcccctgatt ctgtggataa ccgtattacc 3960
   gccatgcatt agttattaat agtaatcaat tacggggtca ttagttcata gcccatatat 4020
   ggagttccgc gttacataac ttacggtaaa tggcccgcct ggctgaccgc ccaacgaccc 4080
   ccgcccattg acgtcaataa tgacgtatgt tcccatagta acgccaatag ggactttcca 4140
   ttgacgtcaa tgggtggagt atttacggta aactgcccac ttggcagtac atcaagtgta 4200
   tcatatgcca agtacgcccc ctattgacgt caatgacggt aaatggcccg cctggcatta 4260
   tgcccagtac atgaccttat gggactttcc tacttggcag tacatctacg tattagtcat 4320
   cgctattacc atggtgatgc ggttttggca gtacatcaat gggcgtggat agcggtttga 4380
   ctcacgggga tttccaagtc tccaccccat tgacgtcaat gggagtttgt tttggcacca 4440
   aaatcaacgg gactttccaa aatgtcgtaa caactccgcc ccattgacgc aaatgggcgg 4500
   taggcgtgta cggtgggagg tctatataag cagagctggt ttagtgaacc gtcagatccg 4560
   ctagcgatta cgccaagctc gaaattaacc ctcactaaag ggaacaaaag ctg 4613
<210> 111
   <211> 45
   <212> DNA
   <213> artificial sequence
<220>
   <223> chemically synthesized
<400> 111
   ggctagcgcc accatggcct gggctctgct cctcctcacc ctcct 45
<210> 112
   <211> 43
   <212> DNA
   <213> artificial sequence
<220>
   <223> chemically synthesized
<400> 112
   gtgaggagga gcagagccca ggccatggtg gcgctagcca gct 43
<210> 113
   <211> 42
   <212> DNA
   <213> artificial sequence
<220>
   <223> chemically synthesized
<400> 113
   cactcagggc acagggtcct gggcccagtc tgagctcctg ca 42
<210> 114
   <211> 44
   <212> DNA
   <213> artificial sequence
<220>
   <223> chemically synthesized
<400> 114
   ggagctcaga ctgggcccag gaccctgtgc cctgagtgag gagg 44
<210> 115
   <211> 35
   <212> DNA
   <213> artificial sequence
<220>
   <223> chemically synthesized
<400> 115
   gaggaggata tcctaggtca gcccaaggct gcccc 35
<210> 116
   <211> 41
   <212> DNA
   <213> artificial sequence
<220>
   <223> chemically synthesized
<400> 116
   gaggagggta ccgtttaaac ctatgaacat tctgtagggg c 41
<210> 117
   <211> 1075
   <212> DNA
   <213> artificial sequence
<220>
   <223> chemically synthseized
<400> 117
   ggcgcgccac catggactgg acctggagga tcctcttctt ggtggcagca gccacaggag     60
   cccactccca gatgcaactg ctcgaggcct ccaccaaggg cccatcggtc ttccccctgg    120
   cgccctgctc caggagcacc tccgagagca cagcggccct gggctgcctg gtcaaggact    180
   acttccccga accggtgacg gtgtcgtgga actcaggcgc tctgaccagc ggcgtgcaca    240
   ccttcccagc tgtcctacag tcctcaggac tctactccct cagcagcgtg gtgaccgtgc    300
   cctccagcaa cttcggcacc cagacctaca cctgcaacgt agatcacaag cccagcaaca    360
   ccaaggtgga caagacagtt gagcgcaaat gttgtgtcga gtgcccaccg tgcccagcac    420
   cacctgtggc aggaccgtca gtcttcctct tccccccaaa acccaaggac accctcatga    480
   tctcccggac ccctgaggtc acgtgcgtgg tggtggacgt gagccacgaa gaccccgagg    540
   tccagttcaa ctggtacgtg gacggcgtgg aggtgcataa tgccaagaca aagccacggg    600
   aggagcagtt caacagcacg ttccgtgtgg tcagcgtcct caccgttgtg caccaggact    660
   ggctgaacgg caaggagtac aagtgcaagg tctccaacaa aggcctccca gcccccatcg    720
   agaaaaccat ctccaaaacc aaagggcagc cccgagaacc acaggtgtac accctgcccc    780
   catcccggga ggagatgacc aagaaccagg tcagcctgac ctgcctggtc aaaggcttct    840
   accccagcga catcgccgtg gagtgggaga gcaatgggca gccggagaac aactacaaga 900
   ccacacctcc catgctggac tccgacggct ccttcttcct ctacagcaag ctcaccgtgg 960
   acaagagcag gtggcagcag gggaacgtct tctcatgctc cgtgatgcat gaggctctgc 1020
   acaaccacta cacgcagaag agcctgtccc tgtctccggg taaatgatta attaa 1075
<210> 118
   <211> 1087
   <212> DNA
   <213> artificial sequence
<220>
   <223> chemically synthesized
<400> 118
   ggcgcgccac catggactgg acctggagga tcctcttctt ggtggcagca gccacaggag 60
   cccactccca gatgcaactg ctcgaggcct ccaccaaggg cccatcggtc ttccccctgg 120
   caccctcctc caagagcacc tctgggggca cagcggccct gggctgcctg gtcaaggact 180
   acttccccga accggtgacg gtgtcgtgga actcaggcgc cctgaccagc ggcgtgcaca 240
   ccttcccggc tgtcctacag tcctcaggac tctactccct cagcagcgtg gtgaccgtgc 300
   cctccagcag cttgggcacc cagacctaca tctgcaacgt gaatcacaag cccagcaaca 360
   ccaaggtgga caagaaagtt gagcccaaat cttgtgacaa aactcacaca tgcccaccgt 420
   gcccagcacc tgaactcctg gggggaccgt cagtcttcct cttcccccca aaacccaagg 480
   acaccctcat gatctcccgg acccctgagg tcacatgcgt ggtggtggac gtgagccacg 540
   aagaccctga ggtcaagttc aactggtacg tggacggcgt ggaggtgcat aatgccaaga 600
   caaagccgcg ggaggagcag tacaacagca cgtaccgtgt ggtcagcgtc ctcaccgtcc 660
   tgcaccagga ctggctgaat ggcaaggagt acaagtgcaa ggtctccaac aaagccctcc 720
   cagcccccat cgagaaaacc atctccaaag ccaaagggca gccccgagaa ccacaggtgt 780
   acaccctgcc cccatcccgg gatgagctga ccaagaacca ggtcagcctg acctgcctgg 840
   tcaaaggctt ctatcccagc gacatcgccg tggagtggga gagcaatggg cagccggaga 900
   acaactacaa gaccacgcct cccgtgctgg actccgacgg ctccttcttc ctctacagca 960
   agctcaccgt ggacaagagc aggtggcagc aggggaacgt cttctcatgc tccgtgatgc 1020
   atgaggctct gcacaaccac tacacgcaga agagcctctc cctgtctccg ggtaaatgat 1080
   taattaa 1087
<210> 119
   <211> 1091
   <212> DNA
   <213> artificial sequence
<220>
   <223> chemically synthesized
<400> 119
   ggcgcgccac catggactgg acctggagga tcctcttctt ggtggcagca gccacaggag      60
   cccactccca gatgcaactg ctcgaggcct ccaccaaggg cccatcggtc ttccccctgg      120
   cgccctgctc caggagcacc tccgagagca cagcggccct gggctgcctg gtcaaggact     180
   acttccccga accggtgacg gtgtcgtgga actcaggcgc cctgaccagc ggcgtgcaca     240
   ccttcccggc tgtcctacag tcctcaggac tctactccct cagcagcgtg gtgaccgtgc     300
   cctccagcag cttgggcacg aagacctaca cctgcaatgt agatcacaag cccagcaaca     360
   ccaaggtgga caagagagtt gagtccaaat atggtccccc gtgcccatca tgcccagcac     420
   ctgaattcct ggggggacca tcagtcttcc tgttcccccc aaaacccaag gacaccctca     480
   tgatctcccg gacccctgag gtcacgtgcg tggtggtgga cgtgagccag gaagaccccg     540
   aggtccagtt caactggtac gtggatggcg tggaggtgca taatgccaag acaaagccgc     600
   gggaggagca gttcaacagc acgtaccgtg tggtcagcgt cctcaccgtc gtgcaccagg     660
   actggctgaa cggcaaggag tacaagtgca aggtctccaa caaaggcctc ccgtcctcca     720
   tcgagaaaac catctccaaa gccaaagggc agccccgaga gccacaggtg tacaccctgc     780
   ccccatccca ggaggagatg accaagaacc aggtcagcct gacctgcctg gtcaaaggct     840
   tctaccccag cgacatcgcc gtggagtggg agagcaatgg gcagccggag aacaactaca     900
   agaccacgcc tcccgtgctg gactccgacg gctccttctt cctctacagc aggctaaccg     960
   tggacaagag caggtggcag gaggggaatg tcttctcatg ctccgtgatg catgaggctc    1020
   tgcacaacca ctacacgcag aagagcctct ccctgtctct gggtaaatga gtgccagggc    1080
   cggttaatta a                                                         1091
<210> 120
   <211> 400
   <212> DNA
   <213> artificial sequence
<220>
   <223> chemically synthesized
<400> 120
   ggcgcgccac catggactgg acctggagga tcctcttctt ggtggcagca gccacaggag     60
   cccactccca gatgcaactg ctcgaggcct ccaccaaggg cccatcggtc ttccccctgg    120
   cgccctgctc caggagcacc tccgagagca cagcggccct gggctgcctg gtcaaggact    180
   acttccccga accggtgacg gtgtcgtgga actcaggcgc tctgaccagc ggcgtgcaca    240
   ccttcccagc tgtcctacag tcctcaggac tctactccct cagcagcgtg gtgaccgtgc    300
   cctccagcaa cttcggcacc cagacctaca cctgcaacgt agatcacaag cccagcaaca    360
   ccaaggtgga caagacagtt gagcgcaaat gattaattaa                          400
<210> 121
   <211> 443
   <212> DNA
   <213> artificial sequence
<220>
   <223> chemically synthesized
<400> 121
   gctagcgcca ccatggacat gagggtcccc gctcagctcc tggggctcct gctactctgg     60
   ctccgaggtg ccagatgtga catcgagctc ctgcaggaat tcgatatcaa acgaactgtg    120
   gctgcaccat ctgtcttcat cttcccgcca tctgatgagc agttgaaatc tggaactgcc    180
   tctgttgtgt gcctgctgaa taacttctat cccagagagg ccaaagtaca gtggaaggtg    240
   gataacgccc tccaatcggg taactcccag gagagtgtca cagagcagga cagcaaggac    300
   agcacctaca gcctcagcag caccctgacg ctgagcaaag cagactacga gaaacacaaa    360
   gtctacgcct gcgaagtcac ccatcagggc ctgagttcgc ccgtcacaaa gagcttcaac    420
   aggggagagt gttaggttta aac                                            443
<210> 122
   <211> 431
   <212> DNA
   <213> artificial sequence
<220>
   <223> chemically synthesized
<400> 122
   gctagcgcca ccatggcctg ggctctgctc ctcctcaccc tcctcactca gggcacaggg     60
   tcctgggccc agtctgagct cctgcaggaa ttcgatatcc taggtcagcc caaggctgcc    120
   ccctcggtca ctctgttccc gccctcctct gaggagcttc aagccaacaa ggccacactg    180
   gtgtgtctca taagtgactt ctacccggga gccgtgacag tggcctggaa ggcagatagc    240
   agccccgtca aggcgggagt ggagaccacc acaccctcca aacaaagcaa caacaagtac    300
   gcggccagca gctatctgag cctgacgcct gagcagtgga agtcccacag aagctacagc    360
   tgccaggtca cgcatgaagg gagcaccgtg gagaagacag tggcccctac agaatgttca    420
   taggtttaaa c                                                         431
<210> 123
   <211> 21
   <212> DNA
   <213> artificial sequence
<220>
   <223> chemically synthesized
<400> 123
   agcgggggct tgccggccct g 21
<210> 124
   <211> 27
   <212> PRT
   <213> Hepatitis B virus Genotyp D
<400> 124
   Pro Leu Gly Phe Phe Pro Asp His Gln Leu Asp Pro Ala Phe Arg Ala
   1               5                   10                  15
   Asn Thr Ala Asn Pro Asp Trp Asp Phe Asn Pro
               20                  25